# EUROPEAN PATENT SPECIFICATION

(11) **EP 1 572 739 B1**
(45) Date of publication and mention of the grant of the patent: **16.07.2008**
(21) Application number: 03767837.2
(22) Date of filing: 19.12.2003
(51) Int. Cl.: C07K 14/705, A61K 38/17, A61P 11/06, C12N 5/12, C12Q 1/68

(54) **ASTHMA SUSCEPTIBILITY LOCUS**
ASTHMA-EMPFINDLICHKEITSORT
LOCUS DE SUSCEPTIBILITE A L'ASTHME

(30) Priority: 20.12.2002 US 435846 P; 03.01.2003 US 437895 P; 26.03.2003 US 458767 P; 09.07.2003 US 486000 P
(43) Date of publication of application: 14.09.2005
(73) Proprietor: Geneos OY, 00250 Helsinki (FI)
(72) Inventor: LAITINEN, Tarja, FIN-04400 Järvenpää (FI); KERE, Juha, FIN-02170 Espoo (FI); LAITINEN, Lauri, A., FIN-02230 Espoo (FI); POLVI, Anne, FIN-02920 Espoo (FI); MÄKELÄ, Siru, FIN-00320 Helsinki (FI); VENDELIN, Johanna, FIN-02610 Espoo (FI); PULKKINEN, Ville, FIN-00380 Helsinki (FI); SALMIKANGAS, Paula, FIN-01800 Klaukkala (FI)
(74) Representative: Matilainen, Mirja Helena
(86) International application number: PCT/FI2003/000973
(87) International publication number: WO 2004/056866

(56) References cited:
- EP-A1- 1 365 030
- WO-A1-01/18206
- WO-A1-01/48188
- WO-A1-01/77137
- WO-A1-02/18412
- WO-A1-02/31145
- WO-A1-03/007187
- WO-A1-03/025179
- WO-A2-01/48015
- WO-A2-01/96400
- WO-A2-02/42458
- WO-A2-02/063004
- DATABASE WPI Derwent Publications Ltd., London, GB; Class B04, AN 2001-610074, XP002190915 & JP 2001 245666 A (KYOWA HAKKO KOGYO KK) 11 September 2001
- ANNE POLVI ET AL.: 'Physical map of an asthma susceptibility locus in 7p15-p14 and association study of TCRG' EUROPEAN JOURNAL OF HUMAN GENETICS vol. 10, 2002, pages 658 - 665, XP002977261

### SEQUENCE LISTING

<110> Geneos Oy
<120> Asthma susceptibility locus
<130> 40564
<150> US 60/435,846
   <151> 2002-12-20
<150> US 60/437,895
   <151> 2003-01-03
<150> US 60/458,767
   <151> 2003-03-26
<150> US 60/486,000
   <151> 2003-07-09
<160> 41
<170> PatentIn version 3.0
<210> 1
   <211> 129017
   <212> DNA
   <213> Homo sapiens
<400> 1
<210> 2
   <211> 1567
   <212> DNA
   <213> Homo sapiens
<220>
   <221> CDS
   <222> (129)..(1241)
<400> 2
   gggctcaggg agggctctgt gcctccgttc agcagagctg cagctgctgc ccagctctca      60
   ggaggcaagc tggactccct cactcagctg caggagcaag gacagtgagg ctcaaccccg     120
   cctgagcc atg cca gcc aac ttc aca gag ggc agc ttc gat tcc agt ggg      170
            Met Pro Ala Asn Phe Thr Glu Gly Ser Phe Asp Ser Ser Gly
            1               5                   10
   acc ggg cag acg ctg gat tct tcc cca gtg gct tgc act gaa aca gtg      218
   Thr Gly Gln Thr Leu Asp Ser Ser Pro Val Ala Cys Thr Glu Thr Val
   15                  20                  25                  30
   act ttt act gaa gtg gtg gaa gga aag gaa tgg ggt tcc ttc tac tac        266
   Thr Phe Thr Glu Val Val Glu Gly Lys Glu Trp Gly Ser Phe Tyr Tyr
               35                  40                  45
   tcc ttt aag act gag caa ttg ata act ctg tgg gtc ctc ttt gtt ttt        314
   Ser Phe Lys Thr Glu Gln Leu Ile Thr Leu Trp Val Leu Phe Val Phe
           50                  55                  60
   acc att gtt gga aac tcc gtt gtg ctt ttt tcc aca tgg agg aga aag        362
   Thr Ile Val Gly Asn Ser Val Val Leu Phe Ser Thr Trp Arg Arg Lys
       65                  70                  75
   aag aag tca aga atg acc ttc ttt gtg act cag ctg gcc atc aca gat        410
   Lys Lys Ser Arg Met Thr Phe Phe Val Thr Gln Leu Ala Ile Thr Asp
   80                  85                  90
   tct ttc aca gga ctg gtc aac atc ttg aca gat att aat tgg cga ttc        458
   Ser Phe Thr Gly Leu Val Asn Ile Leu Thr Asp Ile Asn Trp Arg Phe
   95                  100                 105                 110
   act gga gac ttc acg gca cct gac ctg gtt tgc cga gtg gtc cgc tat        506
   Thr Gly Asp Phe Thr Ala Pro Asp Leu Val Cys Arg Val Val Arg Tyr
                   115                 120                 125
   ttg cag gtt gtg ctg ctc tac gcc tct acc tac gtc ctg gtg tcc ctc        554
   Leu Gln Val Val Leu Leu Tyr Ala Ser Thr Tyr Val Leu Val Ser Leu
               130                 135                 140
   agc ata gac aga tac cat gcc atc gtc tac ccc atg aag ttc ctt caa        602
   Ser Ile Asp Arg Tyr His Ala Ile Val Tyr Pro Met Lys Phe Leu Gln
           145                 150                 155
   gga gaa aag caa gcc agg gtc ctc att gtg atc gcc tgg agc ctg tct        650
   Gly Glu Lys Gln Ala Arg Val Leu Ile Val Ile Ala Trp Ser Leu Ser
       160                 165                 170
   ttt ctg ttc tcc att ccc acc ctg atc ata ttt ggg aag agg aca ctg        698
   Phe Leu Phe Ser Ile Pro Thr Leu Ile Ile Phe Gly Lys Arg Thr Leu
   175                 180                 185                 190
   tcc aac ggt gaa gtg cag tgc tgg gcc ctg tgg cct gac gac tcc tac        746
   Ser Asn Gly Glu Val Gln Cys Trp Ala Leu Trp Pro Asp Asp Ser Tyr
                   195                 200                 205
   tgg acc cca tac atg acc atc gtg gcc ttc ctg gtg tac ttc atc cct        794
   Trp Thr Pro Tyr Met Thr Ile Val Ala Phe Leu Val Tyr Phe Ile Pro
               210                 215                 220
   ctg aca atc atc agc atc atg tat ggc att gtg atc cga act att tgg        842
   Leu Thr Ile Ile Ser Ile Met Tyr Gly Ile Val Ile Arg Thr Ile Trp
           225                 230                 235
   att aaa agc aaa acc tac gaa aca gtg att tcc aac tgc tca gat ggg        890
   Ile Lys Ser Lys Thr Tyr Glu Thr Val Ile Ser Asn Cys Ser Asp Gly
       240                 245                 250
   aaa ctg tgc agc agc tat aac cga gga ctc atc tca aag gca aaa atc        938
   Lys Leu Cys Ser Ser Tyr Asn Arg Gly Leu Ile Ser Lys Ala Lys Ile
   255                 260                 265                 270
   aag gct atc aag tat agc atc atc atc att ctt gcc ttc atc tgc tgt        986
   Lys Ala Ile Lys Tyr Ser Ile Ile Ile Ile Leu Ala Phe Ile Cys Cys
                   275                 280                 285
   tgg agt cca tac ttc ctg ttt gac att ttg gac aat ttc aac ctc ctt      1034
   Trp Ser Pro Tyr Phe Leu Phe Asp Ile Leu Asp Asn Phe Asn Leu Leu
               290                 295                 300
   cca gac acc cag gag cgt ttc tat gcc tct gtg atc att cag aac ctg      1082
   Pro Asp Thr Gln Glu Arg Phe Tyr Ala Ser Val Ile Ile Gln Asn Leu
           305                 310                 315
   cca gca ttg aat agt gcc atc aac ccc ctc atc tac tgt gtc ttc agc      1130
   Pro Ala Leu Asn Ser Ala Ile Asn Pro Leu Ile Tyr Cys Val Phe Ser
       320                 325                 330
   agc tcc atc tct ttc ccc tgc agg gag caa aga tca cag gat tcc aga      1178
   Ser Ser Ile Ser Phe Pro Cys Arg Glu Gln Arg Ser Gln Asp Ser Arg
   335                 340                 345                 350
   atg acg ttc cgg gag aga act gag agg cat gag atg cag att ctg tcc      1226
   Met Thr Phe Arg Glu Arg Thr Glu Arg His Glu Met Gln Ile Leu Ser
                   355                 360                 365
   aag cca gaa ttc atc tagaccctag ggcagtgcca gtgctaggct gagcaccatc      1281
   Lys Pro Glu Phe Ile
               370
   agctctccca ggtccttgtc acctgcttgg gcacgtgcat ggaacccgag ccaacttcac   1341
   cccaccctcg tcattacctg ggagatgcac aagacaaatg ttctaatgac tgcatgcact   1401
   gcttaagtat tggccaacac gaactcccca gttattcatg ccagccagga aggaaacgcc   1461
   ttccttcccc accattccca gccctccttc ccactggcca gcacctgaac ccagtgaaca   1521
   caggcattag tggtccaggg tcctggcttg gagccagtga gtagac                   1567
<210> 3
   <211> 371
   <212> PRT
   <213> Homo sapiens
<400> 3
   Met Pro Ala Asn Phe Thr Glu Gly Ser Phe Asp Ser Ser Gly Thr Gly
   1               5                   10                  15
   Gln Thr Leu Asp Ser Ser Pro Val Ala Cys Thr Glu Thr Val Thr Phe
               20                  25                  30
   Thr Glu Val Val Glu Gly Lys Glu Trp Gly Ser Phe Tyr Tyr Ser Phe
           35                  40                  45
   Lys Thr Glu Gln Leu Ile Thr Leu Trp Val Leu Phe Val Phe Thr Ile
       50                  55                  60
   Val Gly Asn Ser Val Val Leu Phe Ser Thr Trp Arg Arg Lys Lys Lys
   65                  70                  75                  80
   Ser Arg Met Thr Phe Phe Val Thr Gln Leu Ala Ile Thr Asp Ser Phe
                   85                  90                  95
   Thr Gly Leu Val Asn Ile Leu Thr Asp Ile Asn Trp Arg Phe Thr Gly
               100                 105                 110
   Asp Phe Thr Ala Pro Asp Leu Val Cys Arg Val Val Arg Tyr Leu Gln
           115                 120                 125
   Val Val Leu Leu Tyr Ala Ser Thr Tyr Val Leu Val Ser Leu Ser Ile
       130                 135                 140
   Asp Arg Tyr His Ala Ile Val Tyr Pro Met Lys Phe Leu Gln Gly Glu
   145                 150                 155                 160
   Lys Gln Ala Arg Val Leu Ile Val Ile Ala Trp Ser Leu Ser Phe Leu
                   165                 170                 175
   Phe Ser Ile Pro Thr Leu Ile Ile Phe Gly Lys Arg Thr Leu Ser Asn
               180                 185                 190
   Gly Glu Val Gln Cys Trp Ala Leu Trp Pro Asp Asp Ser Tyr Trp Thr
           195                 200                 205
   Pro Tyr Met Thr Ile Val Ala Phe Leu Val Tyr Phe Ile Pro Leu Thr
       210                 215                 220
   Ile Ile Ser Ile Met Tyr Gly Ile Val Ile Arg Thr Ile Trp Ile Lys
   225                 230                 235                 240
   Ser Lys Thr Tyr Glu Thr Val Ile Ser Asn Cys Ser Asp Gly Lys Leu
                   245                 250                 255
   Cys Ser Ser Tyr Asn Arg Gly Leu Ile Ser Lys Ala Lys Ile Lys Ala
               260                 265                 270
   Ile Lys Tyr Ser Ile Ile Ile Ile Leu Ala Phe Ile Cys Cys Trp Ser
           275                 280                 285
   Pro Tyr Phe Leu Phe Asp Ile Leu Asp Asn Phe Asn Leu Leu Pro Asp
       290                 295                 300
   Thr Gln Glu Arg Phe Tyr Ala Ser Val Ile Ile Gln Asn Leu Pro Ala
   305                 310                 315                 320
   Leu Asn Ser Ala Ile Asn Pro Leu Ile Tyr Cys Val Phe Ser Ser Ser
                   325                 330                 335
   Ile Ser Phe Pro Cys Arg Glu Gln Arg Ser Gln Asp Ser Arg Met Thr
               340                 345                 350
   Phe Arg Glu Arg Thr Glu Arg His Glu Met Gln Ile Leu Ser Lys Pro
           355                 360                 365
   Glu Phe Ile
       370
<210> 4
   <211> 1410
   <212> DNA
   <213> Homo sapiens
<220>
   <221> CDS
   <222> (129)..(1259)
<400> 4
   gggctcaggg agggctctgt gcctccgttc agcagagctg cagctgctgc ccagctctca      60
   ggaggcaagc tggactccct cactcagctg caggagcaag gacagtgagg ctcaaccccg     120
   cctgagcc atg cca gcc aac ttc aca gag ggc agc ttc gat tcc agt ggg      170
            Met Pro Ala Asn Phe Thr Glu Gly Ser Phe Asp Ser Ser Gly
            1               5                   10
   acc ggg cag acg ctg gat tct tcc cca gtg gct tgc act gaa aca gtg      218
   Thr Gly Gln Thr Leu Asp Ser Ser Pro Val Ala Cys Thr Glu Thr Val
   15                  20                  25                  30
   act ttt act gaa gtg gtg gaa gga aag gaa tgg ggt tcc ttc tac tac      266
   Thr Phe Thr Glu Val Val Glu Gly Lys Glu Trp Gly Ser Phe Tyr Tyr
                   35                  40                  45
   tcc ttt aag act gag caa ttg ata act ctg tgg gtc ctc ttt gtt ttt      314
   Ser Phe Lys Thr Glu Gln Leu Ile Thr Leu Trp Val Leu Phe Val Phe
                   50                  55                  60
   acc att gtt gga aac tcc gtt gtg ctt ttt tcc aca tgg agg aga aag       362
   Thr Ile Val Gly Asn Ser Val Val Leu Phe Ser Thr Trp Arg Arg Lys
           65                  70                  75
   aag aag tca aga atg acc ttc ttt gtg act cag ctg gcc atc aca gat       410
   Lys Lys Ser Arg Met Thr Phe Phe Val Thr Gln Leu Ala Ile Thr Asp
       80                  85                  90
   tct ttc aca gga ctg gtc aac atc ttg aca gat att aat tgg cga ttc       458
   Ser Phe Thr Gly Leu Val Asn Ile Leu Thr Asp Ile Asn Trp Arg Phe
   95                  100                 105                 110
   act gga gac ttc acg gca cct gac ctg gtt tgc cga gtg gtc cgc tat       506
   Thr Gly Asp Phe Thr Ala Pro Asp Leu Val Cys Arg Val Val Arg Tyr
                   115                 120                 125
   ttg cag gtt gtg ctg ctc tac gcc tct acc tac gtc ctg gtg tcc ctc       554
   Leu Gln Val Val Leu Leu Tyr Ala Ser Thr Tyr Val Leu Val Ser Leu
               130                 135                 140
   agc ata gac aga tac cat gcc atc gtc tac ccc atg aag ttc ctt caa       602
   Ser Ile Asp Arg Tyr His Ala Ile Val Tyr Pro Met Lys Phe Leu Gln
           145                 150                 155
   gga gaa aag caa gcc agg gtc ctc att gtg atc gcc tgg agc ctg tct       650
   Gly Glu Lys Gln Ala Arg Val Leu Ile Val Ile Ala Trp Ser Leu Ser
       160                 165                 170
   ttt ctg ttc tcc att ccc acc ctg atc ata ttt ggg aag agg aca ctg       698
   Phe Leu Phe Ser Ile Pro Thr Leu Ile Ile Phe Gly Lys Arg Thr Leu
   175                 180                 185                 190
   tcc aac ggt gaa gtg cag tgc tgg gcc ctg tgg cct gac gac tcc tac       746
   Ser Asn Gly Glu Val Gln Cys Trp Ala Leu Trp Pro Asp Asp Ser Tyr
                   195                 200                 205
   tgg acc cca tac atg acc atc gtg gcc ttc ctg gtg tac ttc atc cct       794
   Trp Thr Pro Tyr Met Thr Ile Val Ala Phe Leu Val Tyr Phe Ile Pro
               210                 215                 220
   ctg aca atc atc agc atc atg tat ggc att gtg atc cga act att tgg       842
   Leu Thr Ile Ile Ser Ile Met Tyr Gly Ile Val Ile Arg Thr Ile Trp
           225                 230                 235
   att aaa agc aaa acc tac gaa aca gtg att tcc aac tgc tca gat ggg       890
   Ile Lys Ser Lys Thr Tyr Glu Thr Val Ile Ser Asn Cys Ser Asp Gly
       240                 245                 250
   aaa ctg tgc agc agc tat aac cga gga ctc atc tca aag gca aaa atc       938
   Lys Leu Cys Ser Ser Tyr Asn Arg Gly Leu Ile Ser Lys Ala Lys Ile
   255                 260                 265                 270
   aag gct atc aag tat agc atc atc atc att ctt gcc ttc atc tgc tgt       986
   Lys Ala Ile Lys Tyr Ser Ile Ile Ile Ile Leu Ala Phe Ile Cys Cys
                   275                 280                 285
   tgg agt cca tac ttc ctg ttt gac att ttg gac aat ttc aac ctc ctt      1034
   Trp Ser Pro Tyr Phe Leu Phe Asp Ile Leu Asp Asn Phe Asn Leu Leu
               290                 295                 300
   cca gac acc cag gag cgt ttc tat gcc tct gtg atc att cag aac ctg      1082
   Pro Asp Thr Gln Glu Arg Phe Tyr Ala Ser Val Ile Ile Gln Asn Leu
           305                 310                 315
   cca gca ttg aat agt gcc atc aac ccc ctc atc tac tgt gtc ttc agc      1130
   Pro Ala Leu Asn Ser Ala Ile Asn Pro Leu Ile Tyr Cys Val Phe Ser
       320                 325                 330
   agc tcc atc tct ttc ccc tgc agg gtc atc cgt ctc cgt cag ctc cag      1178
   Ser Ser Ile Ser Phe Pro Cys Arg Val Ile Arg Leu Arg Gln Leu Gln
   335                 340                 345                 350
   gag gct gcg cta atg ctc tgc cct caa cga gag aac tgg aag ggt act      1226
   Glu Ala Ala Leu Met Leu Cys Pro Gln Arg Glu Asn Trp Lys Gly Thr
                   355                 360                 365
   tgg cca ggt gta cct tcc tgg gct ctt cca agg tgacagctct caccctgtgc    1279
   Trp Pro Gly Val Pro Ser Trp Ala Leu Pro Arg
               370                 375
   tgcaggtggc cctgtgcctg gtgccacttc tcactgctta ccagggcaca aggacaccag    1339
   tggttcccaa aatgggtcac agcaggatgg cctgcatcag attcaccagg gagggctata    1399
   agaaggcaga c                                                          1410
<210> 5
   <211> 377
   <212> PRT
   <213> Homo sapiens
<400> 5
   Met Pro Ala Asn Phe Thr Glu Gly Ser Phe Asp Ser Ser Gly Thr Gly
   1               5                   10                  15
   Gln Thr Leu Asp Ser Ser Pro Val Ala Cys Thr Glu Thr Val Thr Phe
               20                  25                  30
   Thr Glu Val Val Glu Gly Lys Glu Trp Gly Ser Phe Tyr Tyr Ser Phe
           35                  40                  45
   Lys Thr Glu Gln Leu Ile Thr Leu Trp Val Leu Phe Val Phe Thr Ile
       50                  55                  60
   Val Gly Asn Ser Val Val Leu Phe Ser Thr Trp Arg Arg Lys Lys Lys
   65                  70                  75                  80
   Ser Arg Met Thr Phe Phe Val Thr Gln Leu Ala Ile Thr Asp Ser Phe
   85 90 95
   Thr Gly Leu Val Asn Ile Leu Thr Asp Ile Asn Trp Arg Phe Thr Gly
   100 105 110
   Asp Phe Thr Ala Pro Asp Leu Val Cys Arg Val Val Arg Tyr Leu Gln
   115 120 125
   Val Val Leu Leu Tyr Ala Ser Thr Tyr Val Leu Val Ser Leu Ser Ile
   130 135 140
   Asp Arg Tyr His Ala Ile Val Tyr Pro Met Lys Phe Leu Gln Gly Glu
   145 150 155 160
   Lys Gln Ala Arg Val Leu Ile Val Ile Ala Trp Ser Leu Ser Phe Leu
   165 170 175
   Phe Ser Ile Pro Thr Leu Ile Ile Phe Gly Lys Arg Thr Leu Ser Asn
   180 185 190
   Gly Glu Val Gln Cys Trp Ala Leu Trp Pro Asp Asp Ser Tyr Trp Thr
   195 200 205
   Pro Tyr Met Thr Ile Val Ala Phe Leu Val Tyr Phe Ile Pro Leu Thr
   210 215 220
   Ile Ile Ser Ile Met Tyr Gly Ile Val Ile Arg Thr Ile Trp Ile Lys
   225 230 235 240
   Ser Lys Thr Tyr Glu Thr Val Ile Ser Asn Cys Ser Asp Gly Lys Leu
   245 250 255
   Cys Ser Ser Tyr Asn Arg Gly Leu Ile Ser Lys Ala Lys Ile Lys Ala
   260 265 270
   Ile Lys Tyr Ser Ile Ile Ile Ile Leu Ala Phe Ile Cys Cys Trp Ser
   275 280 285
   Pro Tyr Phe Leu Phe Asp Ile Leu Asp Asn Phe Asn Leu Leu Pro Asp
   290 295 300
   Thr Gln Glu Arg Phe Tyr Ala Ser Val Ile Ile Gln Asn Leu Pro Ala
   305 310 315 320
   Leu Asn Ser Ala Ile Asn Pro Leu Ile Tyr Cys Val Phe Ser Ser Ser
   325 330 335
   Ile Ser Phe Pro Cys Arg Val Ile Arg Leu Arg Gln Leu Gln Glu Ala
               340                 345                 350
   Ala Leu Met Leu Cys Pro Gln Arg Glu Asn Trp Lys Gly Thr Trp Pro
           355                 360                 365
   Gly Val Pro Ser Trp Ala Leu Pro Arg
       370                 375
<210> 6
   <211> 1377
   <212> DNA
   <213> Homo sapiens
<220>
   <221> CDS
   <222> (129)..(1226)
<400> 6
   gggctcaggg agggctctgt gcctccgttc agcagagctg cagctgctgc ccagctctca      60
   ggaggcaagc tggactccct cactcagctg caggagcaag gacagtgagg ctcaaccccg     120
   cctgagcc atg cca gcc aac ttc aca gag ggc agc ttc gat tcc agt ggg      170
            Met Pro Ala Asn Phe Thr Glu Gly Ser Phe Asp Ser Ser Gly
            1               5                   10
   acc ggg cag acg ctg gat tct tcc cca gtg gct tgc act gaa aca gtg      218
   Thr Gly Gln Thr Leu Asp Ser Ser Pro Val Ala Cys Thr Glu Thr Val
   15                  20                  25                  30
   act ttt act gaa gtg gtg gaa gga aag gaa tgg ggt tcc ttc tac tac      266
   Thr Phe Thr Glu Val Val Glu Gly Lys Glu Trp Gly Ser Phe Tyr Tyr
                   35                  40                  45
   tcc ttt aag act gag caa ttg ata act ctg tgg gtc ctc ttt gtt ttt      314
   Ser Phe Lys Thr Glu Gln Leu Ile Thr Leu Trp Val Leu Phe Val Phe
               50                  55                  60
   acc att gtt gga aac tcc gtt gtg ctt ttt tcc aca tgg agg aga aag      362
   Thr Ile Val Gly Asn Ser Val Val Leu Phe Ser Thr Trp Arg Arg Lys
           65                  70                  75
   aag aag tca aga atg acc ttc ttt gtg act cag ctg gcc atc aca gat      410
   Lys Lys Ser Arg Met Thr Phe Phe Val Thr Gln Leu Ala Ile Thr Asp
       80                  85                  90
   att aat tgg cga ttc act gga gac ttc acg gca cct gac ctg gtt tgc      458
   Ile Asn Trp Arg Phe Thr Gly Asp Phe Thr Ala Pro Asp Leu Val Cys
   95                  100                 105                 110
   cga gtg gtc cgc tat ttg cag gtt gtg ctg ctc tac gcc tct acc tac      506
   Arg Val Val Arg Tyr Leu Gln Val Val Leu Leu Tyr Ala Ser Thr Tyr
                   115                 120                 125
   gtc ctg gtg tcc ctc agc ata gac aga tac cat gcc atc gtc tac ccc      554
   Val Leu Val Ser Leu Ser Ile Asp Arg Tyr His Ala Ile Val Tyr Pro
               130                 135                 140
   atg aag ttc ctt caa gga gaa aag caa gcc agg gtc ctc att gtg atc      602
   Met Lys Phe Leu Gln Gly Glu Lys Gln Ala Arg Val Leu Ile Val Ile
           145                 150                 155
   gcc tgg agc ctg tct ttt ctg ttc tcc att ccc acc ctg atc ata ttt      650
   Ala Trp Ser Leu Ser Phe Leu Phe Ser Ile Pro Thr Leu Ile Ile Phe
       160                 165                 170
   ggg aag agg aca ctg tcc aac ggt gaa gtg cag tgc tgg gcc ctg tgg      698
   Gly Lys Arg Thr Leu Ser Asn Gly Glu Val Gln Cys Trp Ala Leu Trp
   175                 180                 185                 190
   cct gac gac tcc tac tgg acc cca tac atg acc atc gtg gcc ttc ctg      746
   Pro Asp Asp Ser Tyr Trp Thr Pro Tyr Met Thr Ile Val Ala Phe Leu
                   195                 200                 205
   gtg tac ttc atc cct ctg aca atc atc agc atc atg tat ggc att gtg      794
   Val Tyr Phe Ile Pro Leu Thr Ile Ile Ser Ile Met Tyr Gly Ile Val
               210                 215                 220
   atc cga act att tgg att aaa agc aaa acc tac gaa aca gtg att tcc      842
   Ile Arg Thr Ile Trp Ile Lys Ser Lys Thr Tyr Glu Thr Val Ile Ser
           225                 230                 235
   aac tgc tca gat ggg aaa ctg tgc agc agc tat aac cga gga ctc atc      890
   Asn Cys Ser Asp Gly Lys Leu Cys Ser Ser Tyr Asn Arg Gly Leu Ile
       240                 245                 250
   tca aag gca aaa atc aag gct atc aag tat agc atc atc atc att ctt      938
   Ser Lys Ala Lys Ile Lys Ala Ile Lys Tyr Ser Ile Ile Ile Ile Leu
   255                 260                 265                 270
   gcc ttc atc tgc tgt tgg agt cca tac ttc ctg ttt gac att ttg gac      986
   Ala Phe Ile Cys Cys Trp Ser Pro Tyr Phe Leu Phe Asp Ile Leu Asp
                   275                 280                 285
   aat ttc aac ctc ctt cca gac acc cag gag cgt ttc tat gcc tct gtg     1034
   Asn Phe Asn Leu Leu Pro Asp Thr Gln Glu Arg Phe Tyr Ala Ser Val
               290                 295                 300
   atc att cag aac ctg cca gca ttg aat agt gcc atc aac ccc ctc atc     1082
   Ile Ile Gln Asn Leu Pro Ala Leu Asn Ser Ala Ile Asn Pro Leu Ile
           305                 310                 315
   tac tgt gtc ttc agc agc tcc atc tct ttc ccc tgc agg gtc atc cgt     1130
   Tyr Cys Val Phe Ser Ser Ser Ile Ser Phe Pro Cys Arg Val Ile Arg
       320                 325                 330
   ctc cgt cag ctc cag gag gct gcg cta atg ctc tgc cct caa cga gag     1178
   Leu Arg Gln Leu Gln Glu Ala Ala Leu Met Leu Cys Pro Gln Arg Glu
   335                 340                 345                 350
   aac tgg aag ggt act tgg cca ggt gta cct tcc tgg gct ctt cca agg      1226
   Asn Trp Lys Gly Thr Trp Pro Gly Val Pro Ser Trp Ala Leu Pro Arg
                   355                 360                 365
   tgacagctct caccctgtgc tgcaggtggc cctgtgcctg gtgccacttc tcactgctta    1286
   ccagggcaca aggacaccag tggttcccaa aatgggtcac agcaggatgg cctgcatcag    1346
   attcaccagg gagggctata agaaggcaga c                                   1377
<210> 7
   <211> 366
   <212> PRT
   <213> Homo sapiens
<400> 7
   Met Pro Ala Asn Phe Thr Glu Gly Ser Phe Asp Ser Ser Gly Thr Gly
   1               5                   10                  15
   Gln Thr Leu Asp Ser Ser Pro Val Ala Cys Thr Glu Thr Val Thr Phe
               20                  25                  30
   Thr Glu Val Val Glu Gly Lys Glu Trp Gly Ser Phe Tyr Tyr Ser Phe
           35                  40                  45
   Lys Thr Glu Gln Leu Ile Thr Leu Trp Val Leu Phe Val Phe Thr Ile
       50                  55                  60
   Val Gly Asn Ser Val Val Leu Phe Ser Thr Trp Arg Arg Lys Lys Lys
   65                  70                  75                  80
   Ser Arg Met Thr Phe Phe Val Thr Gln Leu Ala Ile Thr Asp Ile Asn
                   85                  90                  95
   Trp Arg Phe Thr Gly Asp Phe Thr Ala Pro Asp Leu Val Cys Arg Val
               100                 105                 110
   Val Arg Tyr Leu Gln Val Val Leu Leu Tyr Ala Ser Thr Tyr Val Leu
           115                 120                 125
   Val Ser Leu Ser Ile Asp Arg Tyr His Ala Ile Val Tyr Pro Met Lys
       130                 135                 140
   Phe Leu Gln Gly Glu Lys Gln Ala Arg Val Leu Ile Val Ile Ala Trp
   145 150 155 160
   Ser Leu Ser Phe Leu Phe Ser Ile Pro Thr Leu Ile Ile Phe Gly Lys
   165 170 175
   Arg Thr Leu Ser Asn Gly Glu Val Gln Cys Trp Ala Leu Trp Pro Asp
   180 185 190
   Asp Ser Tyr Trp Thr Pro Tyr Met Thr Ile Val Ala Phe Leu Val Tyr
   195 200 205
   Phe Ile Pro Leu Thr Ile Ile Ser Ile Met Tyr Gly Ile Val Ile Arg
   210 215 220
   Thr Ile Trp Ile Lys Ser Lys Thr Tyr Glu Thr Val Ile Ser Asn Cys
   225 230 235 240
   Ser Asp Gly Lys Leu Cys Ser Ser Tyr Asn Arg Gly Leu Ile Ser Lys
   245 250 255
   Ala Lys Ile Lys Ala Ile Lys Tyr Ser Ile Ile Ile Ile Leu Ala Phe
   260 265 270
   Ile Cys Cys Trp Ser Pro Tyr Phe Leu Phe Asp Ile Leu Asp Asn Phe
   275 280 285
   Asn Leu Leu Pro Asp Thr Gln Glu Arg Phe Tyr Ala Ser Val Ile Ile
   290 295 300
   Gln Asn Leu Pro Ala Leu Asn Ser Ala Ile Asn Pro Leu Ile Tyr Cys
   305 310 315 320
   Val Phe Ser Ser Ser Ile Ser Phe Pro Cys Arg Val Ile Arg Leu Arg
   325 330 335
   Gln Leu Gln Glu Ala Ala Leu Met Leu Cys Pro Gln Arg Glu Asn Trp
   340 345 350
   Lys Gly Thr Trp Pro Gly Val Pro Ser Trp Ala Leu Pro Arg
   355 360 365
<210> 8
   <211> 814
   <212> DNA
   <213> Homo sapiens
<220>
   <221> CDS
   <222> (129)..(410)
<400> 8
   gggctcaggg agggctctgt gcctccgttc agcagagctg cagctgctgc ccagctctca      60
   ggaggcaagc tggactccct cactcagctg caggagcaag gacagtgagg ctcaaccccg     120
   cctgagcc atg cca gcc aac ttc aca gag ggc agc ttc gat tcc agt ggg      170
            Met Pro Ala Asn Phe Thr Glu Gly Ser Phe Asp Ser Ser Gly
            1               5                   10
   acc ggg cag acg ctg gat tct tcc cca gtg gct tgc act gaa aca gtg      218
   Thr Gly Gln Thr Leu Asp Ser Ser Pro Val Ala Cys Thr Glu Thr Val
   15                  20                  25                  30
   act ttt act gaa gtg gtg gaa gga aag gaa tgg ggt tcc ttc tac tac      266
   Thr Phe Thr Glu Val Val Glu Gly Lys Glu Trp Gly Ser Phe Tyr Tyr
                   35                  40                  45
   tcc ttt aag act gag caa ttg ata act ctg tgg gtc ctc ttt gtt ttt      314
   Ser Phe Lys Thr Glu Gln Leu Ile Thr Leu Trp Val Leu Phe Val Phe
               50                  55                  60
   acc att gtt gga aac tcc gtt gtg ctt ttt tcc aca tgg agg aga aag      362
   Thr Ile Val Gly Asn Ser Val Val Leu Phe Ser Thr Trp Arg Arg Lys
           65                  70                  75
   aag aag tca aga atg acc ttc ttt gtg act cag ctg gcc atc aca gta      410
   Lys Lys Ser Arg Met Thr Phe Phe Val Thr Gln Leu Ala Ile Thr Val
       80                  85                  90
   taacaagccc acctgcttga gctgggctgc agtggccagg gtaaacatcc aaggcaccag     470
   tgaaaaatac agagaaggta aaaggagcaa gagttctgaa gatggaacct gggatggggg     530
   aaagtttctt caatctttcc taccaacaag aactccaatt tttcactcct ataaccgtag     590
   aagtagaggt aattaggatc atccagcaaa tgcttagagg caaatatccc tggatgagga     650
   tgccacagct tattttcatt atatttcttc gattacagtg tggtaatgca tgttgtatgg     710
   aactacatat tctttcagaa tgaaaggatt tagaggtggc aagaatatca gcttgaaatt     770
   taaagttttt tcataaacaa taaacaaatg ataattgaaa attc                      814
<210> 9
   <211> 94
   <212> PRT
   <213> Homo sapiens
<400> 9
   Met Pro Ala Asn Phe Thr Glu Gly Ser Phe Asp Ser Ser Gly Thr Gly
   1               5                   10                  15
   Gln Thr Leu Asp Ser Ser Pro Val Ala Cys Thr Glu Thr Val Thr Phe
               20                  25                  30
   Thr Glu Val Val Glu Gly Lys Glu Trp Gly Ser Phe Tyr Tyr Ser Phe
           35                  40                  45
   Lys Thr Glu Gln Leu Ile Thr Leu Trp Val Leu Phe Val Phe Thr Ile
       50                  55                  60
   Val Gly Asn Ser Val Val Leu Phe Ser Thr Trp Arg Arg Lys Lys Lys
   65                  70                  75                  80
   Ser Arg Met Thr Phe Phe Val Thr Gln Leu Ala Ile Thr Val
                   85                  90
<210> 10
   <211> 1463
   <212> DNA
   <213> Homo sapiens
<220>
   <221> CDS
   <222> (129)..(602)
<400> 10
   gggctcaggg agggctctgt gcctccgttc agcagagctg cagctgctgc ccagctctca      60
   ggaggcaagc tggactccct cactcagctg caggagcaag gacagtgagg ctcaaccccg     120
   cctgagcc atg cca gcc aac ttc aca gag ggc agc ttc gat tcc agt ggg      170
            Met Pro Ala Asn Phe Thr Glu Gly Ser Phe Asp Ser Ser Gly
            1               5                   10
   acc ggg cag acg ctg gat tct tcc cca gtg gct tgc act gaa aca gtg      218
   Thr Gly Gln Thr Leu Asp Ser Ser Pro Val Ala Cys Thr Glu Thr Val
   15                  20                  25                  30
   act ttt act gaa gtg gtg gaa gga aag gaa tgg ggt tcc ttc tac tac      266
   Thr Phe Thr Glu Val Val Glu Gly Lys Glu Trp Gly Ser Phe Tyr Tyr
                   35                  40                  45
   tcc ttt aag act gag caa ttg ata act ctg tgg gtc ctc ttt gtt ttt      314
   Ser Phe Lys Thr Glu Gln Leu Ile Thr Leu Trp Val Leu Phe Val Phe
               50                  55                  60
   acc att gtt gga aac tcc gtt gtg ctt ttt tcc aca tgg agg aga aag      362
   Thr Ile Val Gly Asn Ser Val Val Leu Phe Ser Thr Trp Arg Arg Lys
           65                  70                  75
   aag aag tca aga atg acc ttc ttt gtg act cag ctg gcc atc aca ggt      410
   Lys Lys Ser Arg Met Thr Phe Phe Val Thr Gln Leu Ala Ile Thr Gly
       80                  85                  90
   tgt gct gct cta cgc ctc tac cta cgt cct ggt gtc cct cag cat aga      458
   Cys Ala Ala Leu Arg Leu Tyr Leu Arg Pro Gly Val Pro Gln His Arg
   95                  100                 105                 110
   cag ata cca tgc cat cgt cta ccc cat gaa gtt cct tca agg aga aaa      506
   Gln Ile Pro Cys His Arg Leu Pro His Glu Val Pro Ser Arg Arg Lys
                   115                 120                 125
   gca agc cag ggt cct cat tgt gat cgc ctg gag cct gtc ttt tct gtt      554
   Ala Ser Gln Gly Pro His Cys Asp Arg Leu Glu Pro Val Phe Ser Val
               130                 135                 140
   ctc cat tcc cac cct gat cat att tgg gaa gag gac act gtc caa cgg      602
   Leu His Ser His Pro Asp His Ile Trp Glu Glu Asp Thr Val Gln Arg
           145                 150                 155
   tgaagtgcag tgctgggccc tgtggcctga cgactcctac tggaccccat acatgaccat    662
   cgtggccttc ctggtgtact tcatccctct gacaatcatc agcatcatgt atggcattgt    722
   gatccgaact atttggatta aaagcaaaac ctacgaaaca gtgatttcca actgctcaga    782
   tgggaaactg tgcagcagct ataaccgagg actcatctca aaggcaaaaa tcaaggctat    842
   caagtatagc atcatcatca ttcttgcctt catctgctgt tggagtccat acttcctgtt    902
   tgacattttg gacaatttca acctccttcc agacacccag gagcgtttct atgcctctgt    962
   gatcattcag aacctgccag cattgaatag tgccatcaac cccctcatct actgtgtctt   1022
   cagcagctcc atctctttcc cctgcaggga gcaaagatca caggattcca gaatgacgtt   1082
   ccgggagaga actgagaggc atgagatgca gattctgtcc aagccagaat tcatctagac   1142
   cctagggcag tgccagtgct aggctgagca ccatcagctc tcccaggtcc ttgtcacctg   1202
   cttgggcacg tgcatggaac ccgagccaac ttcaccccac cctcgtcatt acctgggaga   1262
   tgcacaagac aaatgttcta atgactgcat gcactgctta agtattggcc aacacgaact   1322
   ccccagttat tcatgccagc caggaaggaa acgccttcct tccccaccat tcccagccct   1382
   ccttcccact ggccagcacc tgaacccagt gaacacaggc attagtggtc cagggtcctg 1442
   gcttggagcc agtgagtaga c 1463
<210> 11
   <211> 158
   <212> PRT
   <213> Homo sapiens
<400> 11
   Met Pro Ala Asn Phe Thr Glu Gly Ser Phe Asp Ser Ser Gly Thr Gly
   1 5 10 15
   Gln Thr Leu Asp Ser Ser Pro Val Ala Cys Thr Glu Thr Val Thr Phe
   20 25 30
   Thr Glu Val Val Glu Gly Lys Glu Trp Gly Ser Phe Tyr Tyr Ser Phe
   35 40 45
   Lys Thr Glu Gln Leu Ile Thr Leu Trp Val Leu Phe Val Phe Thr Ile
   50 55 60
   Val Gly Asn Ser Val Val Leu Phe Ser Thr Trp Arg Arg Lys Lys Lys
   65 70 75 80
   Ser Arg Met Thr Phe Phe Val Thr Gln Leu Ala Ile Thr Gly Cys Ala
   85 90 95
   Ala Leu Arg Leu Tyr Leu Arg Pro Gly Val Pro Gln His Arg Gln Ile
   100 105 110
   Pro Cys His Arg Leu Pro His Glu Val Pro Ser Arg Arg Lys Ala Ser
   115 120 125
   Gln Gly Pro His Cys Asp Arg Leu Glu Pro Val Phe Ser Val Leu His
   130 135 140
   Ser His Pro Asp His Ile Trp Glu Glu Asp Thr Val Gln Arg
   145 150 155
<211> 1473
   <212> DNA
   <213> Homo sapiens
<220>
   <221> CDS
   <222> (129)..(536)
<400> 12
<210> 13
   <211> 136
   <212> PRT
   <213> Homo sapiens
<400> 13
   Met Pro Ala Asn Phe Thr Glu Gly Ser Phe Asp Ser Ser Gly Thr Gly
   1               5                   10                  15
   Gln Thr Leu Asp Ser Ser Pro Val Ala Cys Thr Glu Thr Val Thr Phe
               20                  25                  30
   Thr Glu Val Val Glu Gly Lys Glu Trp Gly Ser Phe Tyr Tyr Ser Phe
           35                  40                  45
   Lys Thr Glu Gln Leu Ile Thr Leu Trp Val Leu Phe Val Phe Thr Ile
       50                  55                  60
   Val Gly Asn Ser Val Val Leu Phe Ser Thr Trp Arg Arg Lys Lys Lys
   65                  70                  75                  80
   Ser Arg Met Thr Phe Phe Val Thr Gln Leu Ala Ile Thr Asp Ser Phe
                   85                  90                  95
   Thr Gly Leu Val Asn Ile Leu Thr Asp Ile Asn Trp Arg Phe Thr Gly
               100                 105                 110
   Asp Phe Thr Ala Pro Asp Leu Val Cys Arg Val Val Arg Tyr Leu Gln
           115                 120                 125
   Lys Ser Lys Pro Gly Ser Ser Leu
       130                 135
<210> 14
   <211> 1369
   <212> DNA
   <213> Homo sapiens
<220>
   <221> CDS
   <222> (129)..(1043)
<400> 14
   gggctcaggg agggctctgt gcctccgttc agcagagctg cagctgctgc ccagctctca      60
   ggaggcaagc tggactccct cactcagctg caggagcaag gacagtgagg ctcaaccccg     120
   cctgagcc atg cca gcc aac ttc aca gag ggc agc ttc gat tcc agt ggg      170
            Met Pro Ala Asn Phe Thr Glu Gly Ser Phe Asp Ser Ser Gly
            1               5                   10
   acc ggg cag acg ctg gat tct tcc cca gtg gct tgc act gaa aca gtg      218
   Thr Gly Gln Thr Leu Asp Ser Ser Pro Val Ala Cys Thr Glu Thr Val
   15                  20                  25                  30
   act ttt act gaa gtg gtg gaa gga aag gaa tgg ggt tcc ttc tac tac      266
   Thr Phe Thr Glu Val Val Glu Gly Lys Glu Trp Gly Ser Phe Tyr Tyr
                   35                  40                  45
   tcc ttt aag act gag caa ttg ata act ctg tgg gtc ctc ttt gtt ttt      314
   Ser Phe Lys Thr Glu Gln Leu Ile Thr Leu Trp Val Leu Phe Val Phe
               50                  55                  60
   acc att gtt gga aac tcc gtt gtg ctt ttt tcc aca tgg agg aga aag      362
   Thr Ile Val Gly Asn Ser Val Val Leu Phe Ser Thr Trp Arg Arg Lys
           65                  70                  75
   aag aag tca aga atg acc ttc ttt gtg act cag ctg gcc atc aca gaa      410
   Lys Lys Ser Arg Met Thr Phe Phe Val Thr Gln Leu Ala Ile Thr Glu
       80                  85                  90
   aag caa gcc agg gtc ctc att gtg atc gcc tgg agc ctg tct ttt ctg        458
   Lys Gln Ala Arg Val Leu Ile Val Ile Ala Trp Ser Leu Ser Phe Leu
   95                  100                 105                 110
   ttc tcc att ccc acc ctg atc ata ttt ggg aag agg aca ctg tcc aac        506
   Phe Ser Ile Pro Thr Leu Ile Ile Phe Gly Lys Arg Thr Leu Ser Asn
                   115                 120                 125
   ggt gaa gtg cag tgc tgg gcc ctg tgg cct gac gac tcc tac tgg acc        554
   Gly Glu Val Gln Cys Trp Ala Leu Trp Pro Asp Asp Ser Tyr Trp Thr
               130                 135                 140
   cca tac atg acc atc gtg gcc ttc ctg gtg tac ttc atc cct ctg aca        602
   Pro Tyr Met Thr Ile Val Ala Phe Leu Val Tyr Phe Ile Pro Leu Thr
           145                 150                 155
   atc atc agc atc atg tat ggc att gtg atc cga act att tgg att aaa        650
   Ile Ile Ser Ile Met Tyr Gly Ile Val Ile Arg Thr Ile Trp Ile Lys
       160                 165                 170
   agc aaa acc tac gaa aca gtg att tcc aac tgc tca gat ggg aaa ctg        698
   Ser Lys Thr Tyr Glu Thr Val Ile Ser Asn Cys Ser Asp Gly Lys Leu
   175                 180                 185                 190
   tgc agc agc tat aac cga gga ctc atc tca aag gca aaa atc aag gct        746
   Cys Ser Ser Tyr Asn Arg Gly Leu Ile Ser Lys Ala Lys Ile Lys Ala
                   195                 200                 205
   atc aag tat agc atc atc atc att ctt gcc ttc atc tgc tgt tgg agt        794
   Ile Lys Tyr Ser Ile Ile Ile Ile Leu Ala Phe Ile Cys Cys Trp Ser
               210                 215                 220
   cca tac ttc ctg ttt gac att ttg gac aat ttc aac ctc ctt cca gac        842
   Pro Tyr Phe Leu Phe Asp Ile Leu Asp Asn Phe Asn Leu Leu Pro Asp
           225                 230                 235
   acc cag gag cgt ttc tat gcc tct gtg atc att cag aac ctg cca gca        890
   Thr Gln Glu Arg Phe Tyr Ala Ser Val Ile Ile Gln Asn Leu Pro Ala
       240                 245                 250
   ttg aat agt gcc atc aac ccc ctc atc tac tgt gtc ttc agc agc tcc        938
   Leu Asn Ser Ala Ile Asn Pro Leu Ile Tyr Cys Val Phe Ser Ser Ser
   255                 260                 265                 270
   atc tct ttc ccc tgc agg gag caa aga tca cag gat tcc aga atg acg        986
   Ile Ser Phe Pro Cys Arg Glu Gln Arg Ser Gln Asp Ser Arg Met Thr
                   275                 280                 285
   ttc cgg gag aga act gag agg cat gag atg cag att ctg tcc aag cca       1034
   Phe Arg Glu Arg Thr Glu Arg His Glu Met Gln Ile Leu Ser Lys Pro
               290                 295                 300
   gaa ttc atc tagaccctag ggcagtgcca gtgctaggct gagcaccatc               1083
   Glu Phe Ile
           305
   agctctccca ggtccttgtc acctgcttgg gcacgtgcat ggaacccgag ccaacttcac   1143
   cccaccctcg tcattacctg ggagatgcac aagacaaatg ttctaatgac tgcatgcact   1203
   gcttaagtat tggccaacac gaactcccca gttattcatg ccagccagga aggaaacgcc 1263
   ttccttcccc accattccca gccctccttc ccactggcca gcacctgaac ccagtgaaca 1323
   caggcattag tggtccaggg tcctggcttg gagccagtga gtagac 1369
<210> 15
   <211> 305
   <212> PRT
   <213> Homo sapiens
<400> 15
   Met Pro Ala Asn Phe Thr Glu Gly Ser Phe Asp Ser Ser Gly Thr Gly
   1               5                   10                  15
   Gln Thr Leu Asp Ser Ser Pro Val Ala Cys Thr Glu Thr Val Thr Phe
               20                  25                  30
   Thr Glu Val Val Glu Gly Lys Glu Trp Gly Ser Phe Tyr Tyr Ser Phe
           35                  40                  45
   Lys Thr Glu Gln Leu Ile Thr Leu Trp Val Leu Phe Val Phe Thr Ile
       50                  55                  60
   Val Gly Asn Ser Val Val Leu Phe Ser Thr Trp Arg Arg Lys Lys Lys
   65                  70                  75                  80
   Ser Arg Met Thr Phe Phe Val Thr Gln Leu Ala Ile Thr Glu Lys Gln
                   85                  90                  95
   Ala Arg Val Leu Ile Val Ile Ala Trp Ser Leu Ser Phe Leu Phe Ser
               100                 105                 110
   Ile Pro Thr Leu Ile Ile Phe Gly Lys Arg Thr Leu Ser Asn Gly Glu
           115                 120                 125
   Val Gln Cys Trp Ala Leu Trp Pro Asp Asp Ser Tyr Trp Thr Pro Tyr
       130                 135                 140
   Met Thr Ile Val Ala Phe Leu Val Tyr Phe Ile Pro Leu Thr Ile Ile
   145                 150                 155                 160
   Ser Ile Met Tyr Gly Ile Val Ile Arg Thr Ile Trp Ile Lys Ser Lys
   165 170 175
   Thr Tyr Glu Thr Val Ile Ser Asn Cys Ser Asp Gly Lys Leu Cys Ser
   180 185 190
   Ser Tyr Asn Arg Gly Leu Ile Ser Lys Ala Lys Ile Lys Ala Ile Lys
   195 200 205
   Tyr Ser Ile Ile Ile Ile Leu Ala Phe Ile Cys Cys Trp Ser Pro Tyr
   210 215 220
   Phe Leu Phe Asp Ile Leu Asp Asn Phe Asn Leu Leu Pro Asp Thr Gln
   225 230 235 240
   Glu Arg Phe Tyr Ala Ser Val Ile Ile Gln Asn Leu Pro Ala Leu Asn
   245 250 255
   Ser Ala Ile Asn Pro Leu Ile Tyr Cys Val Phe Ser Ser Ser Ile Ser
   260 265 270
   Phe Pro Cys Arg Glu Gln Arg Ser Gln Asp Ser Arg Met Thr Phe Arg
   275 280 285
   Glu Arg Thr Glu Arg His Glu Met Gln Ile Leu Ser Lys Pro Glu Phe
   290 295 300
   Ile
   305
<210> 16
   <211> 1532
   <212> DNA
   <213> Homo sapiens
<220>
   <221> CDS
   <222> (231)..(383)
<400> 16
   gacacagaga agaccactcc cacctccccg agtgcaaggt gtgaagggac agatctttta 60
   accatgcctg cccccttata cttgctgttc atagaattgc aactgaaagt gaccatgagg 120
   atccactgga tggagttact tctttcttaa gtgaggaggc taagatctgg agtgacttct     180
   ccccagattt ttgtatacct gactctgttt cagcatccgc ttcccaaaga atg cag        236
                                                          Met Gln
                                                          1
   tgt gaa gca gga gct tat gtg aga aga aac gca ggg aga cag ttc agt       284
   Cys Glu Ala Gly Ala Tyr Val Arg Arg Asn Ala Gly Arg Gln Phe Ser
           5                   10                  15
   cac tgc aat ctt cat gcc cat cag ttt ctt gtg aga aga aaa caa gag       332
   His Cys Asn Leu His Ala His Gln Phe Leu Val Arg Arg Lys Gln Glu
       20                  25                  30
   ctc caa tct gtt aga tgg tat ttt gaa gca ggt ctt tgg gta aag gac       380
   Leu Gln Ser Val Arg Trp Tyr Phe Glu Ala Gly Leu Trp Val Lys Asp
   35                  40                  45                  50
   acc tagacccagt gaaggtcatg gtgattatta ttggacaatg ggacatcact            433
   Thr
   ctgctatttg aacaaataag actttttcct gacatgcatc tggaggcagg tcaaagctcc    493
   aggccaactc caagtttctg atggggtctc tagccaatgg aaggcttctt ctccttcaat     553
   tgcctgactc ttcaggactc ttaaatactg caaagtgaga aaatgagaca ggttgcactg     613
   agggctgtta gccagacaga gtctcgaact ggaagtccat ctagatgttt tgcataagag     673
   aatggaaaca atctgtctgt gatttaggga catactctgg cagcaatatg ggaatacagt     733
   ttcaatcctc attaacaaaa caggtatgaa atacatattt atttagtaag gtgccagctg     793
   tatgaaaaat ccatttctta tttcccataa tgtttctgaa atgtcttagc agtgcataga     853
   gacagcatgt catcattttc tagggactgt gtgttattgc atttttccta gggaagatct     913
   tttctaggtc acctgctcct tcgctaaagc tctgaccaat ctagcttgct aacctgtgac     973
   tccattttcc taagtcctga gagagaaaaa cgctttgcag caaattatgc caggcatcct    1033
   tgtgtctaaa tgaaaaaagg aaaaagcctc cttccttccc tctgttgaga agtgcacggt    1093
   ccacatatgc atgcacagca tatactgtga gggtatttgc agtcccttgg gttgctttga    1153
   taactggcca ggttgctgtt ctatttttcc acattctatt aatcctccta caggcagtta    1213
   ttaggtattg agtgctcaca cacccctggc atagtcacca catgccatta gctccagata    1273
   aacttccaga aaaaagtcca tcccccactt ctctcagctg cctgccaacg ctggacacct    1333
   tctcaccaag ccagcaggac agagaaaagc ctgggcttta agatcaaaca aacacagctt    1393
   caaattagga ctctgtcact tcctgtgtac tgggcacttt gctgagtatg tggtttctca    1453
   tctgtaaaac agagaaagat gattatctcc caatctttct atgttatatg tttgaattaa    1513
   ataaggtact ctccatgaa                                                  1532
<210> 17
   <211> 51
   <212> PRT
   <213> Homo sapiens
<400> 17
   Met Gln Cys Glu Ala Gly Ala Tyr Val Arg Arg Asn Ala Gly Arg Gln
   1               5                   10                  15
   Phe Ser His Cys Asn Leu His Ala His Gln Phe Leu Val Arg Arg Lys
               20                  25                  30
   Gln Glu Leu Gln Ser Val Arg Trp Tyr Phe Glu Ala Gly Leu Trp Val
           35                  40                  45
   Lys Asp Thr
       50
<210> 18
   <211> 1407
   <212> DNA
   <213> Homo sapiens
<220>
   <221> CDS
   <222> (244) .. (402)
<400> 18
   gacacagaga agaccactcc cacctccccg agtgcaaggt gtgaagggac agatctttta      60
   accatgcctg cccccttata cttgctgttc atagaattgc aactgaaagt gaccatgagg      120
   atccactgga tggagttact tctttcttaa gtgaggaggc taagatctga gttcttcaca     180
   tctctctgta gataaaattt ccggtctggt ttcacattcc tctgtcagaa gaactttctt     240
   taa tgt ttc tta aag tac agg tct gct gct tat gtg aga aga aac gca       288
       Cys Phe Leu Lys Tyr Arg Ser Ala Ala Tyr Val Arg Arg Asn Ala
       1               5                   10                  15
   ggg aga cag ttc agt cac tgc aat ctt cat gcc cat cag ttt ctt gtg       336
   Gly Arg Gln Phe Ser His Cys Asn Leu His Ala His Gln Phe Leu Val
                   20                  25                  30
   aga aga aaa caa gag ctc caa tct gtt aga tgg tat ttt gaa gca ggt      384
   Arg Arg Lys Gln Glu Leu Gln Ser Val Arg Trp Tyr Phe Glu Ala Gly
               35                  40                  45
   ctt tgg gta aag gac acc tagacccagt gaaggtcatg gtgattatta             432
   Leu Trp Val Lys Asp Thr
           50
   ttggacaatg ggacatcact ctgctattaa gtgagaaaat gagacaggtt gcactgaggg    492
   ctgttagcca gacagagtct cgaactggaa gtccatctag atgttttgca taagagaatg    552
   gaaacaatct gtctgtgatt tagggacata ctctggcagc aatatgggaa tacagtttca    612
   atcctcatta acaaaacagg tatgaaatac atatttattt agtaaggtgc cagctgtatg    672
   aaaaatccat ttcttatttc ccataatgtt tctgaaatgt cttagcagtg catagagaca    732
   gcatgtcatc attttctagg gactgtgtgt tattgcattt ttcctaggga agatcttttc    792
   taggtcacct gctccttcgc taaagctctg accaatctag cttgctaacc tgtgactcca    852
   ttttcctaag tcctgagaga gaaaaacgct ttgcagcaaa ttatgccagg catccttgtg    912
   tctaaatgaa aaaaggaaaa agcctccttc cttccctctg ttgagaagtg cacggtccac    972
   atatgcatgc acagcatata ctgtgagggt atttgcagtc ccttgggttg ctttgataac   1032
   tggccaggtt gctgttctat ttttccacat tctattaatc ctcctacagg cagttattag   1092
   gtattgagtg ctcacacacc cctggcatag tcaccacatg ccattagctc cagataaact   1152
   tccagaaaaa agtccatccc ccacttctct cagctgcctg ccaacgctgg acaccttctc   1212
   accaagccag caggacagag aaaagcctgg gctttaagat caaacaaaca cagcttcaaa   1272
   ttaggactct gtcacttcct gtgtactggg cactttgctg agtatgtggt ttctcatctg   1332
   taaaacagag aaagatgatt atctcccaat ctttctatgt tatatgtttg aattaaataa   1392
   ggtactctcc atgaa                                                    1407
<210> 19
   <211> 53
   <212> PRT
   <213> Homo sapiens
<400> 19
   Cys Phe Leu Lys Tyr Arg Ser Ala Ala Tyr Val Arg Arg Asn Ala Gly
   1               5                   10                  15
   Arg Gln Phe Ser His Cys Asn Leu His Ala His Gln Phe Leu Val Arg
               20                  25                  30
   Arg Lys Gln Glu Leu Gln Ser Val Arg Trp Tyr Phe Glu Ala Gly Leu
           35                  40                  45
   Trp Val Lys Asp Thr
       50
<210> 20
   <211> 341
   <212> DNA
   <213> Homo sapiens
<220>
   <221> CDS
   <222> (146)..(328)
<400> 20
   gctgatggtg gaaggagaat gagtctctga tgcctttgga cttgatgctg gaaagactta      60
   agactttggg ggactactgg aaaggagtga cttctcccca gatttttgta tacctgactc     120
   tgtttcagca tccgcttccc aaaga atg cag tgt gaa gca gga gct tat gtg       172
                               Met Gln Cys Glu Ala Gly Ala Tyr Val
                               1               5
   aga aga aac gca ggg aga cag ttc agt cac tgc aat ctt cat gcc cat       220
   Arg Arg Asn Ala Gly Arg Gln Phe Ser His Cys Asn Leu His Ala His
   10                  15                  20                  25
   cag ttt ctt gtg aga aga aaa caa gtg gat ata cac tgt tcc aag cag       268
   Gln Phe Leu Val Arg Arg Lys Gln Val Asp Ile His Cys Ser Lys Gln
                   30                  35                  40
   cat gtg ttg aaa aga ttt gtc ttt tcc cca ttt aat ggt ctt ggt acc       316
   His Val Leu Lys Arg Phe Val Phe Ser Pro Phe Asn Gly Leu Gly Thr
               45                  50                  55
   ttt ctc aaa aat tgaccatata tga                                        341
   Phe Leu Lys Asn
           60
<210> 21
   <211> 61
   <212> PRT
   <213> Homo sapiens
<400> 21
   Met Gln Cys Glu Ala Gly Ala Tyr Val Arg Arg Asn Ala Gly Arg Gln
   1               5                   10                  15
   Phe Ser His Cys Asn Leu His Ala His Gln Phe Leu Val Arg Arg Lys
               20                  25                  30
   Gln Val Asp Ile His Cys Ser Lys Gln His Val Leu Lys Arg Phe Val
           35                  40                  45
   Phe Ser Pro Phe Asn Gly Leu Gly Thr Phe Leu Lys Asn
       50                  55                  60
<210> 22
   <211> 710
   <212> DNA
   <213> Homo sapiens
<220>
   <221> CDS
   <222> (175)..(291)
<400> 22
   tctaggactc agaaatatag atgttagtaa gagcaaacag acataacaga taacacatac      60
   aaagtgccta ccacatgcta accactgctg caggcacttt ctatagaaga actaatttaa     120
   tcatcaccat aaccctatgg ggtagatgat atttttacaa cctccatttt acag atg       177
                                                               Met
                                                               1
   aag aaa ctg aag cat aga cct gct tat gtg aga aga aac gca ggg aga       225
   Lys Lys Leu Lys His Arg Pro Ala Tyr Val Arg Arg Asn Ala Gly Arg
               5                   10                  15
   cag ttc agt cac tgc aat ctt cat gcc cat cag ttt ctt gtg aga aga       273
   Gln Phe Ser His Cys Asn Leu His Ala His Gln Phe Leu Val Arg Arg
           20                  25                  30
   aaa caa gaa aac aag gac tgaaatccac acaggaaggt ggcagtgaac              321
   Lys Gln Glu Asn Lys Asp
   35
   tccacagacg gacctggacg cctcaacact cctggcctta cctcccttgc tgaacgtctc 381
   aagtttctct gcgttcagga ctggcaacgc ctgcttcctc ctctgagctg tcaagtagga 441
   agtccgggct gctctgctag aaagagaagt catgtgcagg agcactgagg catcccaggt 501
   gtgacactct tccacctaga gcattccgtc tctcatcctc tgccatgtga cgctgggctt 561
   ctttaacaaa ttaatcccaa gtgcaagaca tttatttctt ctgtacctaa tgacctgagc 621
   aatccttctc tgctgaacct ggtagtgtca tctttagaag tgaagacaca attaacacat 681
   ggtcatttct tcattatatc gttgttact 710
<210> 23
   <211> 39
   <212> PRT
   <213> Homo sapiens
<400> 23
   Met Lys Lys Leu Lys His Arg Pro Ala Tyr Val Arg Arg Asn Ala Gly
   1 5 10 15
   Arg Gln Phe Ser His Cys Asn Leu His Ala His Gln Phe Leu Val Arg
   20 25 30
   Arg Lys Gln Glu Asn Lys Asp
   35
<210> 24
   <211> 949
   <212> DNA
   <213> Homo sapiens
<220>
   <221> CDS
   <222> (175)..(366)
<400> 24
   tctaggactc agaaatatag atgttagtaa gagcaaacag acataacaga taacacatac 60
   aaagtgccta ccacatgcta accactgctg caggcacttt ctatagaaga actaatttaa      120
   tcatcaccat aaccctatgg ggtagatgat atttttacaa cctccatttt acag atg        177
                                                               Met
                                                               1
   aag aaa ctg aag cat aga cct gct tat gtg aga aga aac gca ggg aga        225
   Lys Lys Leu Lys His Arg Pro Ala Tyr Val Arg Arg Asn Ala Gly Arg
               5                   10                  15
   cag ttc agt cac tgc aat ctt cat gcc cat cag ttt ctt gtg aga aga        273
   Gln Phe Ser His Cys Asn Leu His Ala His Gln Phe Leu Val Arg Arg
           20                  25                  30
   aaa caa gac tgg caa cgc ctg ctt cct cct ctg agc tgt caa gta gga        321
   Lys Gln Asp Trp Gln Arg Leu Leu Pro Pro Leu Ser Cys Gln Val Gly
       35                  40                  45
   agt ccg ggc tgc tct gct aga aag aga agt cat gtg cag gag cac            366
   Ser Pro Gly Cys Ser Ala Arg Lys Arg Ser His Val Gln Glu His
   50                  55                  60
   tgaggcatcc caggtgtgac actcttccac ctagagcatt ccgtctctca tcctctgcca      426
   tgtagcaaac tgctatgcat ccttcagctg caagggattg aatgctatca acaaccatac      486
   aagtggagaa gcagatgctt ccctagctga gcctcaggct ttttgatgga attgctacaa      546
   cttggtgcat gcctgctcct aaaagaaata ctcaggaatt gtctcataaa gtcctcacct      606
   actggcaaaa acaagatgtt ctactcccag gttgactttt tcaagcccca agatgttgag      666
   tcagccattc tccaaggatc tcgatttcct tttaatggaa aataacatta aacaccaaat      726
   ataagcctcg ctgtcccaca tgcgtattgg ggacaagatg aaacctgctt ccaggctact      786
   ttggcagcag aactgaaaaa ggcttttttt ccagatatat gatttctcat cgacagggtt      846
   gcacagccct ctttattgtt cgtgtaaatg acacccttgg atctgaacaa tacacaccag      906
   gacaattgtg tgcaacagtt ctacaaactg atatttctaa tta                        949
<210> 25
   <211> 64
   <212> PRT
   <213> Homo sapiens
<400> 25
   Met Lys Lys Leu Lys His Arg Pro Ala Tyr Val Arg Arg Asn Ala Gly
   1               5                   10                  15
   Arg Gln Phe Ser His Cys Asn Leu His Ala His Gln Phe Leu Val Arg
   20 25 30
   Arg Lys Gln Asp Trp Gln Arg Leu Leu Pro Pro Leu Ser Cys Gln Val
   35 40 45
   Gly Ser Pro Gly Cys Ser Ala Arg Lys Arg Ser His Val Gln Glu His
   50 55 60
<210> 26
   <211> 1068
   <212> DNA
   <213> Homo sapiens
<220>
   <221> CDS
   <222> (176)..(367)
<400> 26
   gtctaggact cagaaatata gatgttagta agagcaaaca gacataacag ataacacata      60
   caaagtgcct accacatgct aaccactgct gcaggcactt tctatagaag aactaattta     120
   atcatcacca taaccctatg gggtagatga tatttttaca acctccattt tacag atg      178
                                                                Met
                                                                1
   aag aaa ctg aag cat aga cct gct tat gtg aga aga aac gca ggg aga      226
   Lys Lys Leu Lys His Arg Pro Ala Tyr Val Arg Arg Asn Ala Gly Arg
               5                   10                  15
   cag ttc agt cac tgc aat ctt cat gcc cat cag ttt ctt gtg aga aga      274
   Gln Phe Ser His Cys Asn Leu His Ala His Gln Phe Leu Val Arg Arg
           20                  25                  30
   aaa caa gac tgg caa cgc ctg ctt cct cct ctg agc tgt caa gta gga      322
   Lys Gln Asp Trp Gln Arg Leu Leu Pro Pro Leu Ser Cys Gln Val Gly
       35                  40                  45
   agt ccg ggc tgc tct gct aga aag aga agt cat gtg cag gag cac          367
   Ser Pro Gly Cys Ser Ala Arg Lys Arg Ser His Val Gln Glu His
   50                  55                  60
   tgaggcatcc caggtgtgac actcttccac ctagagcatt ccgtctctca tcctctgcca     427
   tgtagcaaac tgctatgcat ccttcagctg caagggattg aatgctatca acaaccatac     487
   aagtggagaa gcagatgctt ccctagctga gcctcagctc tgttttcctc agtcctaggg     547
   gcagattcta atttggtaca gaaaaaaaaa gtactcctgc caattcccat cctggaatga     607
   ctgaagaaaa gaaaatgcat gtgattattt taaaggcttt ttgatggaat tgctacaact    667
   tggtgcatgc ctgctcctaa aagaaatact caggaattgt ctcataaagt cctcacctac    727
   tggcaaaaac aagatgttct actcccaggt tgactttttc aagccccaag atgttgagtc    787
   agccattctc caaggatctc gatttccttt taatggaaaa taacattaaa caccaaatat    847
   aagcctcgct gtcccacatg cgtattgggg acaagatgaa acctgcttcc aggctacttt    907
   ggcagcagaa ctgaaaaagg ctttttttcc agatatatga tttctcatcg acagggttgc    967
   acagccctct ttattgttcg tgtaaatgac acccttggat ctgaacaata cacaccagga   1027
   caattgtgtg caacagttct acaaactgat atttctaatt a                       1068
<210> 27
   <211> 64
   <212> PRT
   <213> Homo sapiens
<400> 27
   Met Lys Lys Leu Lys His Arg Pro Ala Tyr Val Arg Arg Asn Ala Gly
   1               5                   10                  15
   Arg Gln Phe Ser His Cys Asn Leu His Ala His Gln Phe Leu Val Arg
               20                  25                  30
   Arg Lys Gln Asp Trp Gln Arg Leu Leu Pro Pro Leu Ser Cys Gln Val
           35                  40                  45
   Gly Ser Pro Gly Cys Ser Ala Arg Lys Arg Ser His Val Gln Glu His
       50                  55                  60
<210> 28
   <211> 799
   <212> DNA
   <213> Homo sapiens
<220>
   <221> CDS
   <222> (175)..(363)
<400> 28
   tctaggactc agaaatatag atgttagtaa gagcaaacag acataacaga taacacatac      60
   aaagtgccta ccacatgcta accactgctg caggcacttt ctatagaaga actaatttaa     120
   tcatcaccat aaccctatgg ggtagatgat atttttacaa cctccatttt acag atg       177
                                                               Met
                                                               1
   aag aaa ctg aag cat aga cct gct tat gtg aga aga aac gca ggg aga       225
   Lys Lys Leu Lys His Arg Pro Ala Tyr Val Arg Arg Asn Ala Gly Arg
               5                   10                  15
   cag ttc agt cac tgc aat ctt cat gcc cat cag ttt ctt gtg aga aga       273
   Gln Phe Ser His Cys Asn Leu His Ala His Gln Phe Leu Val Arg Arg
           20                  25                  30
   aaa caa agc aaa ctg cta tgc atc ctt cag ctg caa ggg att gaa tgc       321
   Lys Gln Ser Lys Leu Leu Cys Ile Leu Gln Leu Gln Gly Ile Glu Cys
       35                  40                  45
   tat caa caa cca tac aag tgg aga agc aga tgc ttc cct agc               363
   Tyr Gln Gln Pro Tyr Lys Trp Arg Ser Arg Cys Phe Pro Ser
   50                  55                  60
   tgagcctcag gctttttgat ggaattgcta caacttggtg catgcctgct cctaaaagaa     423
   atactcagga attgtctcat aaagtcctca cctactggca aaaacaagat gttctactcc     483
   caggttgact ttttcaagcc ccaagatgtt gagtcagcca ttctccaagg atctcgattt      543
   ccttttaatg gaaaataaca ttaaacacca aatataagcc tcgctgtccc acatgcgtat     603
   tggggacaag atgaaacctg cttccaggct actttggcag cagaactgaa aaaggctttt     663
   tttccagata tatgatttct catcgacagg gttgcacagc cctctttatt gttcgtgtaa     723
   atgacaccct tggatctgaa caatacacac caggacaatt gtgtgcaaca gttctacaaa     783
   ctgatatttc taatta                                                     799
<210> 29
   <211> 63
   <212> PRT
   <213> Homo sapiens
<400> 29
   Met Lys Lys Leu Lys His Arg Pro Ala Tyr Val Arg Arg Asn Ala Gly
   1               5                   10                  15
   Arg Gln Phe Ser His Cys Asn Leu His Ala His Gln Phe Leu Val Arg
               20                  25                  30
   Arg Lys Gln Ser Lys Leu Leu Cys Ile Leu Gln Leu Gln Gly Ile Glu
           35                  40                  45
   Cys Tyr Gln Gln Pro Tyr Lys Trp Arg Ser Arg Cys Phe Pro Ser
       50                  55                  60
<210> 30
   <211> 834
   <212> DNA
   <213> Homo sapiens
<220>
   <221> CDS
   <222> (30) .. (251)
<400> 30
   gctgatggtg gaaggagaat gagtctctg atg cct ttg gac ttg atg ctg gaa       53
                                   Met Pro Leu Asp Leu Met Leu Glu
                                   1               5
   aga ctt aag act ttg ggg gac tac tgg aaa gct tat gtg aga aga aac      101
   Arg Leu Lys Thr Leu Gly Asp Tyr Trp Lys Ala Tyr Val Arg Arg Asn
       10                  15                  20
   gca ggg aga cag ttc agt cac tgc aat ctt cat gcc cat cag ttt ctt      149
   Ala Gly Arg Gln Phe Ser His Cys Asn Leu His Ala His Gln Phe Leu
   25                  30                  35                  40
   gtg aga aga aaa caa gac tgg caa cgc ctg ctt cct cct ctg agc tgt      197
   Val Arg Arg Lys Gln Asp Trp Gln Arg Leu Leu Pro Pro Leu Ser Cys
                   45                  50                  55
   caa gta gga agt ccg ggc tgc tct gct aga aag aga agt cat gtg cag      245
   Gln Val Gly Ser Pro Gly Cys Ser Ala Arg Lys Arg Ser His Val Gln
               60                  65                  70
   gag cac tgaggcatcc caggtgtgac actcttccac ctagagcatt ccgtctctca       301
   Glu His
   tcctctgcca tgtagcaaac tgctatgcat ccttcagctg caagggattg aatgctatca    361
   acaaccatac aagtggagaa gcagatgctt ccctagctga gcctcaggct ttttgatgga     421
   attgctacaa cttggtgcat gcctgctcct aaaagaaata ctcaggaatt gtctcataaa    481
   gtcctcacct actggcaaaa acaagatgtt ctactcccag gttgactttt tcaagcccca 541
   agatgttgag tcagccattc tccaaggatc tcgatttcct tttaatggaa aataacatta 601
   aacaccaaat ataagcctcg ctgtcccaca tgcgtattgg ggacaagatg aaacctgctt 661
   ccaggctact ttggcagcag aactgaaaaa ggcttttttt ccagatatat gatttctcat 721
   cgacagggtt gcacagccct ctttattgtt cgtgtaaatg acacccttgg atctgaacaa 781
   tacacaccag gacaattgtg tgcaacagtt ctacaaactg atatttctaa tta 834
<210> 31
   <211> 74
   <212> PRT
   <213> Homo sapiens
<400> 31
   Met Pro Leu Asp Leu Met Leu Glu Arg Leu Lys Thr Leu Gly Asp Tyr
   1 5 10 15
   Trp Lys Ala Tyr Val Arg Arg Asn Ala Gly Arg Gln Phe Ser His Cys
   20 25 30
   Asn Leu His Ala His Gln Phe Leu Val Arg Arg Lys Gln Asp Trp Gln
   35 40 45
   Arg Leu Leu Pro Pro Leu Ser Cys Gln Val Gly Ser Pro Gly Cys Ser
   50 55 60
   Ala Arg Lys Arg Ser His Val Gln Glu His
   65 70
<210> 32
   <211> 550
   <212> DNA
   <213> Homo sapiens
<220>
   <221> CDS
   <222> (175) .. (366)
<400> 32
   tctaggactc agaaatatag atgttagtaa gagcaaacag acataacaga taacacatac      60
   aaagtgccta ccacatgcta accactgctg caggcacttt ctatagaaga actaatttaa     120
   tcatcaccat aaccctatgg ggtagatgat atttttacaa cctccatttt acag atg       177
                                                               Met
                                                               1
   aag aaa ctg aag cat aga cct gct tat gtg aga aga aac gca ggg aga       225
   Lys Lys Leu Lys His Arg Pro Ala Tyr Val Arg Arg Asn Ala Gly Arg
               5                   10                  15
   cag ttc agt cac tgc aat ctt cat gcc cat cag ttt ctt gtg aga aga       273
   Gln Phe Ser His Cys Asn Leu His Ala His Gln Phe Leu Val Arg Arg
           20                  25                  30
   aaa caa gac tgg caa cgc ctg ctt cct cct ctg agc tgt caa gta gga       321
   Lys Gln Asp Trp Gln Arg Leu Leu Pro Pro Leu Ser Cys Gln Val Gly
       35                  40                  45
   agt ccg ggc tgc tct gct aga aag aga agt cat gtg cag gag cac           366
   Ser Pro Gly Cys Ser Ala Arg Lys Arg Ser His Val Gln Glu His
   50                  55                  60
   tgaggcatcc caggtgtgac actcttccac ctagagcatt ccgtctctca tcctctgcca     426
   tgtgccatgt tttgaaccac tagattagag ggtcaagcaa tttcttggaa ttttactctg     486
   aattctacgt agaccatttt catgtgtata cctcctctga gtcaccctca ggtagggaca     546
   tttt                                                                  550
<210> 33
   <211> 64
   <212> PRT
   <213> Homo sapiens
<400> 33
   Met Lys Lys Leu Lys His Arg Pro Ala Tyr Val Arg Arg Asn Ala Gly
   1               5                   10                  15
   Arg Gln Phe Ser His Cys Asn Leu His Ala His Gln Phe Leu Val Arg
               20                  25                  30
   Arg Lys Gln Asp Trp Gln Arg Leu Leu Pro Pro Leu Ser Cys Gln Val
           35                  40                  45
   Gly Ser Pro Gly Cys Ser Ala Arg Lys Arg Ser His Val Gln Glu His
       50                  55                  60
<210> 34
   <211> 376
   <212> DNA
   <213> Homo sapiens
<220>
   <221> CDS
   <222> (175)..(363)
<400> 34
   tctaggactc agaaatatag atgttagtaa gagcaaacag acataacaga taacacatac     60
   aaagtgccta ccacatgcta accactgctg caggcacttt ctatagaaga actaatttaa    120
   tcatcaccat aaccctatgg ggtagatgat atttttacaa cctccatttt acag atg      177
                                                               Met
                                                               1
   aag aaa ctg aag cat aga cct gct tat gtg aga aga aac gca ggg aga      225
   Lys Lys Leu Lys His Arg Pro Ala Tyr Val Arg Arg Asn Ala Gly Arg
               5                   10                  15
   cag ttc agt cac tgc aat ctt cat gcc cat cag ttt ctt gtg aga aga      273
   Gln Phe Ser His Cys Asn Leu His Ala His Gln Phe Leu Val Arg Arg
           20                  25                  30
   aaa caa gtg gat ata cac tgt tcc aag cag cat gtg ttg aaa aga ttt      321
   Lys Gln Val Asp Ile His Cys Ser Lys Gln His Val Leu Lys Arg Phe
       35                  40                  45
   gtc ttt tcc cca ttt aat ggt ctt ggt acc ttt ctc aaa aat              363
   Val Phe Ser Pro Phe Asn Gly Leu Gly Thr Phe Leu Lys Asn
   50                  55                  60
   tgaccatata tga                                                       376
<210> 35
   <211> 63
   <212> PRT
   <213> Homo sapiens
<210> 36
   <211> 369
   <212> DNA
   <213> Homo sapiens
<220>
   <221> CDS
   <222> (146)..(247)
<400> 36
   gctgatggtg gaaggagaat gagtctctga tgcctttgga cttgatgctg gaaagactta      60
   agactttggg ggactactgg aaaggagtga cttctcccca gatttttgta tacctgactc     120
   tgtttcagca tccgcttccc aaaga atg cag tgt gaa gca gga gct tat gtg      172
                               Met Gln Cys Glu Ala Gly Ala Tyr Val
                               1               5
   aga aga aac gca ggg aga cag ttc agt cac tgc aat ctt cat gcc cat      220
   Arg Arg Asn Ala Gly Arg Gln Phe Ser His Cys Asn Leu His Ala His
   10                  15                  20                  25
   cag ttt ctt gtg aga aga aaa caa gtt taggaaaact tcctacacct            267
   Gln Phe Leu Val Arg Arg Lys Gln Val
                   30
   tctttgttgg gatgttctct ggactaatga ctccaggcga gaccaccgtt gatcatgaac     327
   tcactttgaa acagaagctg ggttggtaag actggagcta ct                        369
<210> 37
   <211> 34
   <212> PRT
   <213> Homo sapiens
<400> 37
   Met Gln Cys Glu Ala Gly Ala Tyr Val Arg Arg Asn Ala Gly Arg Gln
   1               5                   10                  15
   Phe Ser His Cys Asn Leu His Ala His Gln Phe Leu Val Arg Arg Lys
               20                  25                  30
   Gln Val
<210> 38
   <211> 512
   <212> DNA
   <213> Homo sapiens
<220>
   <221> CDS
   <222> (62) .. (172)
<400> 38
   gagtgacttc tccccagatt tttgtatacc tgactctgtt tcagcatccg cttcccaaag     60
   a atg cag tgt gaa gca gga gct tat gtg aga aga aac gca ggg aga cag    109
   ttc agt cac tgc aat ctt cat gcc cat cag ttt ctt gtg aga aga aaa      157
   caa gaa aac aag gac tgaaatccac acaggaaggt ggcagtgaac tccacagacg      212
   gacctggacg cctcaacact cctggcctta cctcccttgc tgaacgtctc aagtttctct    272
   gcgttcaggt aatgtatagg agggttatga gggcagagaa ttcctaagct cattagtaaa    332
   ttgctcttca gaaaagtgct ttgaagcaaa gctaatttcc tttcccaata tgagaagatt    392
   tggccctacc agaaaaagga aatgatttga atgtgcgcca aaaaatatgt tttctttctt    452
   ttctttgttg aacactcatc gggagttact cttattagtt ccgcattttt attgccattt    512
<210> 39
   <211> 37
   <212> PRT
   <213> Homo sapiens
<400> 39
<210> 40
   <211> 830
   <212> DNA
   <213> Homo sapiens
<220>
   <221> CDS
   <222> (62)..(247)
<400> 40
   gagtgacttc tccccagatt tttgtatacc tgactctgtt tcagcatccg cttcccaaag      60
   a atg cag tgt gaa gca gga gct tat gtg aga aga aac gca ggg aga cag     109
     Met Gln Cys Glu Ala Gly Ala Tyr Val Arg Arg Asn Ala Gly Arg Gln
     1               5                   10                  15
   ttc agt cac tgc aat ctt cat gcc cat cag ttt ctt gtg aga aga aaa       157
   Phe Ser His Cys Asn Leu His Ala His Gln Phe Leu Val Arg Arg Lys
               20                  25                  30
   caa gac tgg caa cgc ctg ctt cct cct ctg agc tgt caa gta gga agt       205
   Gln Asp Trp Gln Arg Leu Leu Pro Pro Leu Ser Cys Gln Val Gly Ser
           35                  40                  45
   ccg ggc tgc tct gct aga aag aga agt cat gtg cag gag cac               247
   Pro Gly Cys Ser Ala Arg Lys Arg Ser His Val Gln Glu His
       50                  55                  60
   tgaggcatcc caggtgtgac actcttccac ctagagcatt ccgtctctca tcctctgcca     307
   tgtagcaaac tgctatgcat ccttcagctg caagggattg aatgctatca acaaccatac     367
   aagtggagaa gcagatgctt ccctagctga gcctcaggct ttttgatgga attgctacaa      427
   cttggtgcat gcctgctcct aaaagaaata ctcaggaatt gtctcataaa gtcctcacct     487
   actggcaaaa acaagatgtt ctactcccag gttgactttt tcaagcccca agatgttgag     547
   tcagccattc tccaaggatc tcgatttcct tttaatggaa aataacatta aacaccaaat     607
   ataagcctcg ctgtcccaca tgcgtattgg ggacaagatg aaacctgctt ccaggctact     667
   ttggcagcag aactgaaaaa ggcttttttt ccagatatat gatttctcat cgacagggtt     727
   gcacagccct ctttattgtt cgtgtaaatg acacccttgg atctgaacaa tacacaccag     787
   gacaattgtg tgcaacagtt ctacaaactg atatttctaa tta                       830
<210> 41
   <211> 62
   <212> PRT
   <213> Homo sapiens
<400> 41
   Met Gln Cys Glu Ala Gly Ala Tyr Val Arg Arg Asn Ala Gly Arg Gln
   1               5                   10                  15
   Phe Ser His Cys Asn Leu His Ala His Gln Phe Leu Val Arg Arg Lys
               20                  25                  30
   Gln Asp Trp Gln Arg Leu Leu Pro Pro Leu Ser Cys Gln Val Gly Ser
           35                  40                  45
   Pro Gly Cys Ser Ala Arg Lys Arg Ser His Val Gln Glu His
       50                  55                  60

## Description

### FIELD OF THE INVENTION

The present invention resides in the field of molecular genetics.

### BACKGROUND OF THE INVENTION

We have previously mapped a susceptibility locus for asthma and high total serum Immunoglobulin E level (IgE) to a large region spanning ~19 cM of chromosome 7p15-p14. This locus was the only locus that reached genome wide significance level among the Finnish asthma families (Laitinen, 2001). Linkage was further confirmed among the French-Canadian families recruited based on asthma and in another independent data set from Finland recruited based on high IgE level (Laitinen, 2001). Daniels *et al.* (1996) have reported among Australian and British families six regions of possible linkage including 7p14-p15 and established by simulations that at least some of them are likely to be true positives. Fine mapping of the region revealed bimodal linkage to bronchial hyperreactivity and blood eosinophil count at D7S484 (P=0.0003) and D7S669 (P=0.006) 63 cM apart (Leaves *et al.* 2002). German (Wjst *et al.* 1999), French (Dizier *et al.* 2000), and Italian families (Malerba *et al.* 2000) have shown some evidence of linkage, but there are also genome scans with inconclusive results (Ober *et al.* 2000; Xu *et al.* 2000; Mathias *et al.* 2001), some of which have been done in ethnic populations other than Caucasian (Yokouchi *et al.* 2000; *Xu et al.* 2001). By comparing genome scans in different immune disorders, it has been suggested that clinically distinct autoimmune diseases may be controlled by a common set of susceptibility genes (Becker *et al.* 1998). 7p15-p14 has also been linked to diseases such as multiple sclerosis (Sawcer *et al.* 1996) and inflammatory bowel disease (Satsangi *et al.* 1996), and genomic regions homologous to human 7p15-p14 have been linked to insulin dependent diabetes (Jacob *et al.* 1992) and inflammatory arthritis (Remmers *et al.* 1996) in rat models.

### SUMMARY OF THE INVENTION

The invention provides an method of determining risk of asthma, or other IgE mediated disease. The method comprises determining whether the individual has a variant polymorphic form wherein presence of the variant polymorphic form indicates risk of asthma, or other IgE mediated disease.

The invention further provides a kit for use in diagnosing or assessing predisposition to asthma, or other IgE-mediated disease.

### BRIEF DESCRIPTION OF THE DRAWINGS

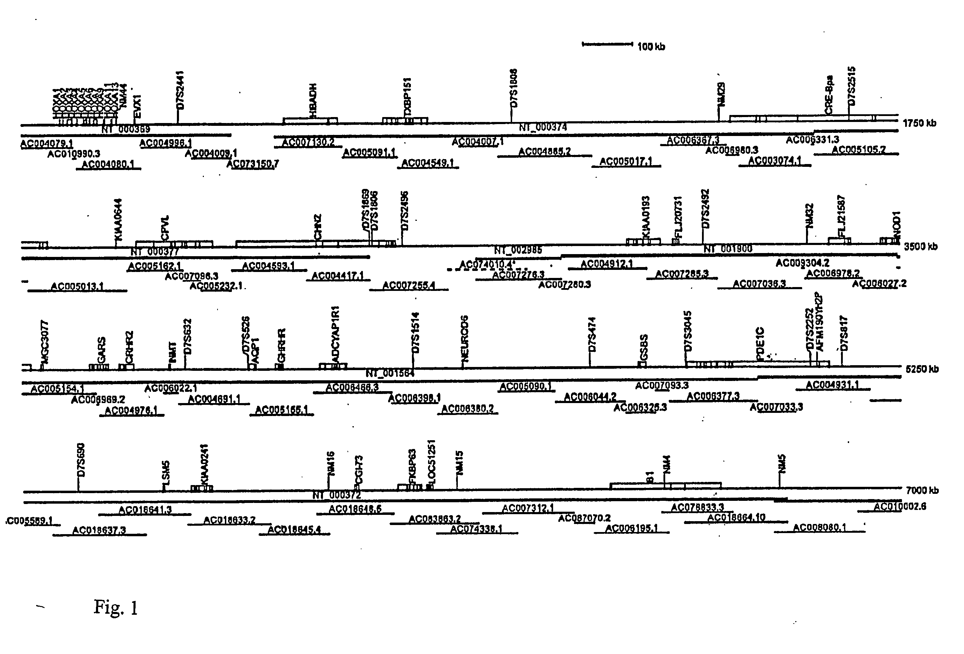

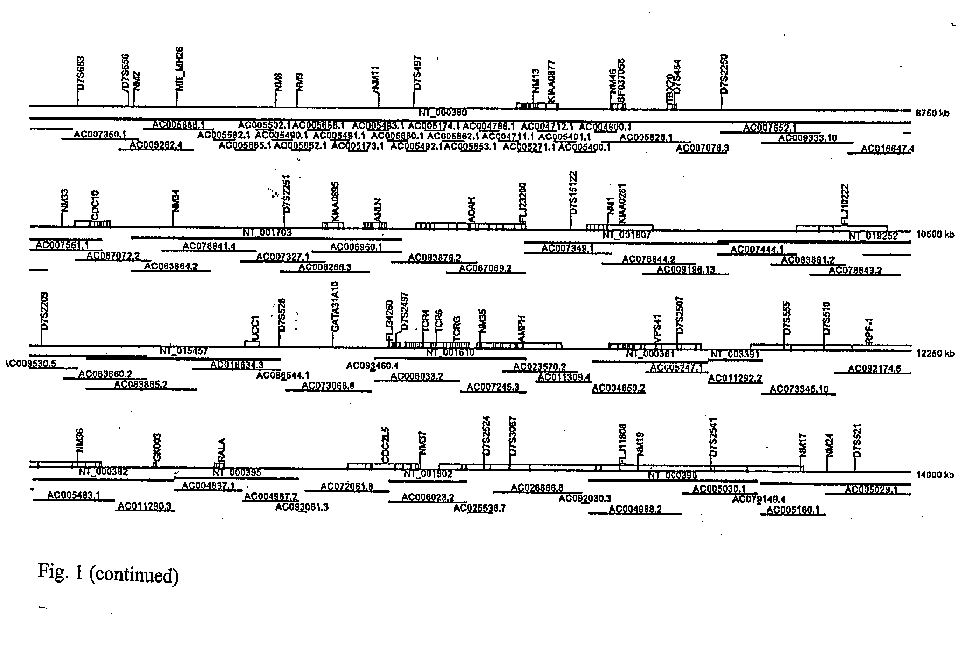

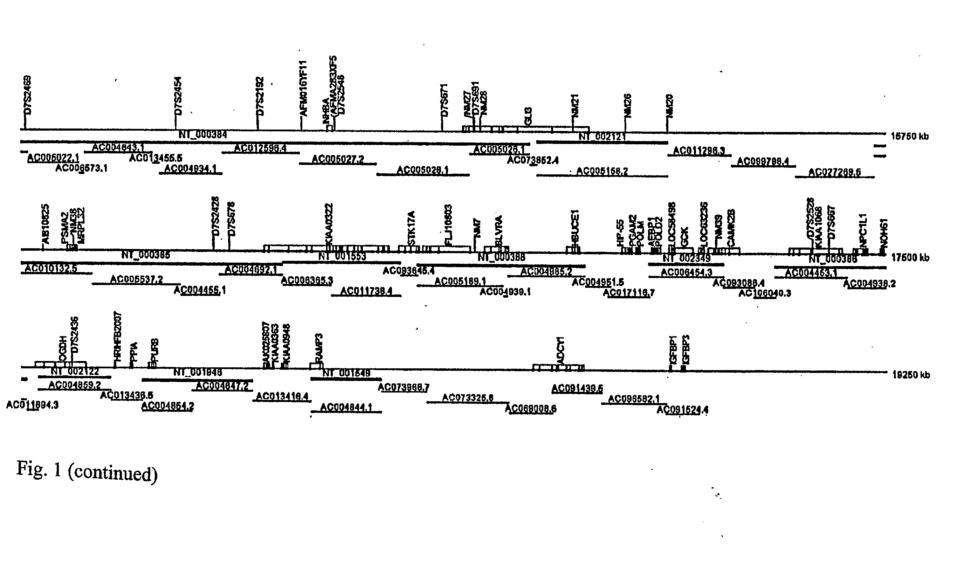

**Fig. 1****:** Physical map across the linkage region showing the organization of exons of known genes and microsatellite markers genotyped (above), and the organization of genomic BAC clones and contigs (below).

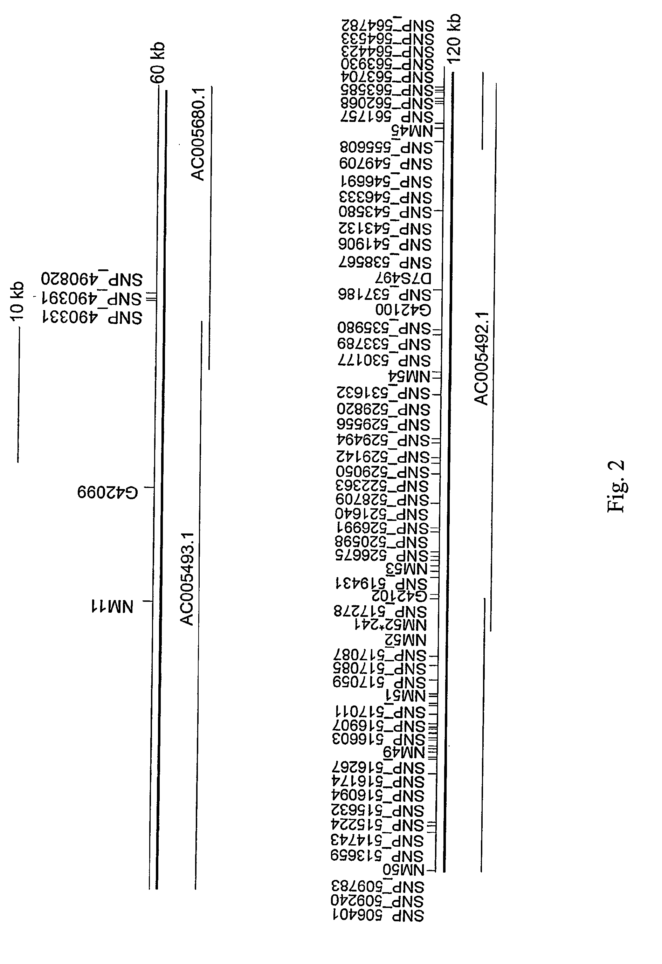

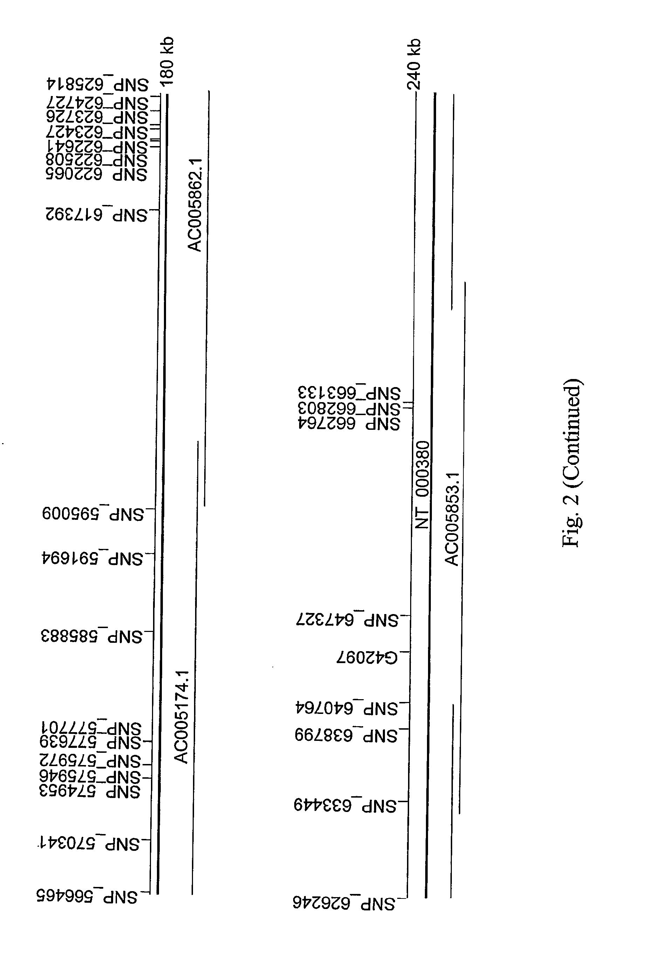

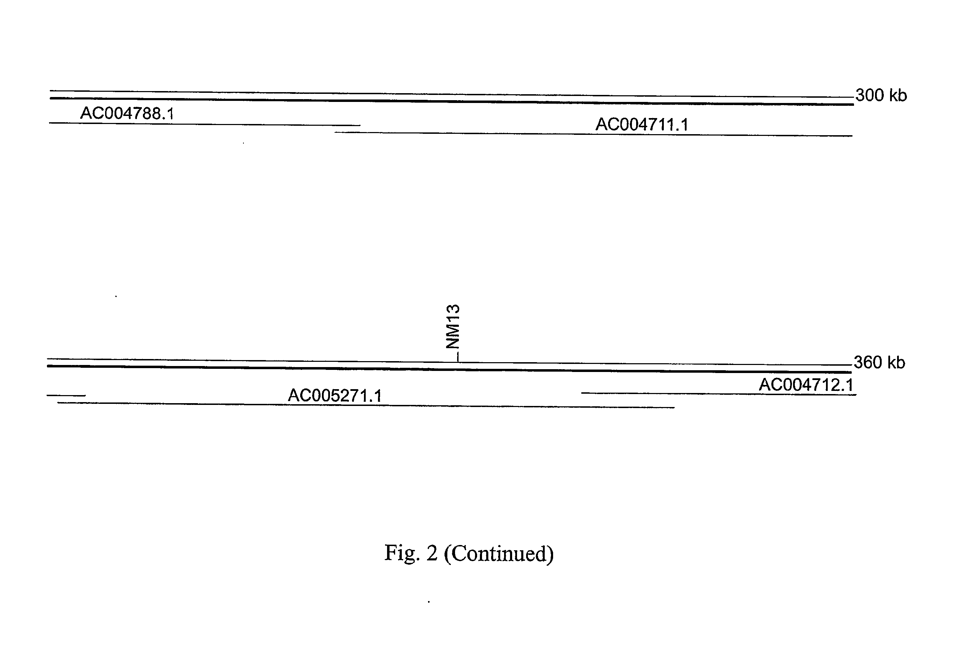

**Fig. 2****:** Physical map across the critical (SNP509783-SNP638799, a total of 129,017 bp) and the flanking regions showing the organization of all markers genotyped in phase three (above), and the organization of genomic BAC clones (below).

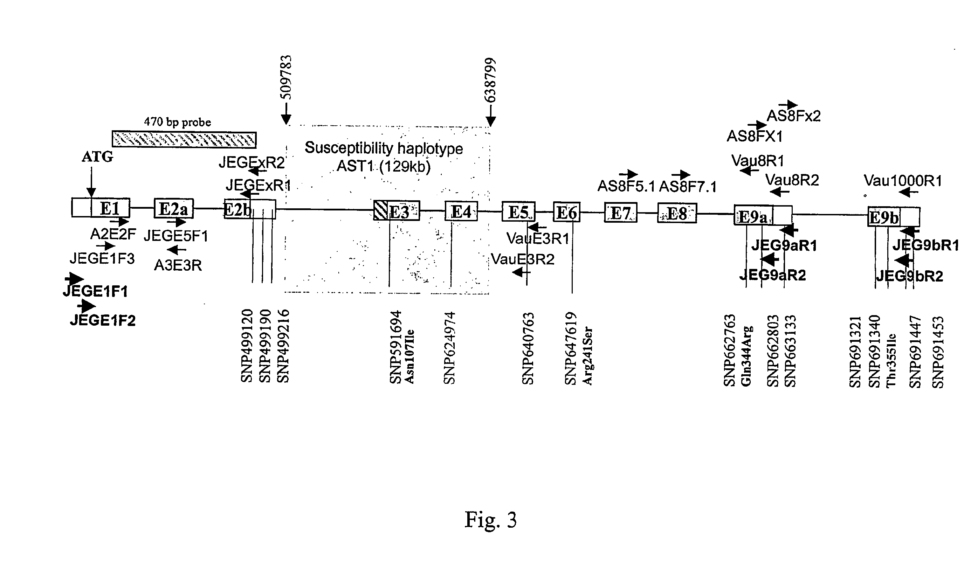

**Fig. 3****:** Genomic localization of the susceptibility haplotype (AST1, gray region) of 129 kb for asthma related traits in relation to verified exons of GPRA (G protein coupled receptor for asthma susceptibility). The cDNA probe used in the Northern blotting is shown in shaded gray. Locations of PCR primers used in the identification of the gene structures and nested PCR primers (in bold) used in cloning of the full length cDNA of splice variants A and B are shown. SNPs in the exons of *GPRA* and their potential amino acid changes are shown below. Both ends of the susceptibility haplotype are shown with arrows as physical positions in the genomic contig NT_000380.

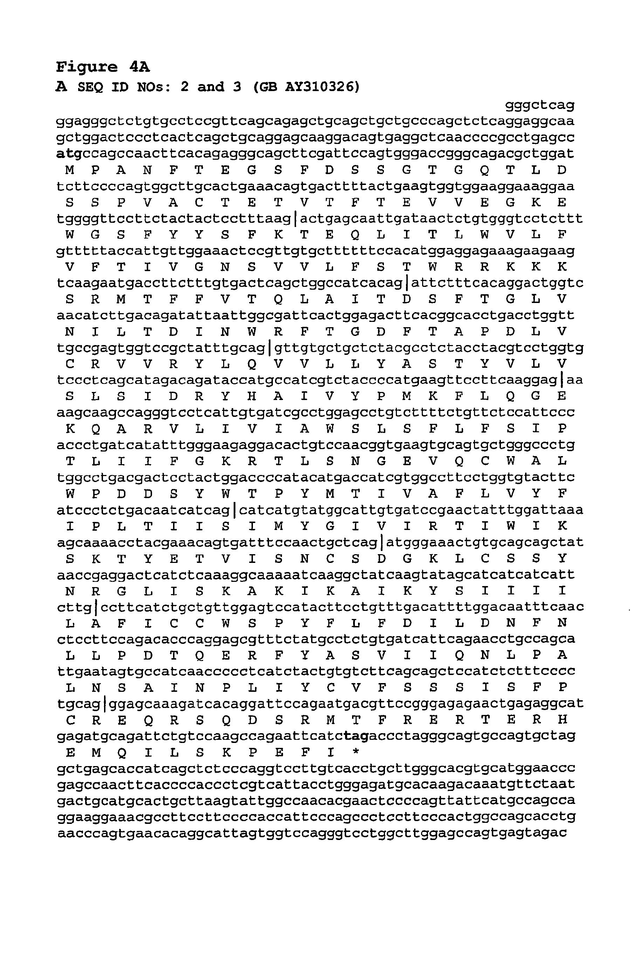

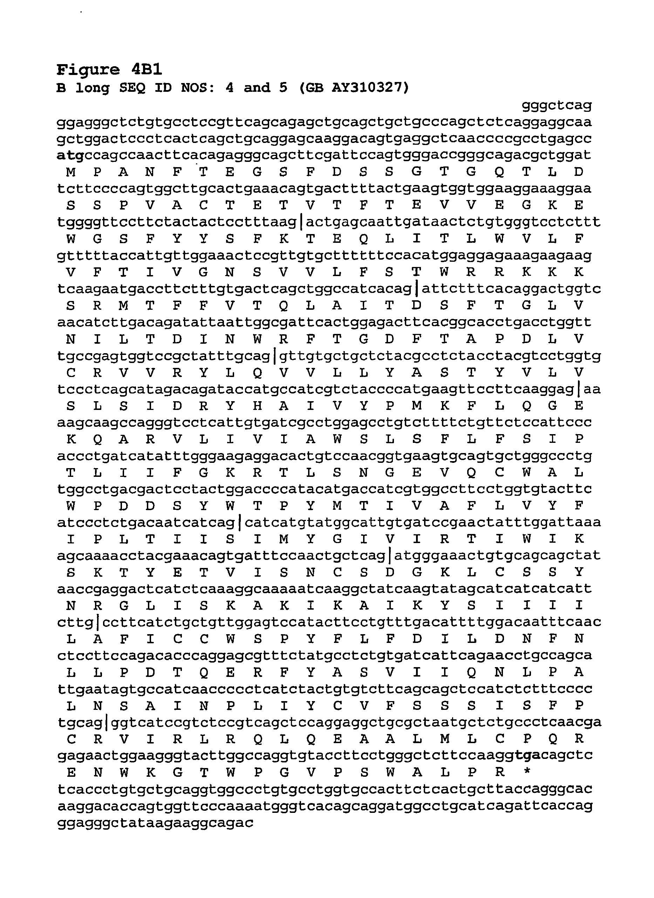

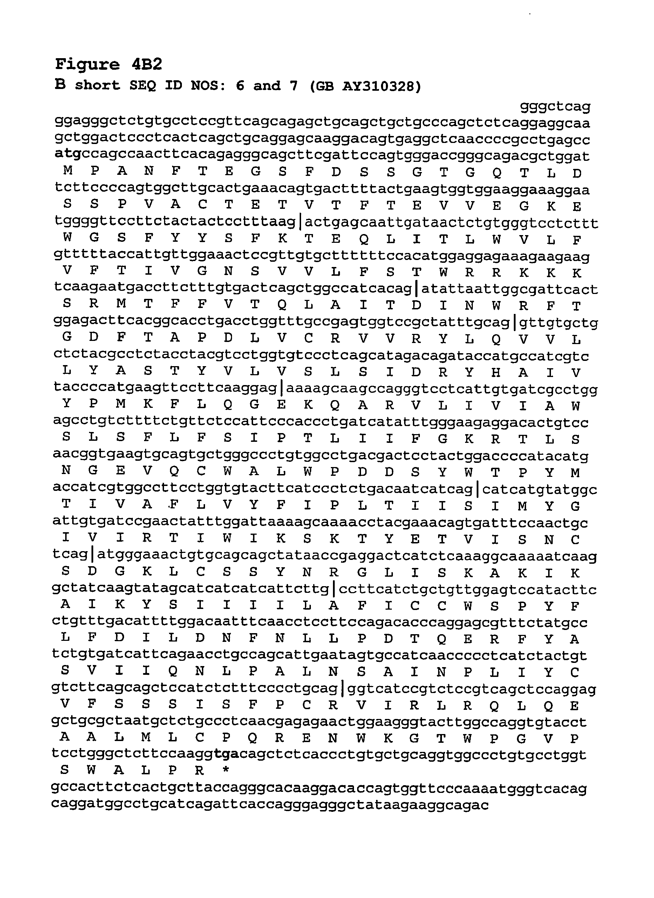

**Figs. 4A****,** **4B1****,** **4B2****,** **4C****,** **4D****,** **4E****, and** **4F****:** Deduced cDNAs (SEQ ID NOS: 2, 4, 6, 8, 10, 12, and 14) and amino acid sequences (SEQ ID NOS: 3, 5, 7, 9, 11, 13, and 15) of the alternative splice variants (A-F) of *GPRA.* Exon borders are indicated by vertical lines. The initiation and stop codons are shown in bold.
**Figs**. **5A, 5B****,** **5C, 5D****,** **5E** and **5F****:** Genomic structures of the alternative splice variants (A-F) of *GPRA.* The exons (gray boxes) are depicted by numbered boxes (E1-E9), the length of the exons are indicated below, and the length of the separating introns above. The translation-initiation site and the stop codons are indicated by arrows. The white boxes indicate the 5' and 3' UTR regions and the shaded box in the B variant exon 3 shows the alternative slicing of the long and short form.
**Figs. 6A****,** **6B****,** **6C, 6D****,** **6E and 6F****:** Predicted 7TM structures of the alternative splice variants of *GPRA* (SEQ ID NOS: 3, 5, 7, 9, 11, 13, and 15). For the splice variants A and B_{long}, cytoplasmic loops (=cytoloop) and extracellular loops (=exoloop) are indicated above the amino acid chain, transmembrane regions are underlined and the conserved amino acids, characteristic for the GPR family A, are bolded. For the other splice variants (Bₛₕₒᵣₜ-F) the number of predicted transmembrane regions varied from one to six.
**Fig. 7**: Predicted topology of the seven transmembrane structure of the protein encoded by the *GPRA* A splice variant by TMpred.
**Fig. 8****:** Expression of *GPRA* variants in the human lung epithelial carcinoma cell line NCI-H358. RT-PCR was performed by using variant A, B, and C specific primers. PCR-products were separated on ethidium bromide stained 1.5 % agarose gel.
**Fig. 9**: Expression of *GPRA* by Northern blot analysis with a 470 bp cDNA probe (comprising the exons E2a and E2b) in human placental tissue. Four transcripts were detected that were approximately 6.0 kb, 4.5 kb, 1.9 kb, and 1.0 kb in size. The expression of β-actin is shown as control.

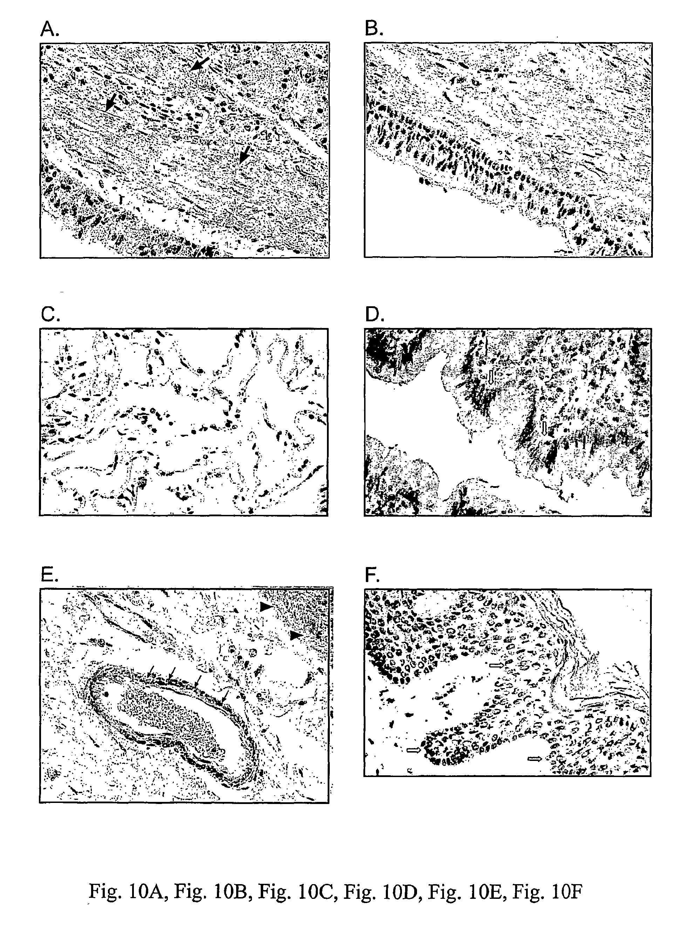

**Figs. 10A, 10B, 10C, 10D, 10E, and 10F****:** Immunohistochemical analysis of *GPRA* A in human bronchial (10A, 10B), lung (10C), and colon (10D, 10E), and skin (10D) tissues. Paraffin sections were stained using immunoperoxidase technique with GPRA variant A specific antibody (10A, 10C - 10D) or pre-immune sera (10B). Strong immunostaining of *GPRA* A in bronchus (10A) and colon (10E) was recorded in smooth muscle cell layer in bronchial walls (thick arrows) and in arterial walls (thin arrows) and subepithelially (arrowheads) in colon, respectively. In alveolar walls and alveolar macrophages (asterisk) intense staining was detected (10C). In colon (10D) and skin (10F) tissues, basal cell layer of the epithelium (white arrows) stained positive for *GPRA* A. Staining of the corresponding sections with the pre-immune serum did not show specific reactivity (10B). Original magnification x400.
**Figs**. **11A, 11B, 11C, 11D, 11E, and 11F****:** Immunohistochemical analysis of *GPRA* B in human bronchial (11A, 11B), lung, small intestine (11D), colon (11E), and skin (11F) tissues. Paraffin sections were stained with the immunoperoxidase technique with GPRA variant B specific antibody (11A, 11C-D) or preimmune serum (11B). Strong immunostaining of *GPRA* B was recorded in the epithelium of the bronchial wall (11A), small intestine (11D), colon (11E), and skin (11F) tissues (arrows). In lung intense staining of alveolar wall and alveolar macrophages (asterisk) was detected (11C). Staining of the corresponding sections with the preimmune serum did not show specific reactivity (11B). Original magnification x 400.

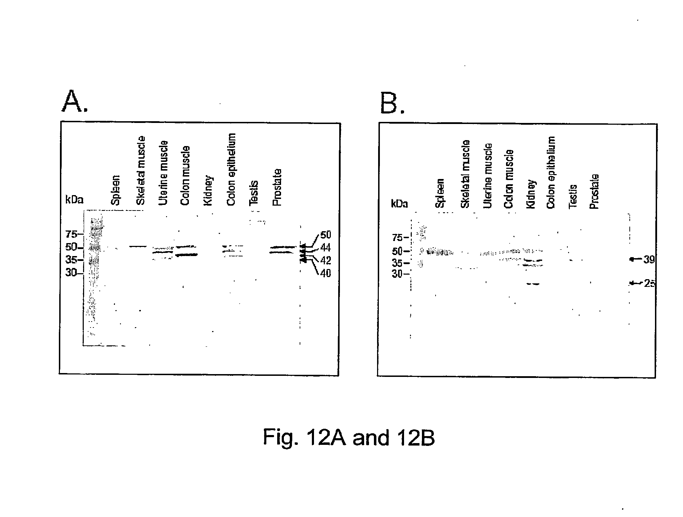

**Figs. 12A and 12B**: In Western blot analysis of several human tissues, the A variant specific antibody recognized four major polypeptide bands at approximately 40, 42, 44, and 50 kDa (12A). 50 kDa band was detectable in skeletal muscle whereas uterine muscle, colon epithelium and prostate showed similar expression patterns of *GPRA A* at approximately 50, 44, 40 kDa. The most intensive *GPRA A* expression was recorded in colon muscle at 42 kDa. The B variant specific antibody recognized two major polypeptide bands at approximately 25 and 39 kDa with the most intensive *GPRA* expression in kidney (12B).

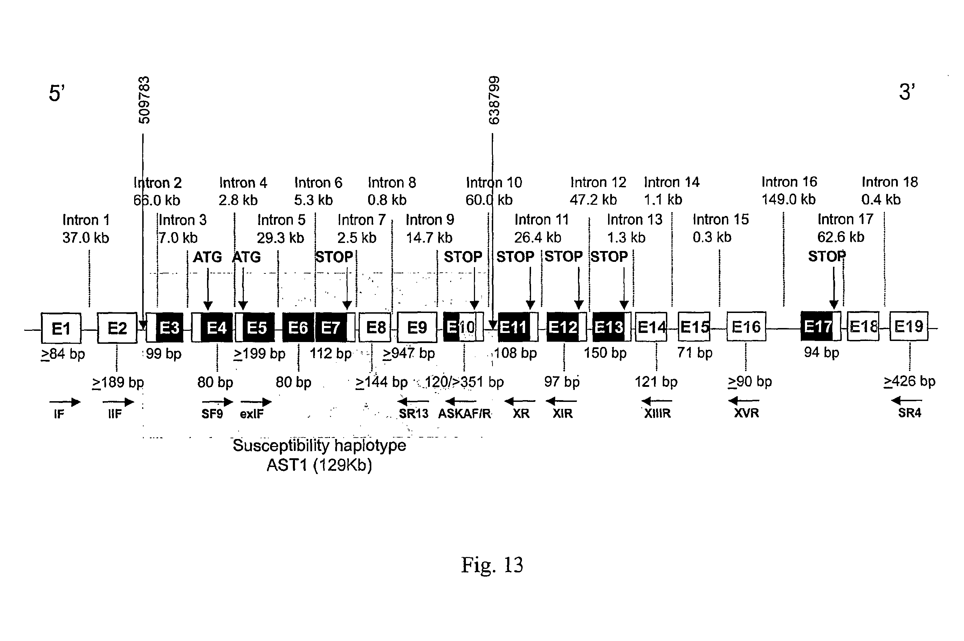

**Fig.13**: Genomic structure of AAA1 showing both the exon (size below) and intron (size above) structures of the gene. AAA1 (asthma associated alternatively spliced gene 1) shows complex splicing, two alternative starting methionines and six alternative stop codons for the predicted polypeptide was identified. Location of the primers used in cloning of different splice variants are shown below. Gray area shows the location of AST1.

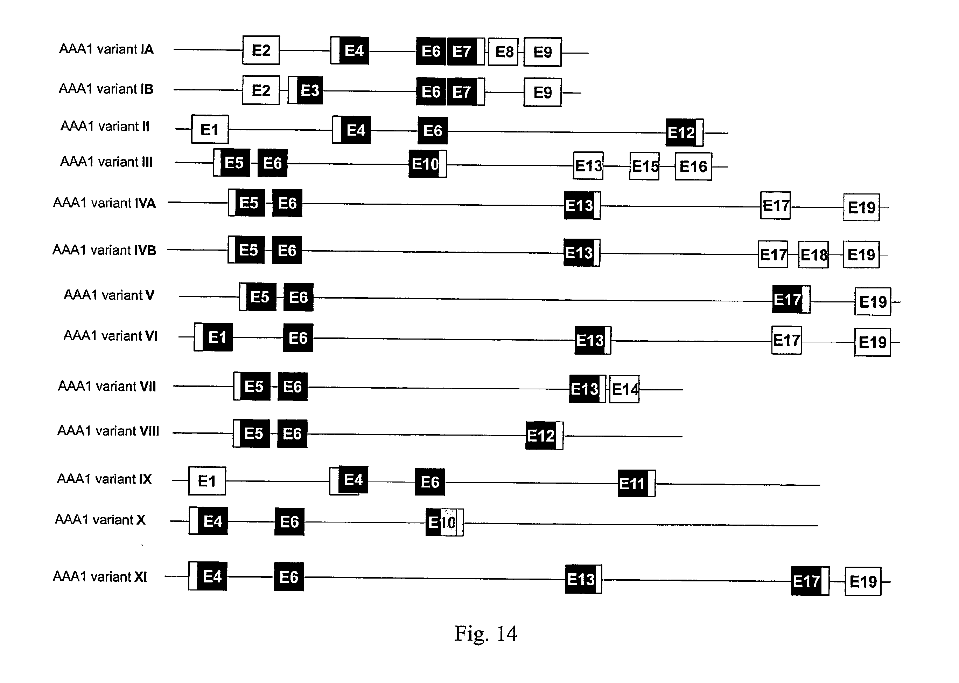

**Fig.14****:** I-XII different splice variants of AAA1, I-XI are full length cDNAs.

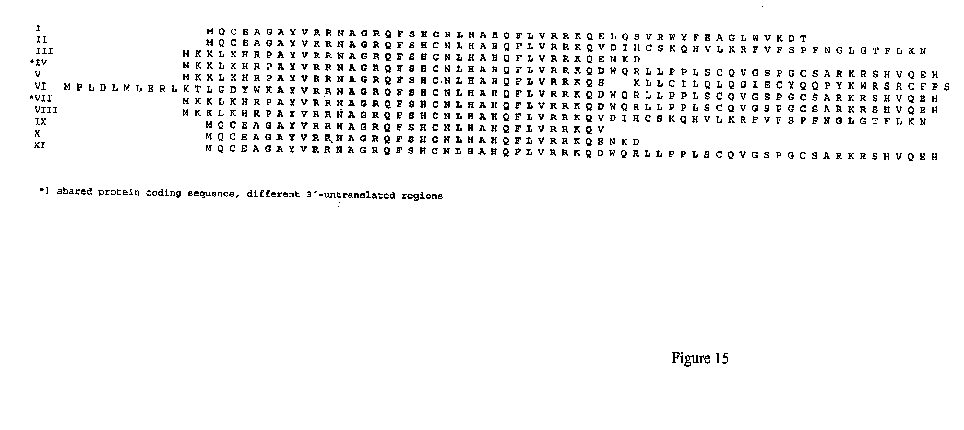

**Fig. 15****:** Sequence alignments of the predicted polypeptides encoded by the I-XI splice variants of AAA1 (CLUSTAL W program). The conserved region of the AAA1 encoded polypeptides is in bold.

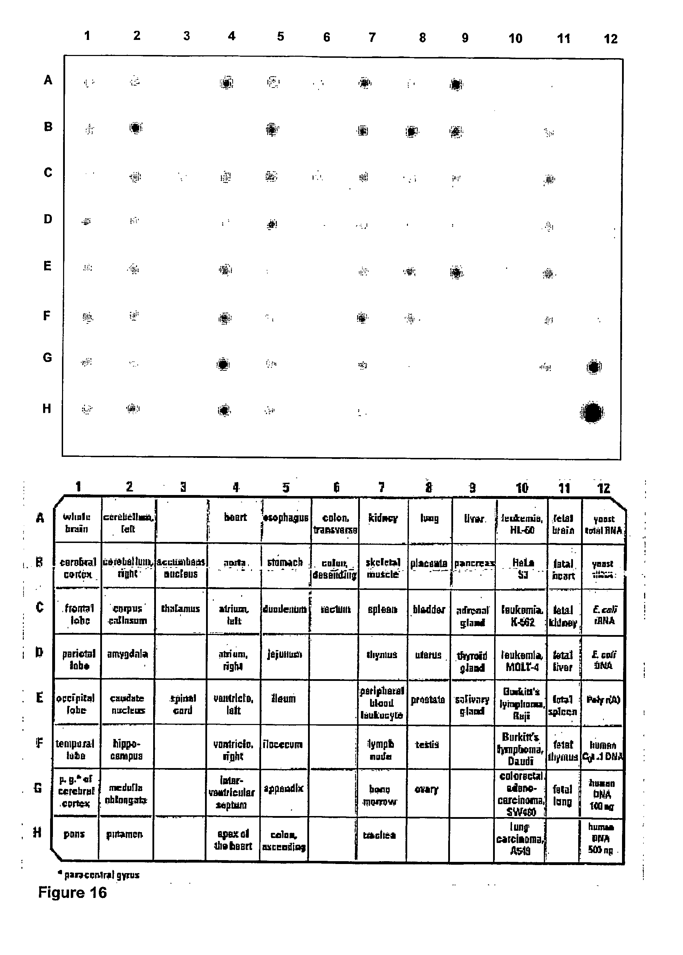

**Fig. 16**: Human multiple tissue expression array (BD, Clontech) hybridized with the probe specific for AAA1 (a mixture of multiple splice variants) (upper panel) and location of studied RNAs in the dot blot (lower panel).

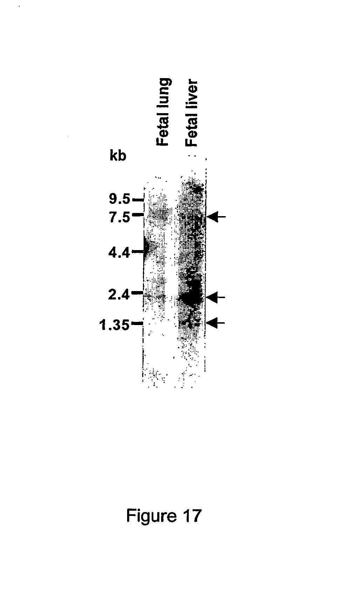

**Fig. 17****:** Human fetal multiple tissue northern blot (BD, Clontech) hybridized with the probe specific for AAA1 (a mixture of multiple splice variants) identifies several alternative transcripts (arrows).

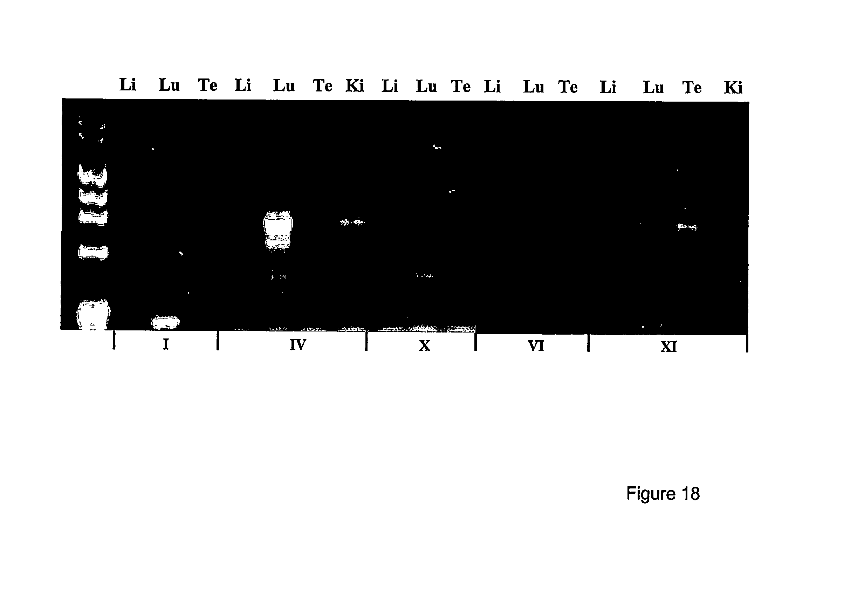

**Fig. 18**: Tissue specific expression AAA1. RT-PCR with the variants I, IV, VI, X, and XI specific primer pairs in liver (Li), lung (Lu), testis (Te), and kidney (Ki) shows different patterns of transcripts in different tissues.

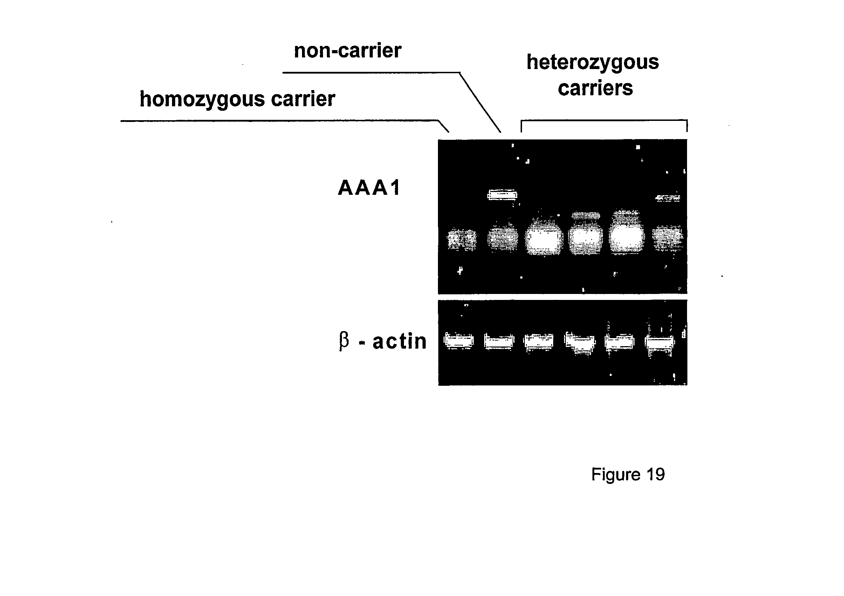

**Fig. 19**: Variable alternative splicing for AAA1 depending on genotype. RT-PCR spanning exons 6 to 10b of AAA1 was performed on lymphoblastic RNA samples genotyped for AST1. Only the non-carrier of AST1 processes normal amount of the exon 6-10b transcript, whereas a homozygote and heterozygotes show either absent transcript or smaller splice variants. Beta-actin was used as control in parallel amplifications.

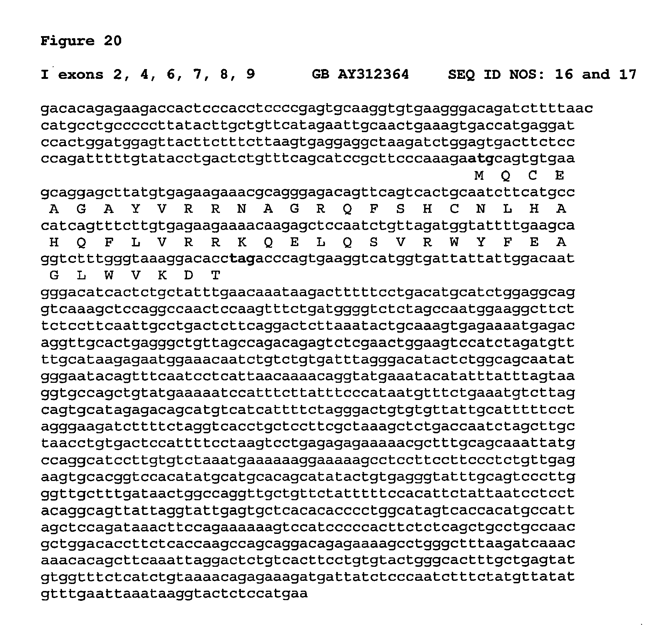

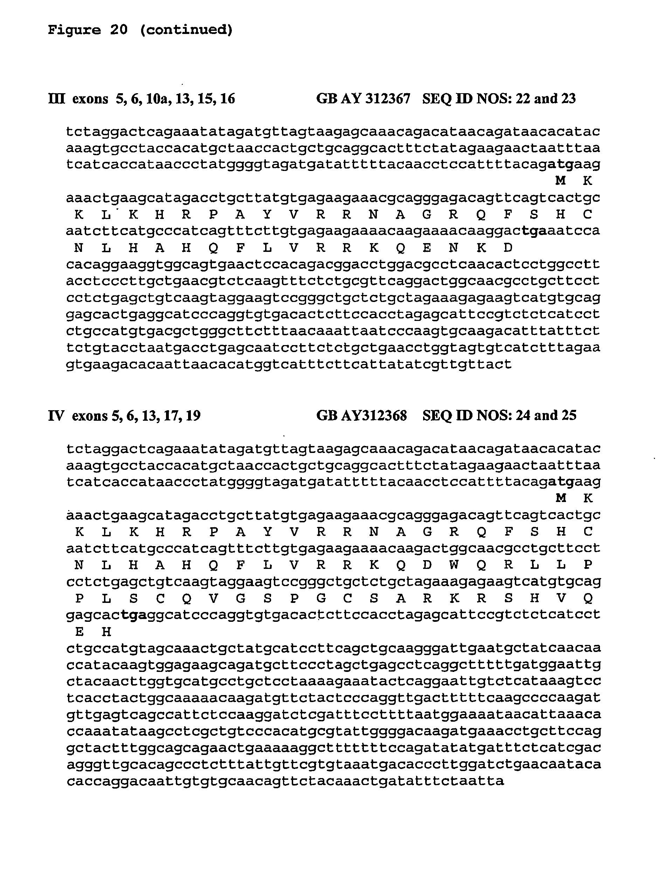

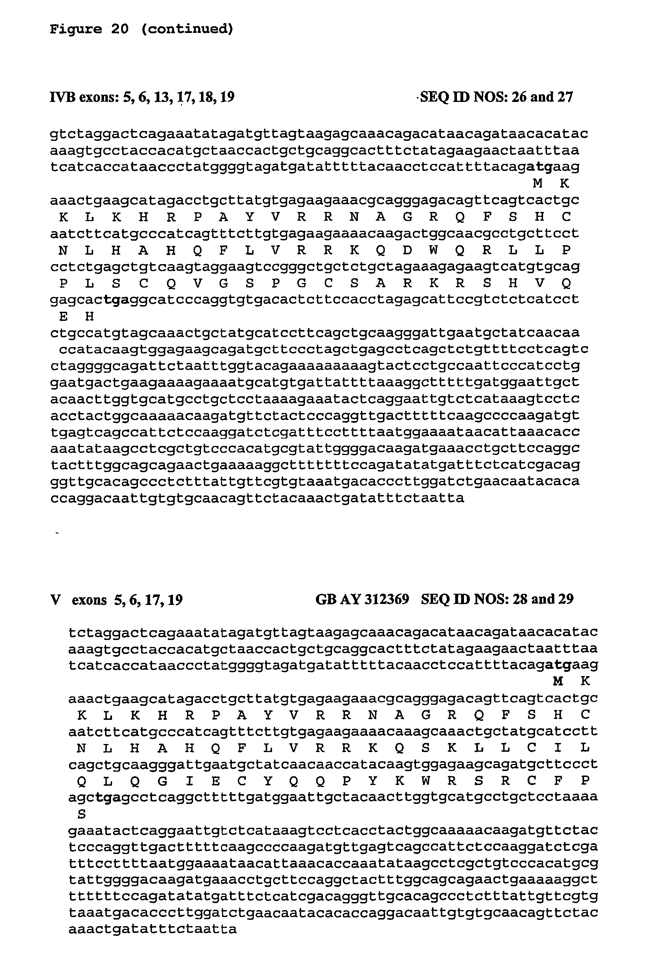

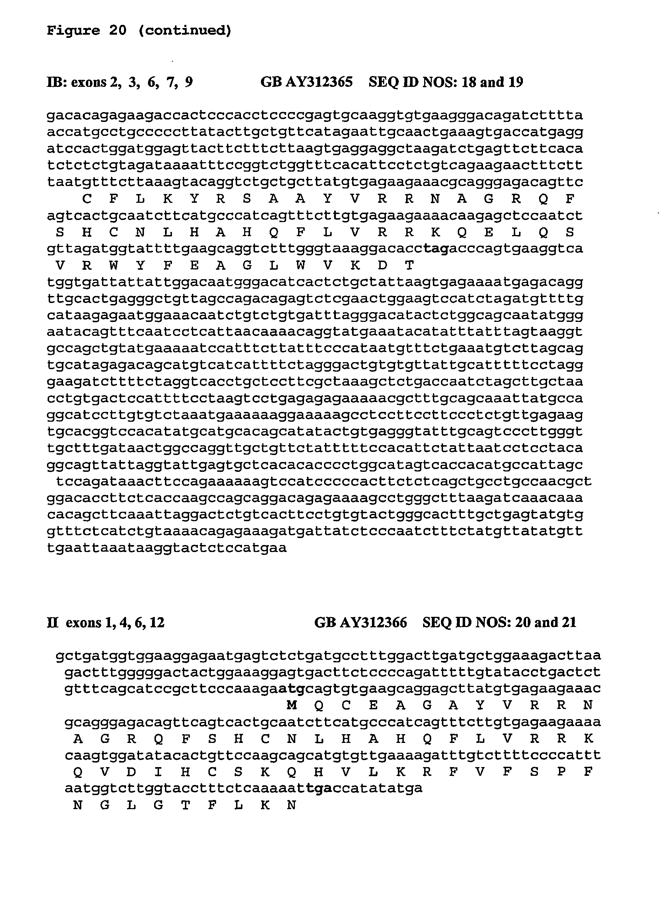

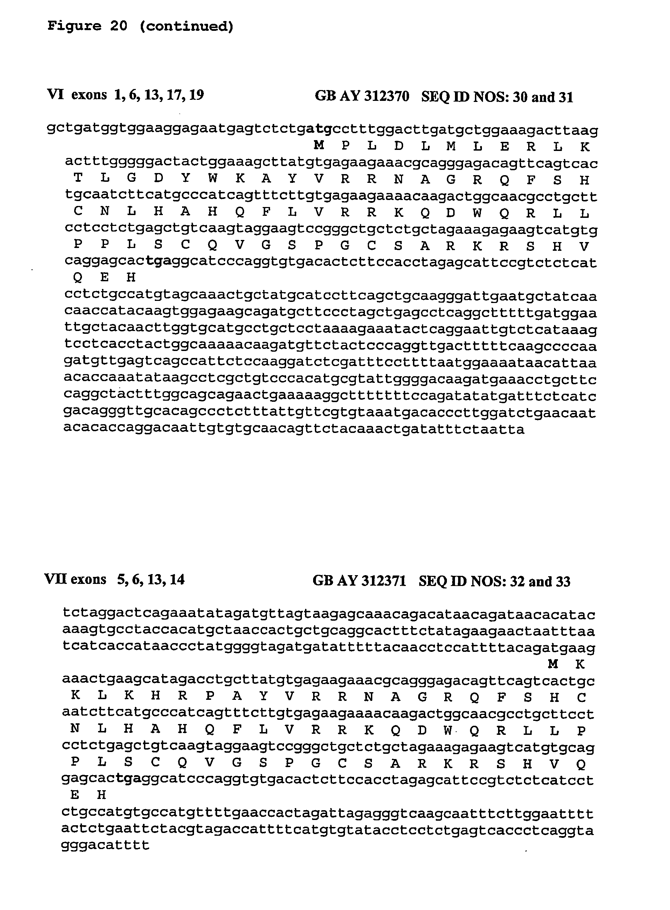

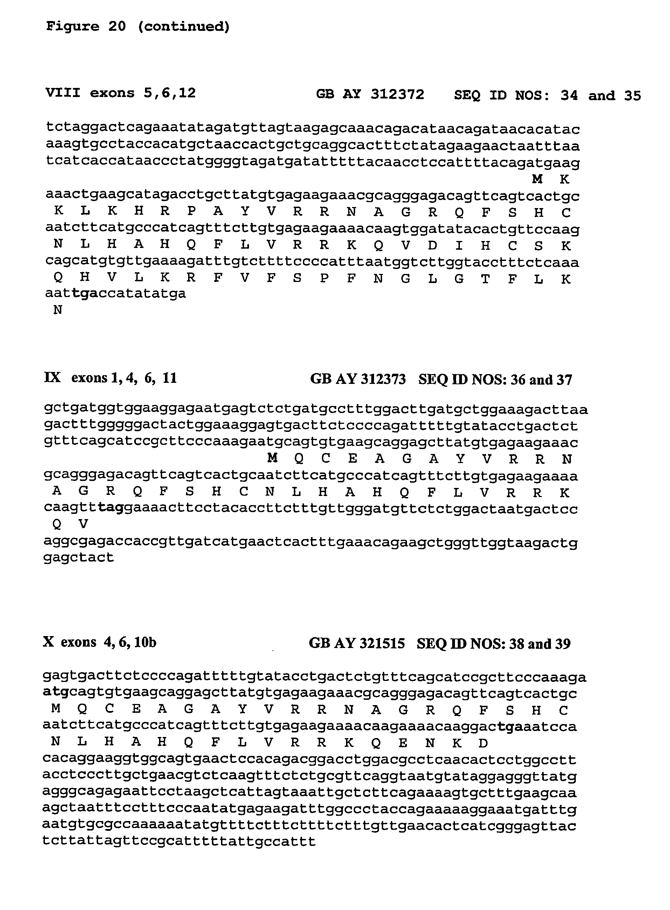

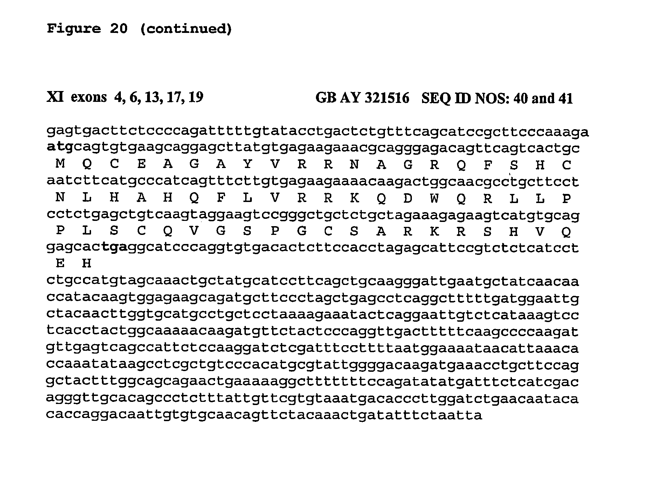

**Fig. 20**: Nucleic (SEQ ID NOS:16, 18, 20, 22, 24, 26, 28, 30, 32, 34, 36, 38 and 40) and amino acid sequences (SEQ ID NOS:17, 19, 21, 23, 25, 27, 29, 31, 33, 35, 37, 39 and 41) of splice variants of AAA1.

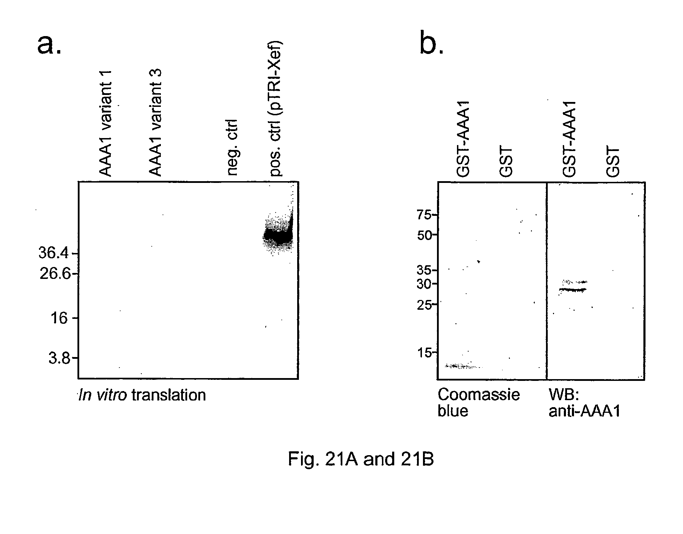

**Figs. 21A and 21B: 21A****.** *In vitro* translation of AAA1 gene using rabbit reticulocyte lysate translation machinery with S³⁵ -labelled methionine in the reaction mixture. 21B. Specificity of the AAA1 antibody (anti-AAA1). Glutathione S-transferase (GST) -fusion proteins for AAA1 were expressed in *E*. *coli* as GST fusion proteins (GST-AAA1).

### DEFINITIONS

Unless defined otherwise, all technical and scientific terms have the same meaning as is commonly understood by one of skill in the art to which this invention is related. The definitions below are presented for clarity.

"Isolated" when referred to a molecule, refers to a molecule that has been identified and separated and/or recovered from a component of its natural environment and thus is altered "by the hand of man" from its natural state. For example, an isolated polynucleotide could be part of a vector or a composition of matter, or could be contained within a cell, and still be "isolated" because that vector, composition of matter, or particular cell is not the original environment of the polynucleotide. The term "isolated" does not refer to genomic or cDNA libraries, whole cell total or mRNA preparations, genomic DNA preparations (including those separated by electrophoresis and transferred onto blots), sheared whole cell genomic DNA preparations or other compositions where the art demonstrates no distinguishing features of the polynucleotide sequences disclosed herein.

"Nucleic acid", includes DNA molecules (*e.g*. cDNA or genomic DNA), RNA molecules (*e.g*., mRNA), analogs of the DNA or RNA generated using nucleotide analogs, and derivatives, fragments and homologs. The nucleic acid or nucleic acid molecule may be single-stranded or double-stranded, but preferably comprises double-stranded DNA. Preferred nucleic acids include segments of DNA, or their complements including any one of the polymorphic sites shown in Table 3, 7 or 12. The segments are usually between 5 and 100 contiguous bases, and often range from 5, 10, 12, 15, 20, or 25 nucleotides to 10, 15, 30, 25, 20, 50 or 100 nucleotides. Nucleic acids between 5-10, 5-20, 10-20, 12-30, 15-30, 10-50, 20-50 or 20-100 bases are common. The polymorphic site can occur within any position of the segment. The segments can be from any of the allelic forms of DNA shown in Table 3, 7 or 12. For brevity in Table 3, the symbol T is used to represent both thymidine in DNA and uracil in RNA. Thus, in RNA oligonucleotides, the symbol T should be construed to indicate a uracil residue. Unless otherwise apparent from the context, reference to a SEQ ID NO., refers to the strand shown in the sequence listing for that SEQ ID NO., the perfect complementary strand thereof or a duplex of the two. Thus, for example, reference to primers hybridizing to SEQ ID NO:1 includes primers hybridizing to the strand shown in the sequence listing and the complementary strand.

"Isolated nucleic acid" is separated from other nucleic acid molecules which are present in the natural source of the nucleic acid. Preferably, an isolated nucleic acid is free of sequences that naturally flank the nucleic acid (*i.e.* sequences located at the 5'- and 3'-termini of the nucleic acid) in the genomic DNA of the organism from which the nucleic acid is derived. For example, in various embodiments, isolated asthma locus-1 molecules can contain less than about 5 kb, 4 kb, 3kb, 2 kb, 1 kb, 0.5 kb or 0.1 kb of nucleotide sequences which naturally flank the nucleic acid molecule in genomic DNA of the cell/tissue from which the nucleic acid is derived (*e.g*., brain, heart, liver, spleen, etc.). Moreover, an isolated nucleic acid molecule, such as a cDNA molecule, can be substantially free of other cellular material or culture medium when produced by recombinant techniques, or of chemical precursors or other chemicals when chemically synthesized.

The term "fragment" or "segment" as applied to a longer nucleic acid, may ordinarily be at least about 10 contiguous nucleotides of the longer nucleic acid in length, typically, at least about 20 contiguous nucleotides, more typically, from about 20 to about 50 contiguous nucleotides, preferably at least about 50 to about 100 contiguous nucleotides, even more preferably at least about 100 contiguous nucleotides to about 300 contiguous nucleotides, yet even more preferably at least about 300 to about 400, and most preferably, the nucleic acid fragment will be greater than about 500 contiguous nucleotides in length.

"Oligonucleotide" comprises a series of linked nucleotide residues, which oligonucleotide has a sufficient number of nucleotide bases to be used in a PCR reaction or other application. A short oligonucleotide sequence may be based on, or designed from, a genomic or cDNA sequence and is used to amplify, confirm, or reveal the presence of an identical, similar or complementary DNA or RNA in a particular cell or tissue. Oligonucleotides comprise portions of a nucleic acid.

"Variant" refers to a polynucleotide differing from the polynucleotide of the present invention, but retaining essential properties thereof. Generally, variants are overall closely similar, and, in many regions, identical to the polynucleotide disclosed herein Allelic variants of a gene refer to variant forms of the same gene between different individuals of the same species. The first identified allelic form is arbitrarily designated as the reference form and other allelic forms are designated as alternative or variant alleles. The allelic form occurring most frequently in a selected population is sometimes referred to as the wildtype form. Diploid organisms may be homozygous or heterozygous for allelic forms. A diallelic polymorphism has two forms. A triallelic polymorphism has three forms. Cognate forms of a gene refers to variation between structurally and functionally related genes between species. For example, the human gene showing the greatest sequence identity and closest functional relationship to a mouse gene is the human cognate form of the mouse gene.

A single nucleotide polymorphism occurs at a polymorphic site occupied by a single nucleotide, which is the site of variation between allelic sequences. The site is usually preceded by and followed by highly conserved sequences of the allele (*e.g.,* sequences that vary in less than 1/100 or 1/1000 members of the populations). A single nucleotide polymorphism usually arises due to substitution of one nucleotide for another at the polymorphic site. A transition is the replacement of one purine by another purine or one pyrimidine by another pyrimidine. A transversion is the replacement of a purine by a pyrimidine or vice versa. Single nucleotide polymorphisms can also arise from a deletion of a nucleotide or an insertion of a nucleotide relative to a reference allele. A set of polymorphisms means at least 2, and sometimes 5, or more of the polymorphisms shown in Table 3, 7 or 12.

"Stringency". Highly stringency conditions are well-known in the art, *e.g*. 6×NaCl/sodium citrate (SSC) at about 45 °C is applied for a hybridization step, followed by a wash of 2×SSC at 50 °C or, *e.g.,* alternatively hybridization at 42°C in 5×SSC, 20 mM NaPO4, pH 6.8, 50% formamide; and washing at 42 °C in 0.2×SSC. These conditions can be varied empirically based on the length and the GC nucleotide base content of the sequences to be hybridized, or based on formulas for determining such variation (See, for example, Sambrook et al,"Molecular Cloning: A Laboratory Manual", Second Edition, pages 9.47-9.51, Cold Spring Harbor, New York: Cold Spring Harbor Laboratory Press (1989)). A low stringency is defined herein as being in 4-6 X SSC/0.1-0.5% w/v SDS at 37-45 degree of C for 2-3 hours. Depending on the source and concentration of nucleic acid involved in the hybridization, alternative conditions of stringency may be employed such as medium stringent conditions which are considered herein to be 1-4 X SSC/0.25-0.5% w/v SDS at 45 degree of Celsius for 2-3 hours or highly stringent conditions considered herein to be 0.1-1 X SSC/0.1% w/v SDS at 60 degree of Celsius for 1-3 hours.

Hybridization probes are capable of binding in a base-specific manner to a complementary strand of nucleic acid. Such probes include nucleic acids, peptide nucleic acids, as described in Nielsen et al., Science 254, 1497-1500 (1991). "Probes" are nucleic acid sequences of variable length, preferably between at least about 10 nucleotides (nt), 100 nt, or many *(e.g.,* 6000 nt) depending on the specific use. Often probes have 15-50 nucleotides. Probes are used to detect identical, similar, or complementary nucleic acid sequences. Longer length probes can be obtained from a natural or recombinant source, are highly specific, and much slower to hybridize than shorter-length oligomer probes. Probes may be single- or double-stranded and designed to have specificity in PCR, membrane-based hybridization technologies, or ELISA-like technologies. Probes also hybridize to nucleic acid molecules in biological samples, thereby enabling immediate applications in chromosome mapping, linkage analysis, tissue identification and/or typing, and a variety of forensic and diagnostic methods.

The term primer refers to a single-stranded oligonucleotide capable of acting as a point of initiation of template-directed DNA synthesis under appropriate conditions (*i.e.,* in the presence of four different nucleoside triphosphates and an agent for polymerization, such as, DNA or RNA polymerase or reverse transcriptase) in an appropriate buffer and at a suitable temperature. The appropriate length of a primer depends on the intended use of the primer but typically ranges from 15 to 30, 40 or 50 nucleotides. Short primer molecules generally require cooler temperatures to form sufficiently stable hybrid complexes with the template. A primer need not reflect the exact sequence of the template but must be sufficiently complementary to hybridize with a template. The term primer site refers to the area of the target DNA to which a primer hybridizes. The term primer pair means a set of primers including a 5' upstream primer that hybridizes with the 5' end of the DNA sequence to be amplified and a 3', downstream primer that hybridizes with the complement of the 3' end of the sequence to be amplified.

"Vector" means any plasmid or virus encoding an exogenous nucleic acid. The term should also be construed to include non-plasmid and non-viral compounds which facilitate transfer of nucleic acid into virions or cells, such as, for example, polylysine compounds and the like. The vector may be a viral vector which is suitable as a delivery vehicle for delivery of the nucleic acid encoding the desired protein, or mutant thereof, to a cell, or the vector may be a non-viral vector which is suitable for the same purpose. Examples of viral and non-viral vectors for delivery of DNA to cells and tissues are well known in the art and are described, for example, in Ma et al. (1997, Proc. Natl. Acad. Sci. U.S.A. 94:12744-12746). Examples of viral vectors include, but are not limited to, a recombinant vaccinia virus, a recombinant adenovirus, a recombinant retrovirus, a recombinant adeno-associated virus, a recombinant avian pox virus, and the like (Cranage et al., 1986, EMBO J. 5.3057-3063; International Patent Application No. W094/17810, published August 18, 1994; International Patent Application No. W094/23744, published October 27, 1994). Examples of non-viral vectors include, but are not limited to, liposomes, polyamine derivatives of DNA, and the like.

"Recombinant polynucleotide" refers to a polynucleotide having sequences that are not naturally joined together. An amplified or assembled recombinant polynucleotide may be included in a suitable vector, and the vector can be used to transform a suitable host cell.

"Mammal" for purposes of treatment refers to any animal classified as a mammal, including humans, domestic and farm animals, and zoo, sports, or pet animals, such as dogs, horses, cats, or cows. Preferably, the mammal is human.

Linkage describes the tendency of genes, alleles, loci or genetic markers to be inherited together as a result of their location on the same chromosome, and can be measured by percent recombination between the two genes, alleles, loci or genetic markers that are physically-linked on the same chromosome. Loci occurring within 50 centimorgan of each other are linked. Some linked markers occur within the same gene or gene cluster.

Polymorphism refers to the occurrence of two or more genetically determined alternative sequences or alleles in a population. A polymorphic marker or site is the locus at which divergence occurs. Preferred markers have at least two alleles, each occurring at frequency of greater than 1%, and more preferably greater than 10% or 20% of a selected population. A polymorphic locus may be as small as one base pair.

Linkage disequilibrium (LD) or allelic association means the preferential association of a particular allele or genetic marker with a specific allele, or genetic marker at a nearby chromosomal location more frequently than expected by chance for any particular allele frequency in the population. For example, if locus X has alleles a and b, which occur equally frequently, and linked locus Y has alleles c and d, which occur equally frequently, one would expect the haplotype ac to occur with a frequency of 0.25 in a population of individuals. If ac occurs more frequently, then alleles a and c are considered in linkage disequilibrium. Linkage disequilibrium may result from natural selection of certain combination of alleles or because an allele has been introduced into a population too recently to have reached equilibrium (random association) with between linked alleles.

A marker in linkage disequilibrium with disease predisposing variants can be particularly useful in detecting susceptibility to disease (or association with other sub-clinical phenotypes) notwithstanding that the marker does not cause the disease. For example, a marker (X) that is not itself a causative element of a disease, but which is in linkage disequilibrium with a gene (including regulatory sequences) (Y) that is a causative element of a phenotype, can be used to indicate susceptibility to the disease in circumstances in which the gene Y may not have been identified or may not be readily detectable. Younger alleles *(i.e.,* those arising from mutation relatively late in evolution) are expected to have a larger genomic segment in linkage disequilibrium. The age of an allele can be determined from whether the allele is shared between different human ethnic human groups and/or between humans and related species.

Unless otherwise apparent from the context, any embodiment, element or feature of the invention can be used in combination with any other.

"Analogs" are nucleic acid sequences that have a structure similar to, but not identical to, the native compound but differ from it in respect to certain components. Usually, an analog has the same or similar function to the native compound.Analogs may be synthetic or from a different evolutionary origin and may have a similar or opposite metabolic activity compared to wild type.

Derivatives and analogs may be full length or other than full length. Derivatives or analogs of the nucleic acids include, nucleic acids that are substantially identical to an exemplified SEQ ID NO. and/or capable of hybridizing to the complement of the sequence under highly stringent, moderately stringent, or low stringent conditions.

The terms "identical" or percent "identity," in the context of two or more nucleic acids or polypeptides, refer to two or more sequences or subsequences that are the same or have a specified percentage of nucleotides or amino acid residues that are the same, when compared and aligned for maximum correspondence, as measured using a sequence comparison algorithm such as those described below for example, or by visual inspection.

The phrase "substantially identical," in the context of two nucleic acids or polypeptides, refers to two or more sequences or subsequences that have at least 80, preferably at least 85%, more preferably at least 90%, 95%, 99% or higher nucleotide or amino acid residue identity, when compared and aligned for maximum correspondence, as measured using a sequence comparison algorithm such as those described below for example, or by visual inspection. Preferably, the substantial identity exists over a region of the sequences that is at least 40 residues (*i.e.,* amino acids or nucleotides) in length, preferably over a longer region than 50 residues, more preferably at least about 90-100 residues, and most preferably the sequences are substantially identical over the full length of the sequences being compared, such as the coding region of a nucleotide for example. For example, when a SEQ ID NO. serves as a reference for comparison with an object nucleic acid, the comparison is preferably performed over the entired length of the SEQ ID NO.

For sequence comparison, typically one sequence acts as a reference sequence, to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, *e*.*g*., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by visual inspection (see generally Ausubel *et al., supra*).

Another example of algorithm that is suitable for determining percent sequence identity and sequence similarity is the BLAST algorithm, which is described in Altschul et al., J. Mol. Biol. 215:403-410 (1990). Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra.).* These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. For identifying whether a nucleic acid or polypeptide is within the scope of the invention, the default parameters of the BLAST programs are suitable. The BLASTN program (for nucleotide sequences) uses as defaults a word length (W) of 11, an expectation (E) of 10, M=5, N=-4, and a comparison of both strands. For amino acid sequences, the BLASTP program uses as defaults a word length (W) of 3, an expectation (E) of 10, and the BLOSUM62 scoring matrix. The TBLATN program (using protein sequence for nucleotide sequence) uses as defaults a word length (W) of 3, an expectation (E) of 10, and a BLOSUM 62 scoring matrix. (see Henikoff & Henikoff, Proc. Natl. Acad. Sci. USA 89:10915 (1989)).

In addition to calculating percent sequence identity, the BLAST algorithm also performs a statistical analysis of the similarity between two sequences (see, *e.g.,* Karlin & Altschul, Proc. Nat'l. Acad Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.1, more preferably less than about 0.01, and most preferably less than about 0.001.

Another indication that two nucleic acid sequences are substantially identical is that the two molecules hybridize to each other under highly stringent conditions. "Bind(s) substantially" refers to complementary hybridization between a probe nucleic acid and a target nucleic acid and embraces minor mismatches that can be accommodated by reducing the stringency of the hybridization media to achieve the desired detection of the target polynucleotide sequence. The phrase "hybridizing specifically to", refers to the binding, duplexing, or hybridizing of a molecule only to a particular nucleotide sequence under highly stringent conditions when that sequence is present in a complex mixture (*e.g*., total cellular) DNA or RNA.

The phrases "specifically binds" refers to a binding reaction which is determinative of the presence of the protein in the presence of a heterogeneous population of proteins and other biologics. Thus, under designated conditions, a specified ligand binds preferentially to a particular protein and does not bind in a significant amount to other proteins present in the sample. A molecule such as antibody that specifically binds to a protein often has an association constant of at least 10⁶M⁻¹ or 10⁷ M⁻¹, preferably 10⁸ M⁻¹ to 10⁹ M⁻¹, and more preferably, about 10¹⁰ M⁻¹ to 10¹¹ M⁻¹ or higher. An antibody that specifically binds to one segment of a protein (e.g., residues 1-10) does not bind to other segments of the protein not included within or overlapping the designted segment.

The term AAA1 gene means nucleotides 163615-684776 of the human genomic contig NT_000380 and allelic or species variants thereof.

The term GPRA gene means nucleotides 471478-691525 of the human genomic contig NT_000380 and allelic or species variants thereof.

A "pharmacological" activity means that an agent exhibits an activity in a screening system that indicates that the agent is or may be useful in the prophylaxis or treatment of a disease. The screening system can be in vitro, cellular, animal or human. Agents can be described as having pharmacological activity notwithstanding that further testing may be required to establish actual prophylactic or therapeutic utility in treatment of a disease.

Pulmonary diseases accociated with lower airways obstruction are classified as chronic obstructive pulmonary disease (COPD) or asthma on the basis of the clinical picture. "Asthma" is a chronic inflammatory disease of the airways causing variable airflow obstruction that is often reversible either spontaneously or with treatment. Asthma is highly correlated with other allergic, IgE mediated diseases, such as allergic rhinitis and dermatitis. COPD is characterized by slowly processing, mainly irreversible airway obstruction and decreased expiratory flow rate.

### DETAILED DESCRIPTION OF THE INVENTION

### I. General

This invention is based in part on the discovery and characterization of a novel susceptibility locus for asthma and other IgE mediated diseases, two overlapping genes within the locus, termed GPRA and AAA1, and proteins encoded by the genes. The genes have opposite transcriptional orientations and share intronic regions although not exons. The locus maps within human chromosome 7p15-p14.

Fig. 3 shows the structure of the human GPRA gene. The gene has nine exons, two of which have alternate forms, and eight introns. The total length of the gene from the first base of the first exon to the last base of the last exon is 220047 bp and the coordinates are 471478 (beginning) 691525 (end) in NT_000380. Part of the genomic sequence of GPRA including part of intron 2, exon 3, intron 3, exon 4 and part of intron 4 defines a sublocus referred to as AST1. This locus is 129,017 bp in length. The locus refers to the part of human chromosome 7 having the 129,017 bp of SEQ ID NO:1 or allelic or species variants thereof.

Fig. 13 shows the structure of the human AAA1 gene. The gene has nineteen exons, nine of which have alternate forms, and 18 introns. The total length of the gene from the first base of the first exon to the last base of the last exon is 521,161 bp and the coordinates are 163,615 (beginning) 684776 (end) in NT_000380. Part of the genomic sequence of AAA1 including part of intron 2, exon 3 (a and b), intron 3, exon 4 (a and b), intron 4, exon 5 (a and b), intron 5, exon 6, intron 6, exon 7 (a and b), intron 7, exon 8, intron 8, exon 9, intron 9, exon 10 (a and b) and part of intron 10 occurs within the AST1 locus.

The present application shows that the GPRA and AAA1 genes and AST-1 locus within them are genetically linked and associated with IgE diseases, such as asthma. In addition, the application provides a collection of polymorphic sites including SNPs (single nucleotide polymorphisms) and DIPs (deletion/insertion polymorphisms) within the locus. Tables 3, 7 and 12 show variant and reference (or wildtype) forms occupying these sites. The variant forms are associated with asthma, other IgE mediated diseases, and/or immune-mediated diseases including autoimmune diseases, and are useful in detection, diagnosis, treatment and prophylaxis thereof.

The AST-1 locus was localized by hierarchical LD mapping in a systemic search for a shared haplotype among the individuals with high IgE serum level across a 19 Mb region that was first identified by a genome wide screen for asthma genes (Laitinen *et al.* 2001). This analysis defined the AST-1 locus between the markers SNP509783 and SNP638799 and provided statistically significant evidence that specific haplotype of 129kb within the linkage peak is a risk factor for asthma related traits (SEQ ID NO:1). The best haplotype patterns of polymorphic sites in GPRA (Table 8) were found with the frequency of 13-18% among the high IgE associated chromosomes compared to that of 3-7% among control chromosomes suggesting the risk ratio of 3.9 [95%CI: 1.8-8.6] for high IgE level (38/304 among affected vs. 7/220 control chromosomes). The best observe associations for polymorphic sites in AAA1 reached the χ² values of 8.9-13.6 All markers identified between the positions 509,783-638,799 in the genomic contig NT_000380 were found to be in strong LD and therefore useful for diagnostic purposes.

### II. GPRA Polypeptides

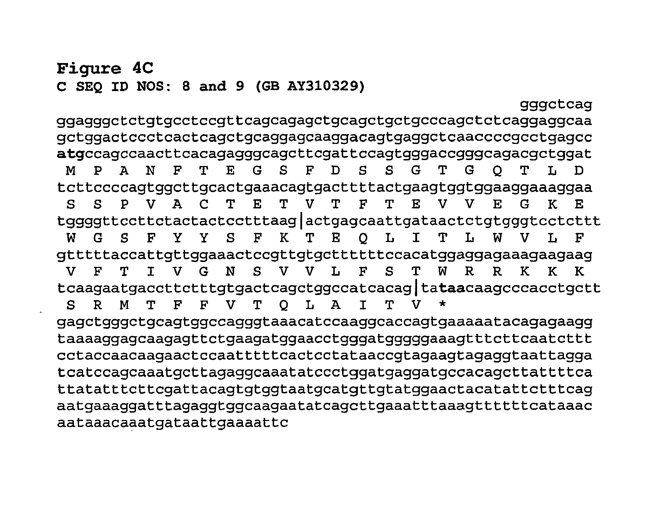

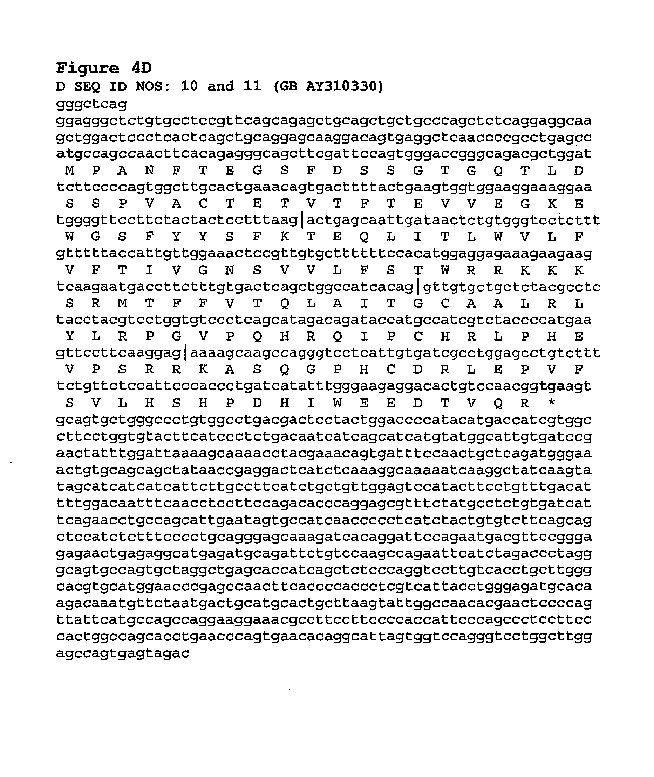

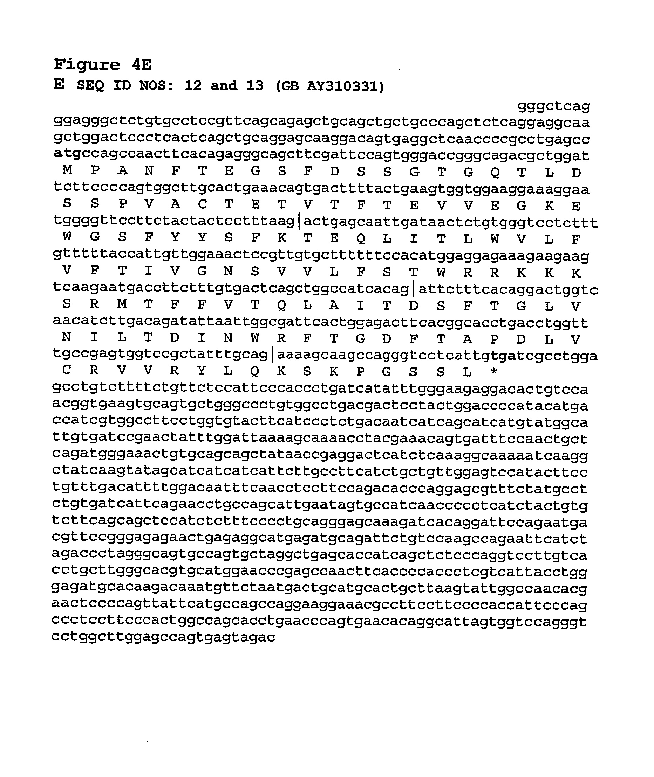

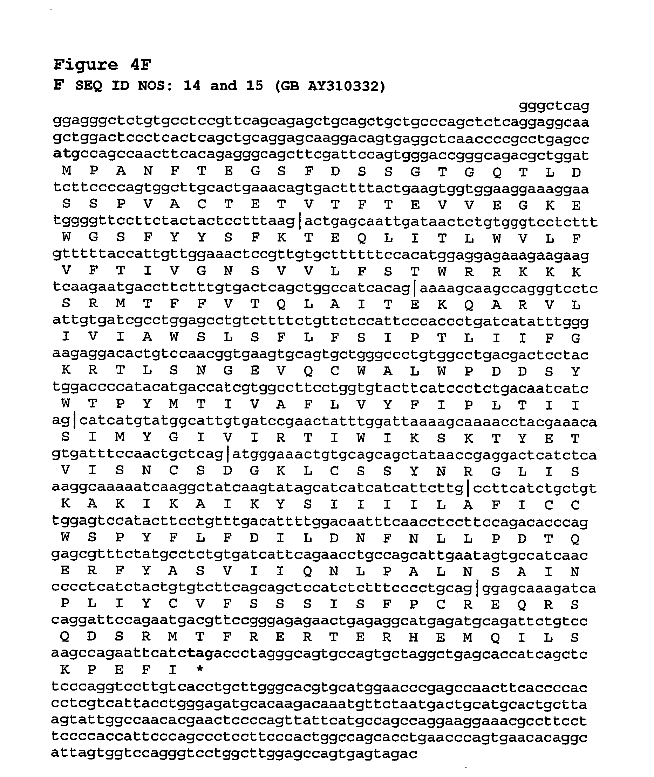

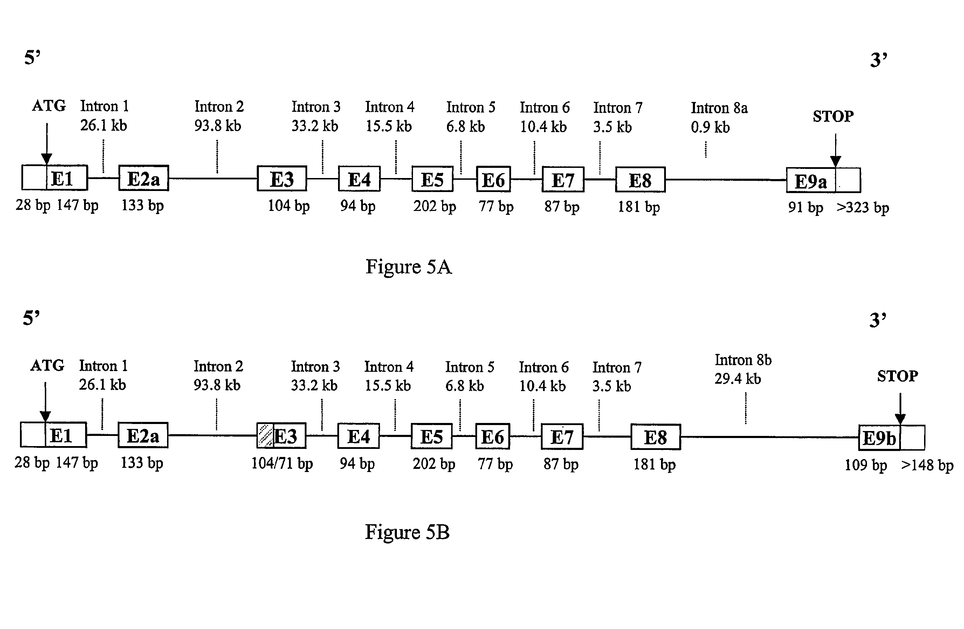

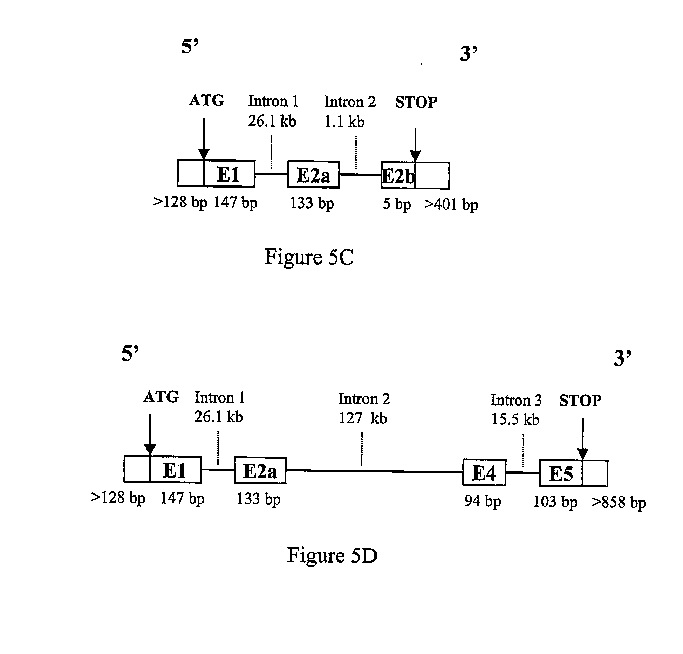

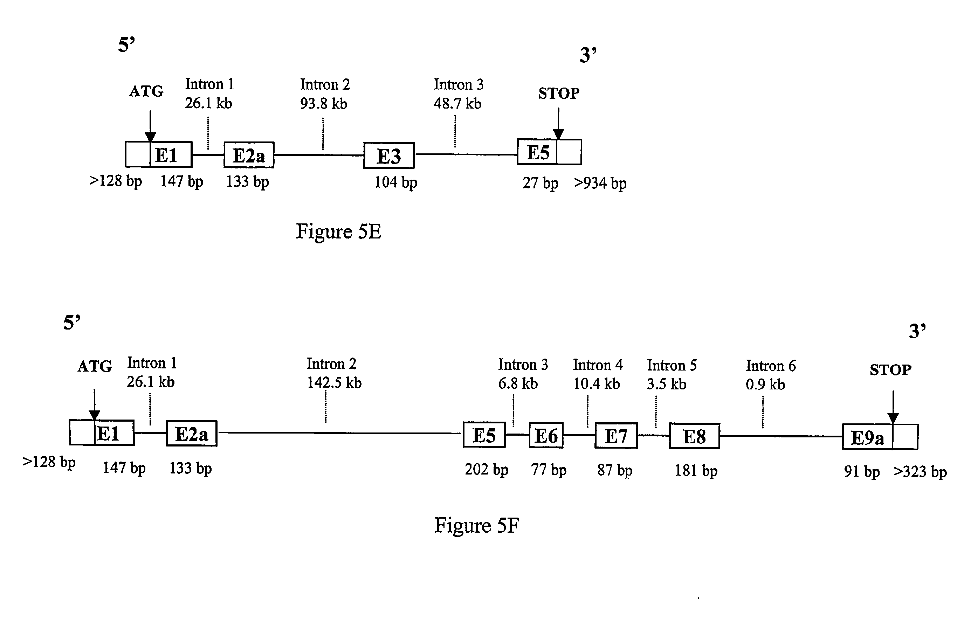

The human GPRA gene can be expressed as at least seven different splice variants termed A, B-long, B-short, C, D, E, and F. The cDNAs encoding each form and amino acid sequence of each form are shown in Fig. 4 (SEQ ID NOS:2-15). The different splicing events that lead to the different forms are shown in Fig. 5. It can be seen that the B-long and B-short forms differ from the A form in that the B forms have a different C-terminus encoded by exon 9b, whereas the C terminus of the A form is encoded by exon 9A. The B-short form (SEQ ID NOS:6 and 7) differs from the B-long form in that the B-short form has a deletion of 10 amino acids encoded by exon III. The C-form (SEQ ID NOS:8 and 9) contains a segment encoded by exon 2b which is not found in other forms. The C-form lacks any segments encoded by exons 3-9. The D form (SEQ ID NOS:10 and 11) lacks a segment encoded by exon 3, causing a frameshift, whereby exons 4 and 5 are read in a different reading frame causing truncation within exon 5. Form E (SEQ ID NOS:12 and 13) lacks exon 4 causing a frameshift as a result of which exon 5 is read in a different reading frame causing truncation within exon 5. Form F (SEQ ID NOS: 14 and 15) lacks exons 3 and 4.

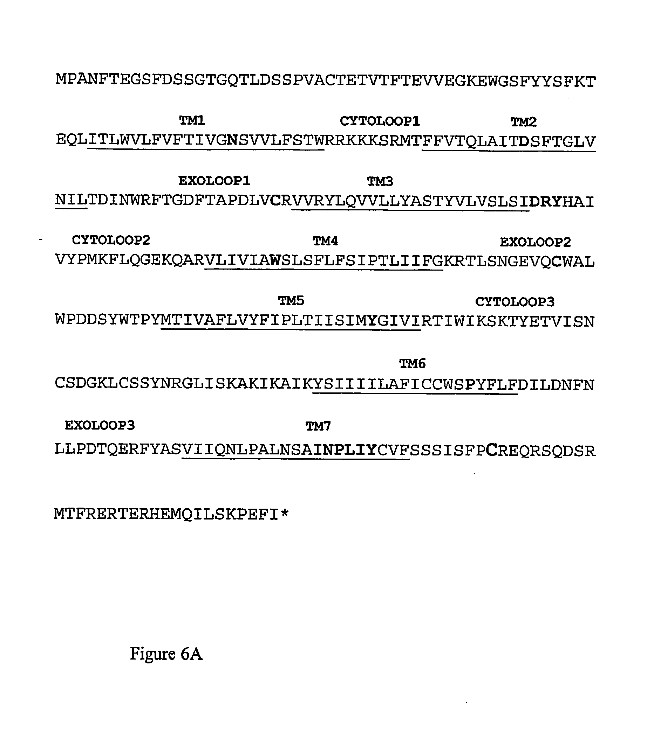

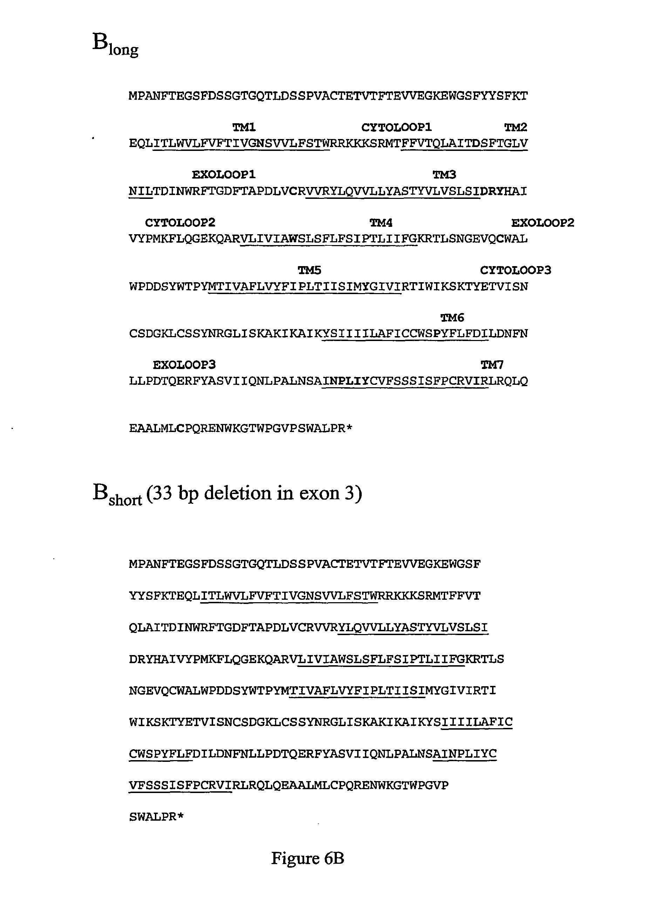

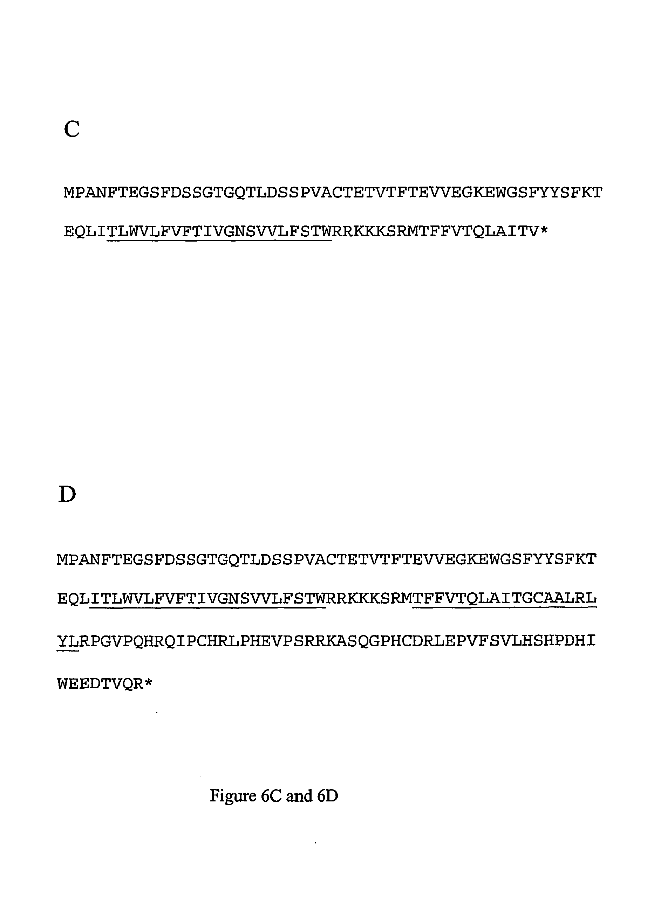

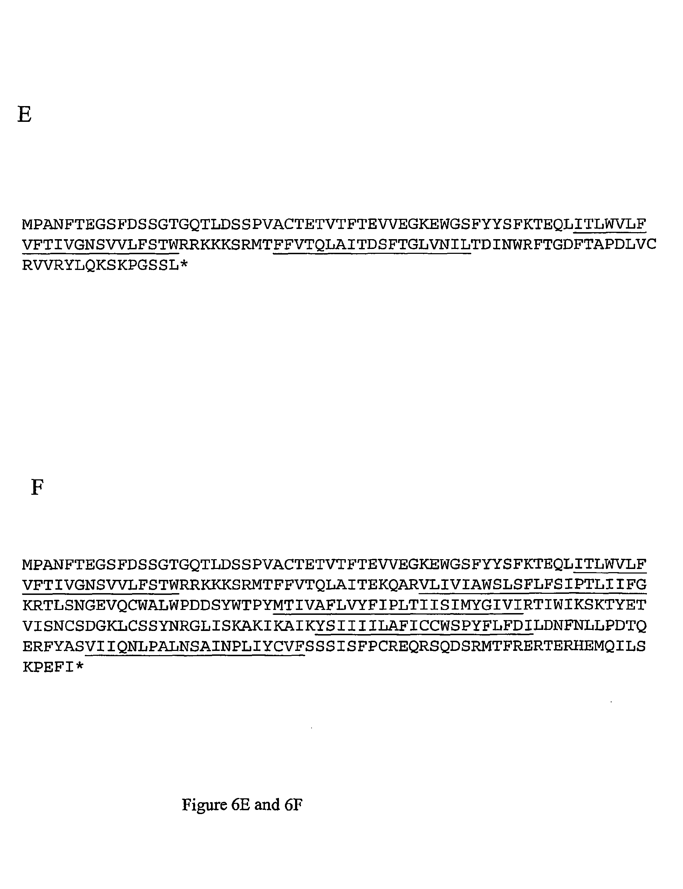

Fig. 6 shows the expected structural characteristics of the different splice variants. The A form includes 371 amino acids and has an extracellular N-terminal domain, seven transmembrane domains and an intracellular C-terminal domains (SEQ ID NO:3). The transmembrane domains are separated by alternating cytoplasmic and extracellular loops.
The B-long form (377 amino acids, SEQ ID NO:5) has a similar structure. The predicted size of the extracellular N-terminal domain is about 50 amino acids anticipating suggesting that the endogenous ligand that interacts with GPRA is a small molecule or peptide. The B-short, C, D, E and F variants of GPRA (SEQ ID NOS: 5, 7, 9, 11, 13, and 15, respectively) lack the 7TM strutucture present in variants A and B-Long.

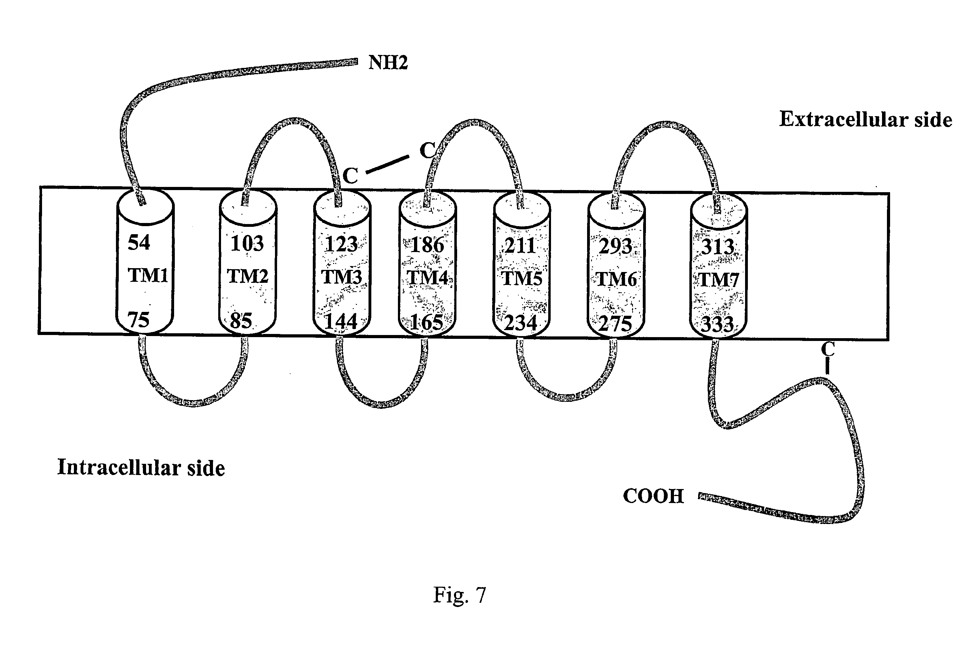

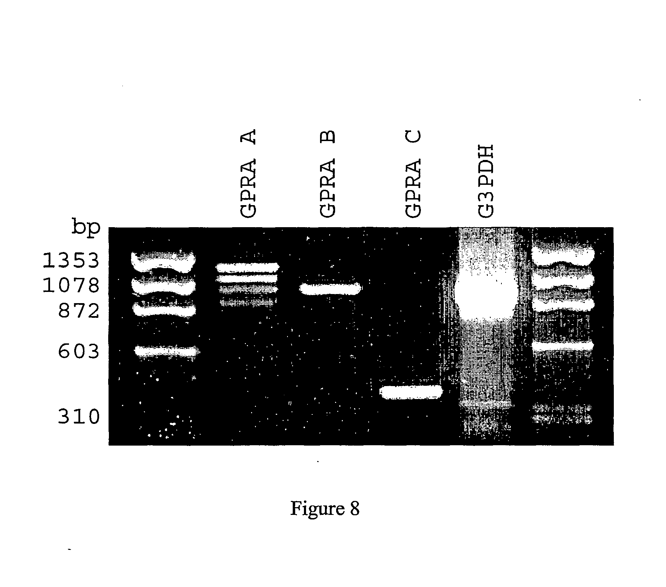

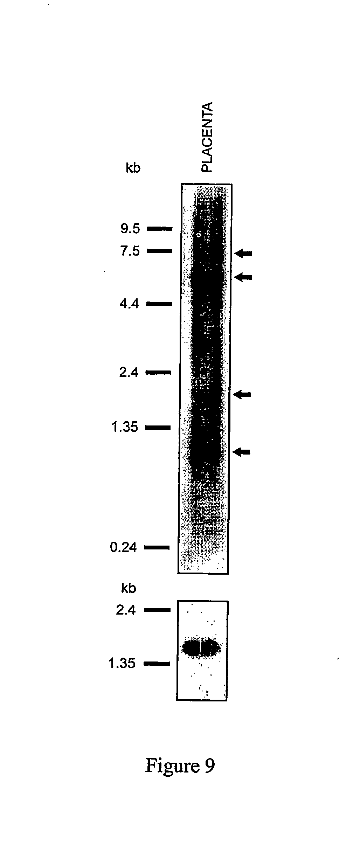

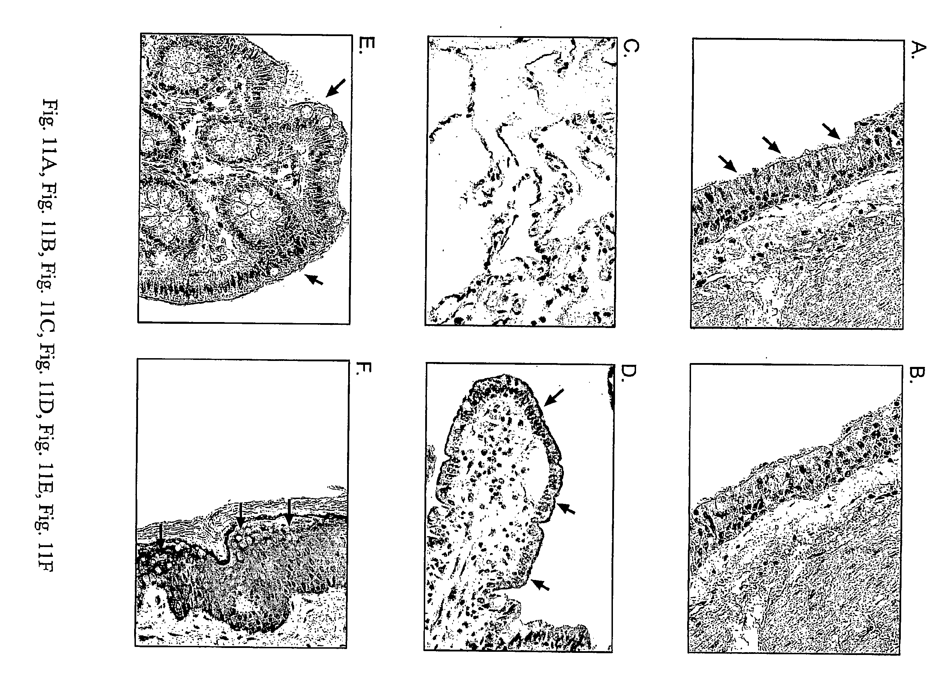

Fig. 7 shows the arrangement of the A or B-Long form in a cell membrane. GPRA falls within the conserved class A of G-protein coupled receptors (GPRs) (Rana *et al.* 2001, Johnson *et al.* 2000). GPRs are a large and functionally diverse protein superfamily that form a seven-transmembrane (7TM) helices bundle with alternating extracellular and intracellular loops (see WO 01/18206). Class A contains most well known members of the GPRs such as vasopressin, oxytocin, and bovine rodopsin receptors. For the latter the crystallographic structure is available (Palczewski *et al.* 2000). GPRA is expressed in human tissues consistent with its role in asthma and IgE mediated disease. Transcripts of *GPRA* variants A, B. and C were expressed in several tissues including lung and NCI-H358 cell line that represent cells of broncho-epithelial origin (Fig. 8 and 9). Based on immunohistochemistry, the A variant of the GPRA protein was expressed in smooth muscle cells of bronchi and arterial wall in human lung as subepithelial of smooth muscle cells in colon (Fig. 10) and the B (-long) variant in the epithelium of bronchi and colon (Fig. 11).

A class of GPRA polypeptides can be defined with reference to the exemplary polypeptides defined by SEQ ID NOS: 3, 5, 7, 9, 11, 13 and 15. The class includes allelic, cognate and induced variants of the exemplified polypeptides. Preferred polypeptides shows substantial sequence identity to one or more of the exemplified polypeptides. The class also includes fragments of the exemplified polypeptides having at least 6, 10, 20, 50 100, 200, or 300 contiguous amino acids from one of the above SEQ ID NOS. Some fragments include one or more isolated domains from one of the exemplified polypeptides, e.g., any of the seven transmembrane domains, any of three extracellular loops, any of three intracellular loops, an N-terminal domain and a C-terminal domain. Some polypeptides comprise the amino acid of one of the above SEQ ID NOS. provided that up to 1, 2, 5, 10, 20, 30 or 34 amino acids can be inserted, deleted or substituted relative to the SEQ ID NO. Some GPRA polypeptides comprise an epitope found in one of the exemplified SEQ ID NOS. but not in others. For example, B-Long (SEQ ID NO:5) has a C-terminal epitope of 35 amino acids not found in any of the other forms. Some GPRA polypeptides contain a polymorphic site shown in Table 7 occupied by a variant form.

Preferred GPRA polypeptides are receptors that can be activated to transduce a signal by the same ligand as the exemplified GPRA polypeptide of SEQ ID NOS: 3,5,7,9,11,13, and 15. Binding of a ligand or analog to a G-protein receptor induces an alteration in receptor G-protein interaction. The receptor G-protein interaction releases GDP specifically bound to the G protein and permits the binding of GTP, which activates the G protein. Activated G-protein dissociates from the receptor and activates an effector protein, which in turn regulates intracellular levels of second messengers, such as adenyl cyclase, guanyl cyclase, and phospholipase C. Signal transduction can be monitored in cells transfected with a reporter construct consisting of several copies of the consensus cAMP response element (Arias et al., Nature 370, 226-229 (1994)), a minimal tk promoter, and a secreted alkaline phosphatase gene (Clontech) or can be monitored in changes of the concentrations of intracellular calcium.

### III. AAA1 Polypeptides

The human AAA1 gene can be expressed as at least thirteen different splice variants termed IA, IB, II, III, IVA, IVB, V, VI, VII, VIII, IX, X, and XI. The cDNAs encoding each form and amino acid sequence of each form are shown in Fig. 20 (SEQ ID NOS:16-41). The different splicing events that lead to the different forms are shown in Fig. 14. It can be seen that all splice variants have exon VI and the same core peptide sequence (AYVRRNAGRQFSHCNLHAHQFLVRRKQ). The AAA1 gene is expressed predominantly in the testis, brain, placenta, lung, heart, skeletal muscle, kidney, liver, fetal liver and fetal lung. In addition to the variants described in Fig. 14, using 3'-RACE we have found also several other variants expressed in different tissues. They all share the exon six.

A class of AAA1 polypeptides can be defined with reference to the exemplary polypeptides defined by SEQ ID NOS:17, 19, 21, 23, 25, 27, 29, 31, 33, 35, 37, 39 and 41. The class includes allelic, cognate and induced variants of the exemplified polypeptides. Preferred polypeptides shows substantial sequence identity to one or more of the exemplified polypeptides. The class also includes fragments of the exemplified polypeptides having at least 6, 10, 20, or 50 contiguous amino acids from one of the above SEQ ID NOS. Some fragments include or consist of the above core peptide sequence. Some polypeptides comprise the amino acid of one of the above SEQ ID NOS. provided that up to 1, 2, 5, 10, or 20 amino acids can be inserted, deleted or substituted relative to the SEQ ID NO. Some AAA1 polypeptides comprise an epitope found in one of the exemplified SEQ ID NOS. but not in others. For example, only the variant 1B contains the peptide sequence encoded by exon III.

### IV. GPRA Nucleic Acids

The present application discloses isolated nucleic acids encoding GPRA polypeptides. The nucleic acids can be, for example, genomic, cDNA, RNA or mini-gene (*i*.*e*., a hybrid of genomic and cDNA). Genomic and mini-gene nucleic acids contain at least one intronic segment from a GPRA gene, meaning the section of human chromosome 7p15-p14 having the structure shown in Fig. 3, or allelic and cognate variants thereof. Nucleic acids can include coding regions, intronic regions, 3' untranslated regions, 5'untranslated regions, 3' flanking regions, 5' flanking regions, enhancers, promoters and other regulatory sequences. A class of GPRA nucleic acids can be defined with reference to exemplified GPRA polypeptide and nucleic acid sequences. The class includes allelic, cognate and induced variants of the exemplified sequences, as well as nucleotide substitutions, which due to the degeneracy of the genetic code, do not effect the amino acid sequence of an encoded polypeptide. The class includes nucleic acids that encode the GPRA polypeptides described above. The class also includes GPRA nucleic acids showing substantial sequence identity to exemplified GPRA nucleic acids (*i.e.,* SEQ ID NOS:1, 2, 4, 6, 8, 10, 12 and 14). SEQ ID NO:1 is a genomic sequence representing the part of a GPRA gene designated Ast-1 and shown in Fig. 3. SEQ ID NO:2 is a 1113 bp cDNA encoding GPRA variant A (Fig. 4A). SEQ ID NO:4 is a 1132 bp cDNA encoding GPRA variant B-Long (Fig. 4B). SEQ ID NOS:6, 8, 10, 12, and 14 encode GPRA variants B-Short, C, D, E and F.

The class also includes GPRA nucleic acids that hybridize under highly stringent conditions with at least one of the exemplified nucleic acids. Some GPRA nucleic acids hybridize under highly stringent conditions with at least one of the GPRA nucleic acids without hybridizing under the same conditions to others. For example, some GPRA nucleic acids hybridize to at least one of SEQ ID NOS:1, 4, 6, 8, 10, 12 and 14 without hybridizing to SEQ ID NO:2. Some GPRA nucleic acids include a polymorphic site occupied by a variant form as shown in Table 3 or Table 7. Some GPRA nucleic acids are genomic or minigene. Such nucleic acids contain at least one intronic sequence from any of the intron of a GPRA gene. Inclusion of an intronic sequence can be useful for increasing expression levels of a nucleic acid in cells or transgenic non-human animals. Inclusion of an intronic sequence from introns 2, 3 or 4 is particularly useful for analyzing splice variation of the GPRA gene. Nucleic acids also include fragments of exemplified sequences SEQ ID NOS:1, 4, 6, 8, 10, 12 and 14. The fragments typically contain up to 10, 20, 25, 50, 100, 300, 400, 500 or 1000 or 1500 nucleic acids from any of the above SEQ ID NOS. Optionally, fragments include a polymorphic site shown in Table 3 or 7. The polymorphic site can be occupied by either a reference or variant form.

GPRA nucleic acids can be linked to other nucleic acids with which they are not naturally associated such as vector or a heterologous promoter sequence. Preferred nucleic acids include or are immediately adjacent to at least one of the polymorphic sites shown in Table 3 or 7. Such nucleic acids are useful as primers or probes for detection of specific alleles. Other nucleic acids are useful for expressing proteins encoded by the AST-1 locus in cells or transgenic non-human animals. Other nucleic acids are useful for achieving gene suppression either by homologous recombination to generate a knockout non-human animal or an antisense or siRNA mechanism. Nucleic acids are also useful for identifying, purifying, and isolating nucleic acids encoding other, non-human, mammalian forms of asthma locus-1.

Nucleic acids can be isolated using standard molecular biology techniques and the provided sequence information (Ausubel et al, In Current protocols in Molecular Biology, John Wiley and Sons, publishers, 1989); Sambrook *et al,* supra). Cognate forms (*i.e.*, nucleic acids encoding asthma locus-1 molecules derived from species other than human) or other related sequences (*e.g*., paralogs) can be obtained by low, moderate or high stringency hybridization with all or a portion of the particular human sequence as a probe using methods well known in the art for nucleic acid hybridization and cloning.

### V. AAA1 Nucleic Acids

The present application discloses isolated nucleic acids encoding AAA1 polypeptides. The nucleic acids can be, for example, genomic, cDNA, RNA or mini-gene (*i.e*., a hybrid of genomic and cDNA). Genomic and mini-gene nucleic acids contain at least one intronic segment from an AAA1 gene, meaning the section of human chromosome 7p15-p14 having the structure shown in Fig. 13, or allelic and cognate variants thereof. Nucleic acids can include coding regions, intronic regions, 3' untranslated regions, 5' untranslated regions, 3' flanking regions, 5' flanking regions, enhancers, promoters and other regulatory sequences. A class of AAA1 nucleic acids can be defined with reference to exemplified AAA1 polypeptide and nucleic acid sequences. The class includes allelic, cognate and induced variants of the exemplified sequences, as well as nucleotide substitutions, which due to the degeneracy of the genetic code, do not effect the amino acid sequence of an encoded polypeptide. The class includes nucleic acids that encode the AAA1 polypeptides described above. The class also includes AAA1 nucleic acids showing substantial sequence identity to exemplified AAA1 nucleic acids (*i.e*., SEQ ID NOS:1, 16, 18, 20, 22, 24, 26, 28, 30, 32, 34, 36, 38 and 40). SEQ ID NO:1 is a genomic sequence representing the part of an AAA1 gene designated Ast-1 and shown in Fig. 13. SEQ ID NOS:16, 18, 20, 22, 24, 26, 28, 30, 32, 34, 36, 38 and 40 encode cDNA splice variants shown in Fig. 20.

The class also includes AAA1 nucleic acids that hybridize under highly stringent conditions with at least one of the exemplified nucleic acids. Some AAA1 nucleic acids hybridize under highly stringent conditions with at least one of the exemplified nucleic acids (i.e., SEQ ID NOS:16, 18, 20, 22, 24, 26, 28, 30, 32, 34, 36, 38 and 40) without hybridizing under the same conditions to other exemplified nucleic acids. Some AAA1 nucleic acids include a polymorphic site occupied by a variant form as shown in Table 12. Some AAA1 nucleic acids are genomic or minigene. Such nucleic acids contain at least one intronic sequence from any of the introns of an AAA1 gene. Nucleic acids also include fragments of exemplified sequences SEQ ED NOS:16, 18, 20, 22, 24, 26, 28, 30, 32, 34, 36, 38 and 40. The fragments typically contain up to 10, 20, 25, 50, or 100, nucleotides from any of the above SEQ ID NOS. Optionally, fragments include a polymorphic site shown in Table 12. The polymorphic site can be occupied by either a reference or variant form.

AAA1 nucleic acids can be linked to other-nucleic acids with which they are not naturally associated such as vector or a heterologous promoter sequence. Preferred nucleic acids include or are immediately adjacent to at least one of the polymorphic sites shown in Table 3 or Table 12. Such nucleic acids are useful as primers or probes for detection of specific alleles. Other nucleic acids are useful for expressing AAA1 polypeptides in cells or transgenic non-human animals. Other nucleic acids are useful for achieving gene suppression either by homologous recombination to generate a knockout non-human animal or an antisense or siRNA mechanism. Nucleic acids are also useful for identifying, purifying, and isolating nucleic acids encoding other, non-human, mammalian forms of the AAA1 gene.

As well as or instead of encoding proteins, AAA1 transcripts can have roles in regulating expression of GPRA nucleic acids. A class of noncoding (ncRNA) has been described with regulatory activity in gene transcription and splicing (see e.g. reviews by Michel 2002; Numata *et al.* 2003).

### VI. Antibodies

The GPRA and AAA1 polypeptides are useful for generating antibodies. The antibodies can be polyclonal antibodies, distinct monoclonal antibodies or pooled monoclonal antibodies with different epitopic specificities. Monoclonal antibodies are made from antigen-containing fragments of the protein by standard procedures according to the type of antibody (see, *e.g*., Kohler, et al., Nature, 256:495, (1975); and Harlow & Lane, Antibodies, A Laboratory Manual (C.S.H.P., NY, 1988) Queen et al., Proc. Natl. Acad Sci. USA 86:10029-10033 (1989) and WO 90/07861; Dower et al., WO 91/17271 and McCafferty et al., WO 92/01047. Phage display technology can also be used to mutagenize CDR regions of antibodies Some antibodies bind to an epitope present in one form of GPRA or AAA1 but not others. For example, some antibodies bind to an epitope within amino acids 343-377 of GPRA long B (SEQ ID NO:4). Some antibodies specifically bind to a GPRA polypeptide containing a variant form at a polymorphic site shown in Table 7 without binding to polypeptides containing a reference form at the site. The antibodies can be purified, for example, by binding to and elution from a support to which the polypeptide or a peptide to which the antibodies were raised is bound.

Antibodies are useful, for example, in screening cDNA expression libraries and for identifying clones containing cDNA inserts which encode structurally-related, immunocrossreactive proteins. See, for example, Aruffo & Seed, Proc. Natl. Acad. Sci. USA 84:8573-8577 (1977). Antibodies are also useful to identify and/or purify immunocrossreactive proteins that are structurally related to SEQ ID NOS:3, 5, 7, 9, 11, 13 and 15 and 17, 19, 21, 23, 25, 27, 29, 31, 33, 35, 37, 39 and 41 and fragments thereof used to generate the antibody.

### VII. Polymorphisms

The present application discloses a collection of polymorphic sites both single nucleotide polymorphisms (SNPs) and deletion/insertion polymorphisms (DIPs) in the GPRA gene. SNPs and DIPs can be used in mapping the human genome and, when a SNP or a DIP is linked with a disease or condition, to clarify genetic basis of the disease or condition, in this particular case, at least of asthma.

Table 3 describes 169 SNPs and 18 DIPs occun-ing within exons 3 and 4 and introns 2,3 and 4 of a GPRA gene having variant forms associated with asthma. For AAA1 the corresponding polymorphisms are located in exons 4, 6, 9, 10a /b and introns 2, 3, 4, 5, 6, 7, 8, 9, and 10 (Figure 13). The locus containing these polymorphisms is also referred to as AST-1 (SEQ ID NO:1). Some of the variant forms of these polymorphism may associate with asthma due to an effect on the splicing of GPRA and/or AAA1 gene in the formation of a transcript Other polymorphic sites may be in linkage disequilibrium with such sites or sites in coding regions that exert effects through changing the activity of GPRA or AAA1 polypeptides. Seven different haplotypes H1-H7 were identified that explain 94.5% of the genetic variation detected at the AST-1 locus and are described in Table 3. Haplotype H1 was found identical to the reference sequence NT_000380. The first column in Table 3 indicates the types of polymorphism (*e.g*., SNP or insertion/deletion). The second column indicates the position that the polymorphism occurs in SEQ ID NO:1. The third column indicates the position of the polymorphism occurs in NT_000380. The fourth column indicates several contiguous nucleotides flanking the polymorphic sites and the nature of the variant (i.e., asthma associated) and reference/wildtype polymorphic forms. In all polymorphic sites, the allele present in SEQ ID NO:1 and NT_000380 is indicated first, and the allele from another source second. Columns 5-11 indicate alleles present at that site in haplotypes H1-H7. Column 12 indicates the location of polymorphisms in GPRA and AAA1 exons. In the AST-1 locus, there are coding polymorphism in AAA1 gene (exon 6, Asn ->Ser) and one in GPRA gene (exon 3, Asn -> Ile). Table 13 shows polymorphisms that are unique for haplotypes H1-H7, respectively, and Table 14 polymorphisms that can be employed to recognize spesific haplotype combinations.

The SNPs include the following substitutions with the variant form being indicated before the reference form of SEQ ID NO: 1. Position 1 (preferably T (i.e., variant form) instead of C (reference form)), position 93 (preferably G instead of A), position 918 (preferably A instead of G), position 983 (preferably T instead of A), position 987 (preferably C instead of T), position 1542 (preferably T instead of C), position 1710 (preferably G instead of A), position 1818 (preferably T instead of C), position 1927 (preferably T instead of A), position 2254 (preferably C instead of T), position 2937 (preferably A instead of G), position 3877 (preferably C instead of G), position 4012 (preferably A instead of C), position 4631 (preferably C instead of T), position 4689 (preferably G instead of C), position 4961 (preferably G instead of A), position 5442 (preferably G instead of C), position 5634 (preferably A instead of G), position 5850 (preferably G instead of A), position 6312 (preferably T instead of C), position 6392 (preferably T instead of G), position 6485 (preferably A instead of G), position 6522 (preferably G instead of C), position 6646 (preferably G instead of A), position 6739 (preferably A instead of G), position 6760 (preferably C instead of T), position 7125 (preferably A instead of C), position 7229 (preferably T instead of C), position 7277 (preferably G instead of C), position 7303 (preferably T instead of G), position 7305 (preferably C instead of G), position 7496 (preferably T instead of C), position 7550 (preferably A instead of G), position 8490 (preferably T instead of C), position 9649 (preferably G instead of T ), position 10816 (preferably C instead of T), position 11858 (preferably G instead of A), position 12581 (preferably C instead of G), position 16845 (preferably C instead of T), position 16893 (preferably C instead of T), position 16980 (preferably C instead of T), position 17147 (preferably A instead of G), position 17209 (preferably A instead of C), position 17435 (preferably G instead of A), position 18383 (preferably A instead of G), position 18927 (preferably T instead of G), position 18978 (preferably G instead of A), position 19268 (preferably C instead of G), position 19272 (preferably T instead of A), position 19360 (preferably A instead of G), position 19452 (preferably A instead of G), position 19671 (preferably A instead of G), position 19712 (preferably A instead of C), position 19774 (preferably A instead of C), position 20038 (preferably C instead of T), position 20089 (preferably A instead of T), position 20309 (preferably A instead of G), position 20395 (preferably C instead of T), position 20789 (preferably T instead of G), position 21850 (preferably T instead of C), position 22475 (preferably C instead of T), position 22493 (preferably G instead of A), position 22715 (preferably A instead of G), position 22869 (preferably C instead of T), position 22934 (preferably T instead of A), position 24007 (preferably C instead of T), position 24264 (preferably T instead of G), position 24869 (preferably C instead of T), position 26198 (preferably T instead of C), position 26356 (preferably T instead of C), position 26675 (preferably G instead of A), position 27404 (preferably T instead of C), position 28197 (preferably A instead of G), position 28770 (preferably T instead of C), position 28785 (preferably A instead of G), position 28858 (preferably C instead of T), position 28866 (preferably C instead of G), position 31224 (preferably A instead of G), position 31910 (preferably A instead of G), position 32124 (preferably A instead of C), position 32185 (preferably C instead of T), position 32976 (preferably C instead of T), position 33350 (preferably A instead of C), position 33798 (preferably A instead of G), position 34362 (preferably C instead of G), position 34716 (preferably C instead of A), position 35559 (preferably T instead of C), position 36551 (preferably A instead of G), position 36909 (preferably A instead of C), position 37327 (preferably T instead of G), position 37415 (preferably A instead of G), position 37685 (preferably G instead of A), position 37931 (preferably T instead of C), position 37959 (preferably T instead of C), position 39314 (preferably A instead of G), position 39343 (preferably T instead of G), position 39927 (preferably C instead of T), position 45826 (preferably T instead of C), position 50197 (preferably T instead of G), position 50334 (preferably A instead of G), position 50493 (preferably A instead of G), position 50632 (preferably A instead of G), position 50835 (preferably C instead of A), position 50955 (preferably C instead of G), position 51217 (preferably A instead of G), position 51476 (preferably G instead of A), position 51536 (preferably T instead of C), position 51861 (preferably G instead of C), position 51884 (preferably T instead of G); position 51975 (preferably C instead of G), position 52573 (preferably A instead of G), position 52776 (preferably C instead of G), position 53803 (preferably T instead of C), position 53922 (preferably C instead of T), position 54148 (preferably A instead of G), position 54199 (preferably C instead of T), position 54641 (preferably C instead of G), position 54751 (preferably C instead of T), position 55000 (preferably A instead of G), position 55134 (preferably A instead of G), position 56683 (preferably T instead of C), position 56856 (preferably T instead of C), position 57790 (preferably C instead of A), position 60559 (preferably G instead of C), position 60604 (preferably T instead of A), position 61165 (preferably A instead of G), position 64559 (preferably T instead of C), position 65171 (preferably G instead of A), position 65857 (preferably G instead of T), position 66164 (preferably T instead of C), position 66190 (preferably T instead of C), position 66526 (preferably T instead of C), position 66902 (preferably G instead of A), position 67857(preferably A instead of T), position 67919 (preferably C instead of T), position 72270 (preferably A instead of G), position 75115 (preferably A instead of G), position 76080 (preferably T instead of C), position 76101 (preferably C instead of G), position 81912 (preferably T instead of A), position 82203 (preferably G instead of A), position 82332 (preferably T instead of C), position 82922 (preferably A instead of T), position 83552 (preferably T instead of C), position 85227 (preferably C instead of T), position 85271 (preferably A instead of G), position 107610 (preferably C instead of T), position 110989 (preferably C instead of T), position 111012 (preferably T instead of C), position 112030 (preferably C instead of G), position 112037 (preferably G instead of T), position 112283 (preferably T instead of A), position 112726 (preferably A instead of T), position 112859 (preferably C instead of A), position 113428 (preferably G instead of C), position 113645 (preferably T instead of A), position 113944 (preferably G instead of T), position 114945 (preferably G instead of A), position 115192 (preferably G instead of C), position 115628 (preferably C instead of T), position 116032 (preferably A instead of G), position 116464 (preferably A instead of G), position 116515 (preferably A instead of G), position 116926 (preferably C instead of T), position 117276 (preferably A instead of T), position 123667 (preferably A instead of G), position (preferably 123770 instead G of A), position 123788 (preferably C instead of G), and position 129017 (preferably G instead of A).

DIPS defined relative to SEQ ID NO: 1 include: positions 184-185 (preferably insertion of AAGATA), positions 1202-1206 (preferably deletion of TAAGT), positions 6786-6817 (preferably repeat of (TAAA)₇), position 6821 (preferably deletion of T), positions 7240-7243 (preferably deletion of ACTT), positions 7306-7308 (preferably deletion of TGT), positions 7334-7335 (preferably deletion of AT), positions 9012-9035 (preferably repeat of (CT)₁₀), positions 9199-9201(preferably deletion of TCT),: position 9355-9356 (preferably insertion of T), positions 9782-9785 (preferably deletion of GTCT), positions 22122-22123 (preferably deletion of TG), positions 26929-26968 (preferably repeat of (TAAA)₁₁), positions 28495-28564 (preferably repeat of (CA)₁₂), position 34909 (preferably deletion of T), positions 38850-38852 (preferably deletion of CTC), positions 51022-51049 (preferably repeat of (CA)₈), and positions 52286-52287 (preferably insertion of CC).

Table 7 provide 13 additional polymorphisms occurring within exonic regions of a GPRA gene. The next eight columns indicate the position of a polymorphic site in SEQ ID NO:1, 2, 4, 6, 8, 10, 12 or 14 respectively. The next column indicates a polymorphic site and flanking nucleotides. The form of the polymorphic site present in one or more of SEQ ID NOS:1, 2, 4, 6, 8, 10, 12, and 14 is shown first and the form present in another source is indicated second. In all cases except those indicated by a *, the polymorphic form indicated first is the wildtype or reference form and the polymorphic form indicated second is the asthma or IgE associated form. In the three polymorphic sites indicated with a *, the variant form of the polymorphic site is found in NT_000380 (and SEQ ID NO:1, 2, 4, 6, 8, 10, 12 and/or 14), and the reference or wildtype allele is found in a different individual. The last column of the table shows whether an amino acid change occurs due to the polymorphisms. The positions of these polymorphisms defined with respect to SEQ ID NO:2 are: position 448 (T (variant) preferably to A (wildtype or reference), position 776 (C preferably to T), position 851 (C preferably to G), position 1159 (G preferably to A), position 1199 (T preferably to C), and position 1529 (C preferably to T); or in any one of the following positions as defined by SEQ ID NO:4: position 1206 (A preferably to C), position 1225 (T preferably to C), position 1330 (T preferably to A), and position 1338 (G preferably to A); or in any one of the following positions as defined by SEQ ID NO:8: position 585 (T preferably to C), position 655 (C preferably to A), and position 681 (A preferably to G).

Table 8 shows the five polymorphic sites within Table 7 that showed the highest association with high serum IgE level. Two of the polymorphic sites involved nonconservative amino acid changes, one within the important region of exon 3 within AST-1. Two of the polymorphic sites were within coding regions but did not cause amino acid changes. These sites may show strong association as a result of linkage disequilibrium with causative polymorphic sites. A fifth polymorphic site is within the 3' UTR of exon 9A. This site may show a strong association for the same reason or because the nucleotide substitution has an effect on mRNA stability.

Table 12 shows associations for five polymorphic sites occurring within the AAA1 gene. At each site, the wildtype polymorphic form is indicated first, and the variant form second. The table shows relative frequency of associating haplotypes in individuals with high serum IgE level compared to those in control individuals with low serum IgE level. All haplotypes shown in the table gave chi square values of at least 8.9 (P <0.01) for association.

### VIII. Analysis of Polymorphisms

### A. Preparation of Samples

Polymorphisms are detected in a target nucleic acid from an individual being analyzed. For assay of genomic DNA, virtually any biological sample (other than pure red blood cells) is suitable. For example, convenient tissue samples include whole blood, semen, saliva, tears, urine, fecal material, sweat, buccal, skin and hair. For assay of cDNA or mRNA, the tissue sample must be obtained from an organ in which the target nucleic acid is expressed.

Many of the methods described below require amplification of DNA from target samples. This can be accomplished by *e.g*., PCR. See generally PCR Technology: Principles and Applications for DNA Amplification (ed. H.A. Erlich, Freeman Press, NY, NY, 1992); PCR Protocols: A Guide to Methods and Applications (eds. Innis, et al., Academic Press, San Diego, CA, 1990); Mattila et al., Nucleic Acids Res. 19, 4967 (1991); Eckert et al., PCR Methods and Applications 1,17 (1991); PCR (eds. McPherson et al., IRL Press, Oxford); and U.S. Patent 4,683,202.

Other suitable amplification methods include the ligase chain reaction (LCR) (see Wu and Wallace, Genomics 4, 560 (1959), Landegren et al., Science 241, 1077 (1988), transcription amplification (Kwoh et al., Proc. Natl. Acad. Sci. USA 86, 1173 (1989)), and self-sustained sequence replication (Guatelli et al., Proc. Nat. Acad. Sci. USA, 87, 1874 (1990)) and nucleic acid based sequence amplification (NASBA). The latter two amplification methods involve isothermal reactions based on isothermal transcription, which produce both single stranded RNA (ssRNA) and double stranded DNA (dsDNA) as the amplification products in a ratio of about 30 or 100 to 1, respectively.

### B. Detection of Polymorphisms in Target DNA

The identity of bases occupying the polymorphic sites shown in Table 3, 7 or 12 can be determined in an individual (*e.g*., a patient being analyzed) by several methods, which are described in turn.

### 1. Single Base Extension Methods

Single base extension methods are described by *e.g*., US 5,846,710, US 6,004,744, US 5,888,819 and US 5,856,092. In brief, the methods work by hybridizing a primer that is complementary to a target sequence such that the 3' end of the primer is immediately adjacent to but does not span a site of potential variation in the target sequence. That is, the primer comprises a subsequence from the complement of a target polynucleotide terminating at the base that is immediately adjacent and 5' to the polymorphic site. The hybridization is performed in the presence of one or more labeled nucleotides complementary to base(s)that may occupy the site of potential variation. For example, for a biallelic polymorphisms two differentially labeled nucleotides can be used. For a tetraallelic polymorphism four differentially labeled nucleotides can be used. In some methods, particularly methods employing multiple differentially labeled nucleotides, the nucleotides are dideoxynucleotides. Hybridization is performed under conditions permitting primer extension if a nucleotide complementation a base occupying the site of variation in the target sequence is present. Extension incorporates a labeled nucleotide thereby generating a labeled extended primer. If multiple differentially labeled nucleotides are used and the target is heterozygous then multiple differentially labeled extended primers can be obtained. Extended primers are detected providing an indication of which bas(s) occupy the site of variation in the target polynucleotide. The methods are particularly useful for SNPs.

### 2. Allele-Specific Probes

The design and use of allele-specific probes for analyzing polymorphisms is described by *e.g.,* Saiki et al., Nature 324, 163-166 (1986); Dattagupta, EP 235,726, Saiki, WO 89/11548. Allele-specific probes can be designed that hybridize to a segment of target DNA from one individual but do not hybridize to the corresponding segment (corresponding segments being defined by maximum alignment of the sequences being compared) from another individual due to the presence of different polymorphic forms in the respective segments from the two individuals. Hybridization conditions should be sufficiently stringent that there is a significant difference in hybridization intensity between alleles, and preferably an essentially binary response, whereby a probe hybridizes to only one of the alleles. Some probes are designed to hybridize to a segment of target DNA such that the polymorphic site aligns with a central position (*e.g*., in a 15 mer at the 7 position; in a 16 mer, at either the 8 or 9 position) of the probe. This design of probe achieves good discrimination in hybridization between different allelic forms.

Allele-specific probes are often used in pairs, one member of a pair showing a perfect match to a reference form of a target sequence and the other member showing a perfect match to a variant form. Several pairs of probes can then be immobilized on the same support for simultaneous analysis of multiple polymorphisms within the same target sequence.

### 3. Tiling Arrays

SNPs and insertion deletion polymorphism can be detected by hybridizing target nucleic acids to arrays of probes tiling a reference sequence or one or more variant forms of the reference sequence as described by WO 95/11995.

### 4. Allele-Specific Amplification Methods

An allele-specific primer hybridizes to a site on target DNA overlapping a polymorphism and only primes amplification of an allelic form to which the primer exhibits perfect complementarily. See Gibbs, Nucleic Acid Res. 17, 2427-2448 (1989). This primer is used in conjunction with a second primer which hybridizes at a distal site. Amplification proceeds from the two primers leading to a detectable product signifying the particular allelic form is present. A control is usually performed with a second pair of primers, one of which shows a single base mismatch at the polymorphic site and the other of which exhibits perfect complementarily to a distal site. The single-base mismatch prevents amplification and no detectable product is formed. In some methods, the mismatch is included in the 3'-most position of the oligonucleotide aligned with the polymorphism because this position is most destabilizing to elongation from the primer. See, *e.g.*, WO 93/22456.

### 5. Direct-Sequencing

The direct analysis of the sequence of polymorphisms disclosed herein can be accomplished using either the dideoxy- chain termination method or the Maxam -Gilbert method (see Sambrook et al., Molecular Cloning, A Laboratory Manual (2nd Ed., CSHP, New York 1989); Zyskind et al., Recombinant DNA Laboratory Manual, (Acad. Press, 1988)).

### 6. Denaturing Gradient Gel Electrophoresis

Amplification products generated using the polymerase chain reaction can be analyzed by the use of denaturing gradient gel electrophoresis. Different alleles can be identified based on the different sequence-dependent melting properties and electrophoretic migration of DNA in solution. Erlich, ed., PCR Technology, Principles and Applications for DNA Amplification, (W.H. Freeman and Co, New York, 1992), Chapter 7.

### 7. Single-Strand Conformation Polymorphism Analysis

Alleles of target sequences can be differentiated using single-strand conformation polymorphism analysis, which identifies base differences by alteration in electrophoretic migration of single stranded PCR products, as described in Orita et al., Proc. Nat. Acad. Sci. 86, 2766-2770 (1989). Amplified PCR products can be generated as described above, and heated or otherwise denatured, to form single stranded amplification products. Single-stranded nucleic acids may refold or form secondary structures which are partially dependent on the base sequence. The different electrophoretic mobilities of single-stranded amplification products can be related to base-sequence difference between alleles of target sequences.

### 8. Other Methods

Include oligonucleotide ligation assay or restriction fragment length polymorphism (RFLP) (see, for example, Current Protocols in Molecular Biology, eds. Ausubel et al, John Wiley & Sons: 1992, and Landegren et al, "Reading Bits of Genetic Information: Methods for Single-Nucleotide Polymorphism Analysis", Genome Research 8:769-776).

### VII. Methods of Use

### A. Discovery of New Polymorphic Sites in AST-1 Locus

Additional polymorphic sites beyond those listed in Tables 3, 7 and 12 within GPRA and AAA1 genes and particularly, the AST-1 locus thereof can be identified by comparing a GPRA or AAA1 nucleic acid from different individuals with a reference sequence, such as SEQ. ID NO:1, 2, 4; 6, 8, 10, 12 or 14 or SEQ ID NOS:16, 18, 20, 22, 24, 26; 28, 30, 32, 34, 36, 38 or 40. Preferably, the different individuals suffer or at risk of an immune disorder, such as autoimmune disease, an IgE-mediated disorder, such as asthma chronic obstructive pulmonary disease or cancer. If a new polymorphic site (*e*.*g*., SNP) is found, then the link with a specific disease can be determined by comparing the frequency of variant polymorphic forms occupying this site in populations of patients with or without a disease being analyzed (or susceptibility thereto). A polymorphic site or region may be located in any part of the locus, *e.g.,* exons, introns and promoter regions of genes.

### B. Diagnostic and Detection Methods

The present application further discloses means for prognostic or diagnostic assays for determining if a subject has or is increasingly likely to develop a disease associated with the variation or dysfunction of the asthma locus disclosed herein. Such diseases include in addition to asthma, other IgE mediated diseases, autoimmune diseases, atopic and other immune diseases, and chronic obstructive pulmonary disease. Basically, such assays comprise a detection step, wherein the presence or absence of a variant polymorphic form in the asthma locus-1 relative to SEQ ID NO:1, 2, 4, 6, 8, 10, 12, or 14 or SEQ-ID NO: 16, 18, 20, 22, 24, 26, 28, 30, 32, 34,36,38 and 40 is determined in a biological sample from the subject. The detection step can be performed by any of the methods described above. Analogous assays can be performed in which variations in protein sequence relative to SEQ ID NOS:3, 5, 7, 9, 11, 13 or 15 or SEQ ID NO: 17, 19, 21, 23, 25, 27, 29, 31, 33, 35, 37, 39 and 41 are determined.

In particular, the present application is directed to a method of determining the presence or absence of a variant form of a polymorphic site in a biological sample from a human for diagnosis of asthma or for assessing the predisposition of an individual to asthma, other IgE-mediated disease, or chronic obstructive pulmonary disease. The method comprises determining the sequence of the nucleic acid of a human at one or more the above SNP or DIP positions shown in Tables 3, 7 and 12. Optionally, the sample is contacted with oligonucleotide primers so that the nucleic acid region containing the potential single nucleotide polymorphism is amplified by polymerase chain reaction prior to determining the sequence.

The polymorphic sites can be analyzed individually or in sets for diagnostic and prognostic purposes. The conclusion drawn from the analysis depends on the nature and number of polymorphic sites analyzed. Some polymorphic sites have variant polymorphic forms that are causative of disease. Detection of such a polymorphic form provides at least a strong indication of presence or susceptibility to disease. Other polymorphic sites have variant polymorphic forms that are not causative of disease but are in equilibrium dislinkage with a polymorphic form that is causative. Detection of noncausative polymorphic forms provides an indication of risk of presence or susceptibility to disease. Detection of multiple variant forms at several polymorphic sites in a GPRA and or AAA1 gene and particularly the parts of these genes within the AST-1 locus thereof provides an indication of increased risk of presence or susceptibility to disease. The results from analyzing the polymorphic sites disclosed herein can be combined with analysis of other loci that associate with the same disease (*e*.*g*., asthma). Alternatively, or additionally risk of disease can be confirmed by performing conventional medical diagnostic tests of patient symptoms.

### C. Clinical Trials

The polymorphisms disclosed herein are also useful for conducting clinical trials of drug candidates for the diseases noted above particularly asthma. Such trials are performed on treated or control populations having similar or identical polymorphic profiles at a defined collection of polymorphic sites. Use of genetically matched populations eliminates or reduces variation in treatment outcome due to genetic factors, leading to a more accurate assessment of the efficacy of a potential drug.

Furthermore, the polymorphisms disclosed herein may be used after the completion of a clinical trial to elucidated differences in response to a given treatment. For example, the set of polymorphisms may be used to stratify the enrolled patients into disease sub-types or classes. It may further be possible to use the polymorphisms to identify subsets of patients with similar polymorphic profiles who have unusual (high or low) response to treatment or who do not respond at all (non-responders). In this way, information about the underlying genetic factors influencing response to treatment can be used in many aspects of the development of treatment (these range from the identification of new targets, through the design of new trials to product labeling and patient targeting). Additionally, the polymorphisms may be used to identify the genetic factors involved in adverse response to treatment (adverse events). For example, patients who show adverse response may have more similar polymorphic profiles than would be expected by chance. This allows the early identification and exclusion of such individuals from treatment. It also provides information that can be used to understand the biological causes of adverse events and to modify the treatment to avoid such outcomes.

### VIII. Kits

The application also features diagnostic or prognostic kits for use in detecting the presence of a SNP in a GPRA and/or AAA1 gene or particularly an AST-1 locus thereof in a biological sample. The kit provides means for the diagnostics of asthma, other IgE-mediated disease, or chronic obstructive pulmonary disease or for assessing the predisposition of an individual to a disease mediated by variation or dysfunction of a GPRA and/or AAA1 gene. The kit can comprise a labeled compound capable of detecting the nucleic acid of a GPRA and/or AAA1 gene in a biological sample. The kit can also comprise nucleic acid primers or probes capable of hybridizing specifically to at least of portion of a GPRA gene. Preferably, the primer is a minisequencing primer specific to any one of above polymorphisms. The kit can be packaged in a suitable container and preferably it contains instructions for using the kit.

Often, the kits contain one or more pairs of allele-specific oligonucleotides hybridizing to different-forms of a polymorphism. In some kits, the allele-specific oligonucleotides are provided immobilized to a substrate. For example, the same substrate can comprise allele-specific oligonucleotide probes for detecting at least 10, 50 or all of the polymorphisms shown in Tables 3, 7, 12, 13, and 14. Optional additional components of the kit include, for example, restriction enzymes, reverse-transcriptase or polymerase, the substrate nucleoside triphosphates, means used to label (for example, an avidin-enzyme conjugate and enzyme substrate and chromogen if the label is biotin), and the appropriate buffers for reverse transcription, PCR, or hybridization reactions.

### IX. Transgenic non-human Animals and Cells

The present application also makes available plasmids and vectors comprising nucleic acid, which constructs can be used when studying biological activities of the AST-1 locus. The selecting of a suitable plasmid or vector for a certain use is within the abilities of a skilled artisan. As the host cell may be any prokaryotic or eukaryotic cell, a plasmid or vector comprising the locus can be used in the duplication of the locus (see, for example, Sambrook et al, "Molecular Cloning: A Laboratory Manual", Second Edition, Cold Spring Harbor, New York: Cold Spring Harbor Laboratory Press (1989)).

Nucleic acids can be used to generate either transgenic non-human animals or "knock out" non-human animals which, in turn, are useful in the development and screening of therapeutically useful reagents. A transgenic non-human animal (*e.g.,* a bovine, pig, sheep, rabbit, rat, mouse or other rodent) is an animal having cells that contain a transgene, which transgene was introduced into the animal or an ancestor of the animal at an embryonic (e.g., one-cell) stage. Often all or substantially all of the somatic and germline cells of the transgenic non-human animal have a copy of the transgene in their genome. A transgene is a DNA which is integrated into the genome of a cell from which a transgenic non-human animal develops. Methods for generating transgenic non-human animals, particularly animals such as mice, have become conventional in the art and are described, for example, in U.S. Pat. Nos. 4,736,866 and 4,870,009. Typically, a GPRA and/or AAA1 nucleic acid is operably linked to a promoter and optionally an enhancer. The promoter and enhancer can be selected confer tissue specific expression. Transgenic non-human animals that include a copy of a transgene introduced into the germ line of the animal at an embryonic stage can be used to examine the effect of increased expression of GPRA and/or AAA1 polypeptides. Such animals can be used as tester animals for reagents thought to confer protection from, for example, diseases related to GPRA and/or AAA1. Such a transgenic non-human animal can be treated with agent and a reduced incidence of a characteristic of disease compared to untreated animals (i.e., without the agent) bearing the transgene indicates a potential therapeutic intervention for the disease. Results from animal models can be confirmed in placebo-controlled clinical trials on patients with asthma, other IgE mediated disease, chronic obstructive pulmonary disease or cancer.

Transgenic offspring are identified by demonstrating incorporation of the microinjected transgene into their genomes, preferably by preparing DNA from short sections of tail and analyzing by Southern blotting for presence of the transgene ("Tail Blots"). A preferred probe is a segment of a transgene fusion construct that is uniquely present in the transgene and not in the mouse genome. Alternatively, substitution of a natural sequence of codons in the transgene with a different sequence that still encodes the same peptide yields a unique region identifiable in DNA and RNA analysis. Transgenic "founder" mice identified in this fashion are bred with normal mice to yield heterozygotes, which are backcrossed to create a line of transgenic mice. Tail blots of each mouse from each generation are examined until the strain is established and homozygous. Each successfully created founder mouse and its strain vary from other strains in the location and copy number of transgenes inserted into the mouse genome, and hence have widely varying levels of transgene expression or activity.

Knockout non-human animals or cells can also be made by functionally disrupting an endogenous cognate form of one or more genes within the AST-1 locus (i.e., GPRA and/or AAA1). Functional disruption means that the transgenic non-human animal is incapale of making a functional GPRA or AAA1 polypeptide and/or transcript encoding the same. Inactivation of a protein can be achieved by forming a transgene in which a cloned variant gene is inactivated by insertion of a positive selection marker within the coding region. See Capecchi, Science 244, 1288-1292 (1989). Inactivation of a transcript can be achieved by mutation within a promoter region. The transgene is then introduced into an embryonic stem cell, where it undergoes homologous recombination with an endogenous variant gene. Mice and other rodents are preferred animals. Such animals provide useful drug screening systems.

### X. Methods of Screening for Drugs

The GPRA and AAA1 polypeptides and nucleic acids are useful for screening for agents that modulate (e.g., agonize or antagonize) their function (e.g., as a G-protein coupled receptor). Such agents can be useful as drugs in compensating for genetic variations affecting GPRA and/or AAA1 function. For example, an agonist of GPRA and/or AAA1 function is useful in a patient having a genetic variation that decreases endogenous GPRA function, and an antagonist is useful in a patient having a genetic variation that increases endogenous GPRA and/or AAA1 function. GPRA and AAA1 polypeptides can also be used to screen known drugs to determine whether the drug has an incidental effect on GPRA and/or AAA1 activity. A drug that has an incidental agonist activity should generally be avoided in patients having atypically high levels of IgE antibodies, and an antagonist should generally be avoided in immunosuppressed patients.

Agents can initially be screened in a binding assay with a GPRA or AAA1 polypeptides. A binding assay reduces the pool of initial agents to a subset that can be screened by a functional assay. Agents can be screened in cells transfected with nucleic acids encoding a GPRA and/or AAA1 polypeptide or in transgenic non-human animals transfected with a GPRA and/or AAA1 nucleic acids and optionally and a reporter construct containing cAMP response element, a minimal promoter and a coding sequence for secreted alkaline phosphatase. Methods for identifying agents that have a functional activity in transducing a signal through a cellular receptor are described by US 6,309,842. Agents for screening can be obtained by producing and screening large combinatorial libraries, as described above, or can be known drugs, or can be known ligands to related receptors to GPRA.

Agents can also be screened for capacity to inhibit or increase expression of GPRA and/or AAA1 nucleic acids. Suitable agents to screen include antisense polynucleotides, see Antisense RNA and DNA, (1988), D.A. Melton, Ed., Cold Spring Harbor Laboratory, Cold Spring Harbor, NY); Dagle et al., Nucleic Acids Research, 19:1805 (1991); Uhlmann et al., Chem. Reviews, 90:543-584 (1990), zinc finger proteins (see WO 00/00388 and EP.95908614.1), and siRNAs WO 99/32619, Elbashir, EMBO J. 20,6877-6888 (2001) and Nykanen et al., Cell 107, 309-321 (2001); WO 01/29058.

Agents having pharmacological activity evidenced by cellular or animal testing can be subject to clinical trials in patients suffering from asthma, other IgE-mediated disease, chronic obstructive pulmonary disease or cancer in comparison with a placebo.

### MATERIALS AND METHODS

Families were recruited in central eastern Finland in 1994 and 1996: The methods for recruitment, control for population stratification, and clinical evaluation have been described previously in detail (Kauppi *et al.* 1998; Laitinen *et al.* 1997). We used self-reported asthma as a sampling method. Altogether 253 families were recruited, two thirds of which were trios. Based on retrospective verification of the disease history and the results of diagnostic tests (spirometry, histamine or methacholine challenge test, expiratory peak flow measurements), 87% of the self-reported asthma patients were accepted as verified cases (Kauppi *et al* 1998). Criteria for asthma were based on the recommendations of the American Thoracic Society (Dantzker, D.R. *et al.* 1987). In addition to the 86 multiplex pedigrees included already into our previous genome scan, 103 nuclear families with full phase information (a total of 874 study individuals) were included to the association analysis without further selection.

Total serum IgE level was determined by Diagnostics CAP FEIA (Kabi Pharmacia, Sweden) in one batch. Allergy screening was done to all study individuals at the time of the study (Kauppi *et al.* 1998; Laitinen *et al.* 1997). Based on total serum IgE level the study individuals were divided into two groups: high IgE responders (IgE > 100kU/L) and low IgE responders (IgE ≤ 100 kU/L).

### EXAMPLES

### EXAMPLE I

### Construction Of The Physical Map

We have ordered the markers used in the analysis and estimated the physical distance between the markers using several publicly available sources as described previously by Polvi *et al* (2002). Completed chromosome 7 sequence confirms the distance and order of our markers
(http://www.ncbi.nlm.nih.gov/entrez/query.fcgi?cmd=Retrieve&db=Nucleotide&list_uids=22 096428&dopt=GenBank).

### EXAMPLE II

### Microsatellite Discovery and Genotyping

To create a dense map of polymorphic markers spread evenly across the linkage region, we screened the publicly available genomic sequences for potentially polymorphic tandem repeats (Polvi *et al.* 2002). Eighty microsatellites were genotyped in the whole data set (874 samples) giving the average marker density of 220kb (range from 904bp to 790kb) across the linkage region. All microsatellite markers and small insertions and deletions (Table 1C) found in the critical region were genotyped using fluorescently labeled primers in gel electrophoresis on the ABI377 sequencer as described previously (Polvi *et al.* 2002 and http://www.genome.helsinki.fi/eng/research/asthma/detail.htm).

### EXAMPLE III

### Discovery of Genetic Variation in the Critical Region by Direct Sequencing

To identify all sequence polymorphisms in the critical region, we generated a 129 kb reference sequence by assembling the sequence (genomic clones AC005680, AC005492, AC005174, AC005862.1, and AC005853.1) from the Human Genome Project (in the contig NT_000380 from the position 509,783 to the position 638,799). SNP discovery by re-sequencing was done in four selected patients and in three control individuals who were homozygous across the region and for the flanking markers. Repeat regions such as SINE, LINE, LTR, MER1 and MER2 elements covered 60% of the sequence (a total of 129,017 bp). PCR assays were designed to be ~650 bp in length with 100 bp overlap with adjacent assays. Repeat regions were re-sequenced when it was possible to design primers in unique sequence within the range mentioned above.

PCR assays were carried out in 20µl volumes containing 20ng of genomic DNA, 1x PCR Buffer II, 0.1mM dNTPs, 2.5 mM MgCl2, 0.1µM primer mix, and 0.5 U of DNA polymerase (AmpliTaqGold, Applied Biosystems). The samples were denatured for 10 min at 94°C, followed by 35 cycles each of 30s at 94 C, 30 s at 58 C, and 30 s at 72 °C. Elongation was performed for 10 min at 72°C. Purified PCR fragments (Quickstep 2 PCR purification Kit, Edge BioSystems, MD) were sequenced in both directions using ABI Prism3100 sequencer and dye-terminator chemistry. We assembled the sequence reads using the Gap4 program.

### EXAMPLE IV

### SNP Genotyping

SNP genotyping was done using two different methods: single base pair extension (SBE) or using altered restriction sites. SBE was done using the chemistry of Molecular Dynamics according to the suggestion made by the manufacturer on a Megabase 1000 sequencer (Molecular Dynamics, CA) using the primers listed in the Table 1A. Allele calling was performed by using the MegaBACE SNP Profiler software (Molecular Dynamics).

Twenty-nine of the SNPs were genotyped using different restriction enzyme digestions. All primers, restriction enzymes, and lengths of the digestion fragments of corresponding allele used in the genotyping are given in Table 1B. If the SNP did not produce a natural site for altered restriction, mutations were induced in PCR-primers (shown in capital letters in primer sequence). To improve allele calling by growing size difference between alleles, in some primers plasmid sequence tail of 20-30bp was added (shown in bold in the primer sequence). Altered restriction sites of the PCR products stained with ethidium bromide and visualized on agarose gels in UV light were called manually by two independent observers. All the markers were in Hardy-Weinberg equilibrium and observed Mendel errors were less than 0.1%.

### EXAMPLE V

### Haplotype Association Analysis

The haplotype analysis was done using Haplotype Pattern Mining (HPM) (Toivonen *et al.* 2000) program. For haplotyping, large pedigrees were divided into trios using an in-house computer program. The program identifies the maximum number of trios that are not overlapping and in which one or two members were affected (not both parents). Trios that included members who had not been genotyped or members with unknown phenotype were excluded. In phase three of hierarchical LD mapping, high density genotyping of the critical region was done in the trios informative for the association analysis (a total of 132 trios, 396 study individuals, and revealing 304 unrelated affected and 220 control chromosomes). Haplotyping was done within each trio and four independent chromosomes were obtained from each trio. In case of ambiguities (missing genotype data, identical heterozygotic genotypes in all of the family members, or Mendel errors), the alleles were discarded. If the child was affected, the transmitted chromosomes were considered disease associated and the non-transmitted chromosomes as controls. If one of the parents was affected, his/her chromosomes were considered disease associated and the spouse's chromosomes as controls. If both the parent and the child were affected, only the non-transmitted chromosome of an unaffected parent was considered as the control and the other three as disease associated. These haplotypes were used as input for HPM.

To estimate the overall significance of detected association, accounting for simultaneous testing of multiple markers, we used a second level permutation test by performing nested permutation tests described in detail elsewhere (Sevon *et al.* 2001 a and b).

### RESULTS

### 1. Haplotype Association

Our genotyping effort searching for a shared haplotype among individuals who have high total IgE serum level was done in three phases using a hierarchical approach. Based on the initial observation on potential association, the marker map was made denser in the regions of primary interest. In the haplotype analysis we used the HPM algorithm that is known to be powerful in locating a disease-causing gene when one is known to exist (Toivonen *et al.* 2000). By allowing gaps in the haplotype patterns, the algorithm is robust for genotyping errors, marker mutations, unrecognized recombinations and missing data. The data set consisted of 86 multiplex pedigrees that were included also in the previous linkage study and additional 103 nuclear families recruited solely for the association study. These families revealed 304 unrelated high IgE associated and 220 control chromosomes which were then used as the input data for the HPM analysis.

First we built a marker map across the whole linkage region with 80 microsatellite markers (Fig. 1). In the first phase of haplotype association analysis the marker density was approximately 220kb across the linkage region. The best associations (chi square ≥7.4) formed only one cluster of haplotypes located between the markers D7S690-NM13 (~3.21cM). In the second phase we added 6 new microsatellites in between D7S690 and NM13. The associated haplotypes (chi square ≥8.9) clustered now at the centromeric end of the region, between markers G42099 and NM13 (301kb) (Fig. 2). HPM algorithm was used to evaluate the overall haplotype distribution among the high IgE associated compared to that among control chromosomes. A permutation test incorporated to the HPM computer package was used to evaluate the statistical significance of our finding by comparing the overall haplotype distribution among the high IgE associated and control chromosomes. Using chi square 7.0 as the threshold for association, 7 markers as the maximum length of the haplotype, and allowing one gap in the haplotype patterns, the best associations were computed for three adjacent markers G42102, D7S497, and G4296 (marker-wise P values 0.05-0.06 based on 10 000 simulations).

Fine mapping was then further continued by adding 49 new markers between G42099 and NM13 giving an average marker density of 6kb. By using the HPM algorithm, all associated haplotype (chi square >13) patterns spanned between SNP509783 and SNP638799 (129,017 bp).

To study if the overall haplotype distribution in the locus differs in high IgE associated compared to that in control chromosomes, we used again a permutation test. HPM analysis was done using the following parameters: maximum pattern length 40 markers, one gap allowed for missing data and possible errors, and chi-square threshold for the association 13.0. The observed scores (=number of qualified haplotype patterns spanning across the marker) for associated haplotypes varied from 0 to 40. Permutation test showed statistically biased haplotype distribution for high IgE. The marker-wise P values ≤0.005 were observed for all eleven of the markers between SNP531632 and SNP563930 and the marker wise P values ≤0.01 for all twenty-eight markers between NM51 and SNP617392 based on 10 000 simulations. To estimate the effect of simultaneous testing of multiple markers, we used a second level permutation test, yielding a corrected P value of 0.03 (Sevon 2001a and b).

### 2. Genetic Variation in the Critical Region

Based on haplotype analysis we had determined the critical region in between the markers SNP509783 and SNP638799 (Fig. 2). SNP genotyping resulted in the identification of seven haplotypes H1-H7 that together explain 94.5% of the genetic variation detected at the AST-1 locus. To identify all potentially causative genetic variations on the risk haplotype, we sequenced four patients who carried haplotypes H2/H2, H4/H4, H5/H5, and H7/H7, respectively, and were homozygous across the AST1-locus region. The patients' sequences polymorphisms were compared with the reference sequence (SEQ ID NO:1) **(http://www.ncbi.nlm.nih.gov/entrez/guery.fcgi?cmd=Retrieve&db=nucleotide&list uid s=7382309&dopt=GenBank,** human genomic contig NT_000380) and to three population based controls homozygous for the same markers, but with different haplotypes H1/H1, H3/H3, and H6/H6, respectively. The differences are highlighted via SEQ ID NO:1. Genetic variation including small deletions and insertions (≤ 6 base pairs), variation in the length of microsatellite repeats, and multiple SNPs are presented in Table 3 that can be used as genetic markers of the risk haplotypes. Tables 13 and 14 show the SNPs unique for each haplotype and unique for certain haplotype combinations, respectively. In diagnostic testing these markers identify the haplotypes and the haplotype combinations of an individual without phase information.

### Example VI: Identification of the GPRA Gene

### A. Materials and Methods

### 1. Exon Prediction

Exon predictions were performed by GENSCAN http://genes.mit.edu/GENSCAN.html) and software using genomic clones from AC005493.1 to AC005826.1 (690 kb) (Polvi *et al.* 2002) as one block. Predictions were performed for both unmasked and masked sequence. In the latter, the repetitive sequences were masked using the Repeat Masker Service (http://repeatmasker.genome.washington.edu/).

### 2. Reverse Transcriptase PCR

The exon-specific primers were designed based on exon predictions (Table 4, Fig. 3). First strand cDNAs were synthesized from 1 µg of commercially available poly A⁺ RNAs from human brain (Invitrogen), lung, testis, placenta, and thymus (BD Clontech) by the SMART RACE cDNA Amplification Kit (BD Clontech, Palo Alto, CA) according to manufacturer's instructions. Alternatively, Marathon-Ready cDNA (Clontech) or cDNA from Multiple Tissue cDNA Panels I and II (Clontech) were used as templates in some PCR amplifications. All the PCR amplifications were performed in 20-50 µl volumes using 2.0-5.0 µl cDNA as template, 2 mM MgCl₂, 0.2 mM dNTPs (Finnzymes), 0.2 mM of each primer, 0.15-0.4 U AmpliTaqGold, and 1x PCR Gold buffer (Applied Biosciences) under the following conditions: 94°C for 10 min, 35-40 cycles of 94 °C for 30 s, 58 °C -68 °C for 30 s, 72 °C for 1 min 40 s to 2 min followed by 72 °C for 10 min. PCR products were analyzed on 1 % agarose gel and extracted from the gel using QIAquick Gel Extraction Kit (Qiagen).

### 3. Rapid Amplification of cDNA Ends (RACE-PCR)

To generate 3' and 5' cDNA ends, rapid amplification of cDNA ends (SMART RACE) was performed using human testis cDNA and Human Marathon-Ready Fetal Thymus cDNA (Clontech) according to the manufacturer's protocol for the SMART RACE cDNA Amplification Kit (BD Clontech). RACE-PCR products were cloned using pGEM-T Easy Vector system (Promega) or TOPO TA Cloning kit (Invitrogen) and plasmid DNA was purified using QIAprep Spin Miniprep Kit (Qiagen).

The purified RT-PCR products and the cloned RACE-PCR products were verified by automated sequencing with dye-terminator chemistry (ABI Prism3100, Applied Biosystems, Inc).

### 4. Cloning of the Full-Lenggh cDNAs of GPRA

Nested PCR amplification was used in cloning of the full length cDNAs for *GPRA* splice variants A and B. The first round of PCR for variant A was done with the primer pair JEGE1F1 and JEGE9aR1, and for variant B with JEGE1F1 and JEGE9bR1 (Table 4, Fig. 3). The following inner primers were used in the re-amplification: JEGE1F2 and JEGE9aR2 for variant A; JEGE1F2 and JEGE9bR2 for variant B, respectively. Full length C variant was amplified with the primer pair JEGE1F2 and JEGExR1.

Primary PCR amplifications of variants A and B were performed in 25 µl volumes using 2.5 µl Human Brain Marathon-Ready cDNA (Clontech) as template, 1x DyNAzyme EXT buffer (containing 1.5 mM MgCl₂), 0.2 mM dNTPs (Finnzymes), 0.52 µM of each primer, 5% DMSO and 0.5 U DyNAzyme EXT polymerase (Finnzymes) under the following conditions: 94°C for 4 min, 38 cycles of 94°C for 30 s, 65°C for 30 s, 72°C for 1 min followed by final extension of 72°C for 10 min. The aliquot of primary PCR product was re-amplified by 30 PCR cycles under the same conditions as above. PCR amplification of variant C was performed in the above conditions. NCI-H358 cDNA was used as template.

PCR products were cloned into pCR 2.1 TOPO -vector using TOPO TA cloning kit (Invitrogen) according to manufacturer's instructions and plasmid DNA were purified using QIAprep Spin Miniprep Kit (Qiagen). The cloned RT-PCR products were verified by automated sequencing with dye-terminator chemistry (MegaBACE 1000, Molecular Dynamics).

### 5. Cell Culture and Isolation of Poly A⁺ RNA

In addition to commercially available poly A⁺ RNAs and Marathon-Ready cDNAs, human lung epithelial carcinoma cell line NCI-H358 (ATCC) was cultured as mRNA source for expression studies. Cells were cultured in RPMI 1640 medium (Gibco BRL) supplemented with 1 mM sodium puryvate (Gibco BRL), 10% FCS (Biological Industries), and 1% Penicillin/Streptomycin (GibcoBRL). Poly A⁺ RNA was isolated by Dynabeads mRNA DIRECT Kit (Dynal) according to the manufacturer's instructions.

### 6. Northern Blot Hybridization

The GPRA C specific (comprising the nucleotides from 58 to 527 of the *GPRA* C variant) 470 bp probe was generated by RT-PCR using human testis cDNA as template. The probe was radiolabelled with α[³²P]-dCTP using RediPrime Kit (Amersham Biosciences) according to the manufacturer's instructions. A Human Multiple Tissue 8-lane Northern blot (BD Clontech) was prehybridized in ExpressHyb solution (BD Clontech) for 1 h at 68 °C followed by hybridization with the specific probe for 1 h 15 min at 68 °C. Herring sperm DNA (100 µg/ml) was used as the blocking reagent. The filter was washed with 2 x SSC and 0.05 % SDS at room temperature and then exposed to X-ray film at -20 °C for 6 days.

### 7. Western Blot and Immunohistochemistry

Two specific antibodies against the alternative carboxy terminals of *GPRA* were raised by immunizing rabbits with the following peptides: CREQRSQDSRMTFRERTER (corresponding to the nucleotides 1148-1205 of the variant A) and CPQRENWKGTWPGVPSWALPR (corresponding to the nucleotides 1196-1259 of the variant B of *GPRA*). GPRA A peptide synthesis and antibody production were purchased from Sigma-Genosys Ltd (London Road, Pampisford, Cambridge). GPRA B peptide synthesis was purchased from the University of Helsinki and antibody production from the University of Oulu. A total of 6 immunizations were performed at 2 weeks intervals using the total of 2 mg of KLH-conjugated peptide purified by gel filtration. GPRA antibodies were purified from whole serum by affinity chromatography with the peptide coupled to iodoacetyl on a crosslinked agarose support according to the manufacturer's (Pierce, Meridian Road, Rockford IL, USA) instructions.

For western blot analysis, human tissue lysates from spleen, skeletal muscle, uterine muscle, colon muscle, kidney, colon epithelium, testes, and prostate were obtained by mechanically homogenizing the frozen tissue samples in 10 mM Tris HCl 100mM NaCl 2 % Triton X-100 buffer with proteinase inhibitors. 50 µg of the protein lysates were run on reducing 12.5 % SDS-PAGE gels and electroblotted to the PVDF membrane according to standard procedures. Nonspecific protein binding was prevented by incubating the membrane with 5 % milk in 0.1 % Tween 20 in TBS (TBST). Antigenic sites were revealed by incubating the membrane with the anti-GPRA C-terminal antibodies or pre-immune serum followed by the alkaline phosphatase-conjugated goat anti-rabbit secondary antibody (Jackson ImmunoResearch Laboratories Inc., West Baltimore Pike West Grove, PA, USA) in 5 % milk in 0.1 % TBST.

The color reaction was revealed by the NBT/BCIP method (Pierce). Negative controls did not show specific reactivity.

Formalin fixed, paraffin-embedded specimens of normal adult human bronchus, skin and colon, and human normal tissue array slides (MaxArray, Zymed Laboratories Inc., CA) containing 30 different tissues were used for immunohistochemistry. For pre-treatment, slides were deparaffinized by xylene-treatment followed by decreasing alcohol series. The slides were heated in microwave oven in 10 mM citrate buffer, pH 6.0 for 5 minutes. Immunohistochemical analyses were performed using the ABC method (Vectastain Elite ABC kit, Vector Laboratories, Burlingame, California). Omission of primary antibody and staining with the nonimmunized sera was used as negative control for parallel sections. Neither of these controls showed any immunoreactivity.

### 8. Transient transfections and Elisa

Cos-1 cells were cultured in Dulbecco's modified Eagle's medium supplemented with 10% fetal calf serum, 1% PS and 5% non-essential amino acids. Cos-1 cells were transiently transfected with pCMV-myc- GPRA A, B, C, D, E, F or Bshort separately using Fugene6 transfection reagent (Roche) following the manufacturer's protocol. After 24 hours, the transfected cells from one well of 6 well plate were divided into 16 wells of 96 well plate. After 48 hours of transfection, the cells were fixed with 3.5 % PFA. Half of the cells were permeabilized with 0.5% TX-100 for 10-15 min, blocked with TBS containing 2% milk and 1% goat normal serum at 37 °C for 30 min, incubated with 1:1000 dilution of myc- specific primary antibody (Babco) for 1 h at 37 °C, washed three times with TBS and thereafter incubated with a dilution of 1:2000 of HRP-conjugaded anti-mouse IgG antibody for 30 min at room temperature and washed three times with TBS. TMB-subrate (Sigma) was added to cells and the reaction was let to proceed for 3 to 6 minutes after which the reaction was stopped by adding 1.5 M HCL. Absorbance was measured at 450 nm.

### 9. Computational Protein Sequence Analysis

The transmembrane topology and putative N-glycosylation sites of *GPRA* protein were predicted using PredictProtein (http://cubic.bioc.columbia.edu/ predictprotein/submit def.html#top) and TMpred (http://www.ch.embnet.org/ software/TMPRED form.html) softwares. In protein homology comparisons the BlastP (http://www.ncbi.nlm.nih.gov/blast/) and T-Coffee (http://www.ch.embnet.org/soflware/TCoffee.html) softwares were used.

### 10. Family Collection

Genotyping of *GPRA* was done in a family collection recruited in central eastern Finland. The methods for recruitment, control for population stratification, and clinical evaluation have been described previously in detail (Kauppi *et al.* 1998; Laitinen *et al.* 1997). We used self-reported asthma as a sampling method. Total serum IgE level was determined by Diagnostics CAP FEIA (Kabi Pharmacia, Sweden) in one batch for all the participants. Based on total serum IgE level the study individuals were divided into two groups: high IgE responders (IgE > 100kU/L) and low IgE responders (IgE ≤ 100 kU/L).

Altogether 253 families were recruited, two thirds of which were trios. Based on retrospective verification of the disease history and the results of diagnostic tests (spirometry, histamine or methacholine challenge test, expiratory peak flow measurements), 87% of the self-reported asthma patients were accepted as verified cases (Kauppi *et al.* 1998). Criteria for asthma were based on the recommendations of the American Thoracic Society (Dantzker, D.R. *et al.* 1987). 86 large pedigrees were included into our previous genome scan (Laitinen *et al.* 2001). For the association study, those pedigrees were divided into trios using a in-house computer program. Non-overlapping trios with full phase and phenotype information and additional 103 trios were included to the association analysis without further selection.

### 11. Screening for Exon Polymorphisms

Genomic DNA of four asthma patients and one healthy control from above mentioned study group were screened for sequence variation with 12 primer pairs covering all the verified exons of *GPRA* (Table 5). All the patients were homozygous for microsatellites G42099, G42100, D7S497, and G42097 (from the NT_000380 position 476,213 to 644,425, a total of 168 kb) and expressed the susceptibility haplotype (Patent 1). The control individual showed homozygosity for the same markers, but to a different, non-associating haplotype.

PCR assays were carried out in 20 µl volumes containing 20 ng of genomic DNA, 0.1mM dNTPs (Finnzymes), 2.5 mM MgCl2 (Applied Biosystems), 0.1µM primer mix, and 0.5 U of DNA polymerase, and 1x PCR Buffer II (AmpliTaqGold, Applied Biosystems). The samples were denatured for 10 min at 94 °C, followed by 35 cycles each of 30s at 94 °C, 30 s at 58 °C, and 30 s at 72 °C. Elongation was performed for 10 min at 72 °C. Purified PCR fragments (Quickstep 2 PCR purification Kit, Edge BioSystems) were sequenced from both directions using ABI Prim3100 (Applied Biosystems) sequencer and dye-terminator chemistry. We assembled forward and reverse sequence reads using the Gap4 program (Staden Package software).

### 12. Genotyping

Genotyping of five exonic SNPs in *GPRA* was done using two different methods. One marker was genotyped using single base pair extension (SBE) with the chemistry of Molecular Dynamics on a Megabase 1000 sequencer (Molecular Dynamics) according to the suggestion made by the manufacturer (Table 6A). Allele calling was performed by using the MegaBACE SNP Profiler software (Molecular Dynamics).

Four of the SNPs were genotyped using different restriction enzyme digestions. All primers, restriction enzymes, and lengths of the digestion fragments of corresponding allele used in the genotyping are given in Table 6B. If the SNP did not produce a natural site for altered restriction, mutations were induced in PCR-primers. To improve allele calling by growing size difference between alleles, in one primer plasmid sequence tail was added. Altered restriction sites of the PCR products were visualized on ethidium bromide stained agarose gels in UV light and called manually by two independent observers. All the markers were in Hardy-Weinberg equilibrium and observed Mendel errors were less than 0.1 %.

### 13. Haplotype Association Analysis

The haplotype analysis was done using Haplotype Pattern Mining (HPM) (Toivonen *et al.* 2000) program. The data set provided a total of 132 informative trios, 396 study individuals, and revealing 304 unrelated affected and 220 control chromosomes. Haplotyping was done within each trio and four independent chromosomes were obtained from each trio. In case of ambiguities (missing genotype data, identical heterozygotic genotypes in all of the family members, or Mendel errors), the alleles were discarded. If the child was affected, the transmitted chromosomes were considered disease associated and the non-transmitted chromosomes as controls. If one of the parents was affected, his/her chromosomes were considered disease associated and the spouse's chromosomes as controls. If both the parent and the child were affected, only the non-transmitted chromosome of an unaffected parent was considered as the control and the other three as disease associated. These haplotypes were used as input for HPM.

### RESULTS

### 1. Characterization GPRA at RNA And Protein Level

### A. Genomic Structure of Different Splice Variants of GPRA

The full-length cDNA sequence of *GPRA* was assembled by using RT-PCR with primers designed for predicted exons and by using RACE-PCR to generate the 5' and 3' ends of the gene. The genomic location of the primers is shown in the Fig. 3 and sequence in the Table 4. Nested PCR amplification was used to produce full length cDNAs of different splice variants in one fragment. Using different (Marathon-Ready Brain cDNAs, RNA extracted form NCI-H358 cell line) template three splice variants were identified repeatedly (A, B_{long}, and C variants in the Fig. 3 and 4). All variants possess the same initiation site, but used alternative exons to encode the 3' end of the gene (exons 9A, 9B and 2B, respectively). The sequence flanking the putative ATG translation initiation site (GCCATGC) contains the -3 purine, but not the +4 guanine residue, of the Kozak consensus sequence. In two of the variants, the open reading frame was distributed across 9 exons producing cDNAs of 1116 bp and 1134 bp in size (variants A and B_{long}, Fig. 5A and 5B) and encoding 371 and 377 amino-acid proteins, respectively. The C variant is a shorter transcript of *GPRA* including only the exons E1, E2a and E2b (Fig. 5C). GPRA spanned 0.2 Mb of the genomic contig NT_000380. The second intron is the largest, comprising of 93.8 kb.

When nested PCR specific for the B variant was applied in the cloning process a new splice variant was observed (Bₛₕₒᵣₜ in Figs. 4B and 5B). A 33 bp deletion was observed in the 5' prime end of the exon 3.

### B. Expression Profiling of GPRA Using RT-PCR and Northern Blot Hybridization

RT-PCR analysis showed that different variants of *GPRA* were expressed in several human tissues. These tissues include testis, brain, pituitary gland, placenta, lung, heart, fetal thymus, and fetal heart. In addition to commercially available cDNAs, we analyzed the *GPRA* expression profile in NCI-H358 cell line that represents broncho-epithelial origin. Variants A (JEG5F1 and Vau8R2), B (JEG5F1 and Vaul000R1) and C (JEG5F1 and JEGEXR1) specific primer pairs were used in amplifications (Fig. 8, Table 4). All forms were expressed and using A variant specific primers, we found several transcripts. PCR fragments were cloned and sequenced. Sequence verification revealed variants A, D, E, and F (Figs. 4 and 5). Northern blot hybridization was done using a 470 bp cDNA probe comprising exons E1, E2A, and E2B (Fig. 3). On Human 8-Lane MTN blot, the probe detected multiple transcripts sized approximately 6.5 kb, 6.0 kb, 1.8 kb, and 1.0 kb as shown for placental tissue in Fig. 9.

### C. The Predicted Structure of the GPRA Protein

Both TMpred and PredictProtein softwares predicted that the variants A and B_{long} encode a 7TM protein with an extracellular N-terminus (approximately 50 amino acids) and intracellular C-terminus (Figs. 6A-6F). Their structure shared the common features of GPR family A with 16 conserved amino acids in the TM, loop, and C-terminal regions. These include among others two conserved Cys residues in exoloops 1 and 2 that potentially form a disulfide bridge; the Asp-Arg-Tyr sequence (DRY motif) in the proximity of TM3; Asn-ProX-X-Tyr motif in TM7; and a Cys residue in the C-terminal region (Figs. 6A-6F). The putative N-glycosylation site Asn⁴ was predicted by PredictProtein. All the other variants lacked the 7TM structure. For variant Bₛₕₒᵣₜ a 6TM structure was predicted (Fig. 6B). Variant C encodes 94 amino acid protein that has potentially only one TM region (Fig. 6C). For the variants D and E, deletions of the exons 3 or 4 caused a shift in the ORF, presumably producing truncated forms of the protein. When both exons were deleted (variant F), the ORF remained intact, but only five-transmembrane regions were predicted (Fig. 6F).

### D. Sequence Searches

Determined by BlastP comparisons, the GPRA protein shows 31 % amino acid identity to human vasopressin receptor 1B, 28 % identity to human vasopressin receptor 2, 32 % identity to human oxytocin receptor, and 43 % amino acid sequence identity to gene product CG6111 of *Drosophila melanogaster* which is considered as an orthology to human vasopressin/oxytocin receptor family (Park *et al.* 2002). The best sequence identity between proteins was found in TM regions and in the first exoloop. Based on T Coffee homology comparisons, *GPRA* showed 20% amino acid identity to the bovine rhodopsin receptor. Sequence comparisons between *GPRA* and mouse ESTs BB638128, BB632343, BB625809, BB228269, and publicly available mouse genomic sequence suggest the existence of a mouse orthology.

### 2. Expression Profiling of GPRA Variants A and B Using Western Blot Analysis and Immunohistochemistry

Immunostaining of normal adult human tissue samples with GPRA A variant specific antibody showed strongest expression in smooth muscle cells (SMC), such as SMC layer in bronchial and arterial walls in human lung and colon shown in Fig. 10A and E. In alveolar wall and alveolar macrophages of lung (Fig. 10C) intense staining of *GPRA* A was detected. In colon epithelium (Fig. 10D), strong basal staining was observed, whereas in the bronchial epithelium (Fig. 10A) and keratinocytes (Fig. 10F), only mild/weak immunostaining was detected.

Western blot analysis with GPRA A variant specific antibody revealed four intensive bands corresponding to molecular weights of approximately 50,44,42 and 40 kDa (Fig. 12A). In skeletal muscle only 50 kDa band was detectable whereas uterine muscle, colon epithelium and prostate showed similar expression patterns at 50, 44, and 40 kDa. The most intensive GPRA A expression was recorded in colon muscle with the major band at 42 kDa. Weak or nonexistent bands were found in spleen, kidney, and testis.

Immunostaining with GPRA B variant specific antibody revealed most intense expression of the protein in the epithelium of several mucosal tissues including bronchus, small intestine, and colon as shown in Figs. 11A, D, E. Contrary to the A variant, the expression of the B variant was strongest in the apical surface of the epithelium (Figs. 11A, D, E, and F). Alveolar walls and alveolar macrophages (Fig. 11C) showed strong and SMCs in several tissues mild immunostaining of the variant B.

With GPRA B variant specific antibody, two sharp polypeptide bands corresponding to molecular weights of approximately 39 and 25 kDa were revealed by Western blot analysis with the strongest expression in kidney (Fig. 12B). The specificity of the antibody was confirmed by the blocking experiment with 10x molar excess of the peptide used in immunization.

Cos-1 cells were transiently transfected with pCMV-myc GPRA A, B, Bshort, C, D, E, or F in order to study translocation of different GPRA variants *in vitro.* After 48 hours, cells were fixed and studied by cell based ELISA assay using myc antibody. 71% of recombinant GPRA A and 52% of recombinant GPRA B were translocated to the plasma membrane while all the other variants were located in cytoplasm (Table 15).

### 3 Characterization Of GPRA as a Genetic Regulator of Asthma Related Traits

### A. Detection of GPRA Polymorphisms Associated to Asthma Related Traits

To detect sequence variations in *GPRA* associated to asthma related traits, we sequenced its verified exons and exon-intron boundaries in four patients homozygous for the haplotype significantly associated with high serum total IgE level among the Finnish asthma families. Comparison between patients and one control individual from the same data set were made to the reference sequence (genomic contig NT_000380) in the public data base.

A total of 14 SNPs were found distributed in seven exons of *GPRA* (Table 7). Four of the SNPs were predicted to cause a non-conservative amino acid change: Asn>Ile located in the first extracellular loop, Ser>Arg in the third cytoloop, Gln>Arg and Thr>Ile in the C termini, one in each alternative splice variant. Six of the SNPs were located in the 3' UTRs of the A, B, and C variants of the gene.

### B. Association Analysis

We have previously defined a susceptibility haplotype that significantly associates with asthma related phenotypes (best marker-wise P value based on 10,000 permutations 0.001) (Fig. 3, gray area). The region covers the genomic region between *GPRA* exons 2b and 5. We replicated the association analysis with five SNPs genotyped in the exons of *GPRA* in the same data set (304 disease associated and 220 control chromosomes). Best haplotype associations of *GPRA* for high serum IgE level when one gap was allowed in the haplotype patterns are shown in Table 8. The best observed associations reached χ² values of 5.7-10.3. Only one of markers (SNP591694) was part of the previously determined susceptibility haplotype and the same amino acid change, 107Ile, showed also significant association to high IgE level together with other *GPRA* SNPs.

To study whether the overall haplotype distribution in *GPRA* differs in high IgE associated compared to that in control chromosomes, we used a permutation test. HPM analysis was done using the following parameters: maximum pattern length 5 markers, one gap allowed for missing data and possible errors, and chi-square threshold for the association >5.0. The observed scores (=number of qualified haplotype patterns spanning across the marker) for associated haplotypes varied from 6 to 14. Permutation test showed statistically biased haplotype distribution for high IgE. Based on 10,000 simulations, the best marker-wise P value ≤0.01 was observed for a silent SNP (in position 640,764) in exon 5. P ≤0.02 was observed for three markers in the middle of the haplotype and P<0.04 for all the markers in the haplotype.

### Example VII

This example describes a second gene occurring partially within the AST-1 locus and overlapping the GPRA gene.

### A. Materials and Methods

### 1. Exon Prediction

Exon predictions were performed by GENSCAN (http://genes.mit.edu/GENSCAN.html) software using genomic clone NT_000380 (Polvi *et al.* 2002). Predictions were performed for both unmasked and masked sequence. In the latter, the repetitive sequences were masked using the Repeat Masker Service (http://repeatmasker.genome.washington.edu/).

### 2. Reverse Transcriptase PCR

The exon-specific primers were designed based on exon predictions (Table9, Figure13). First strand cDNAs were synthesized from 1 µg of commercially available poly A⁺ RNAs from human lung, testis, kidney and fetal liver (BD Clontech) by the SMART RACE cDNA Amplification Kit (BD Clontech, Palo Alto, CA) according to manufacturer's instructions. Alternatively, Marathon-Ready cDNA (Clontech) or cDNA from Multiple Tissue cDNA Panels I and II (Clontech) were used as templates in some PCR amplifications.

All the PCR amplifications were performed in 25-50 µl volumes using 2.0-5.0 µl cDNA as template, 2 mM MgCl₂, 0.2 mM dNTPs (Finnzymes), 0.2 mM of each primer, 0.15-0.4 U AmpliTaqGold, and 1x PCR Gold buffer (Applied Biosciences) under the following conditions: 94°C for 5 min, 35-40 cycles of 94 °C for 30 s, 58 °C -62 °C for 40 s, 72 °C for 1 min 30 s followed by 72 °C for 10 min. PCR products were analyzed on 1 % agarose gel and extracted from the gel using QIAquick Gel Extraction Kit (Qiagen). Purified PCR products were analyzed by automated sequencing with dye-terminator chemistry (Megabase 1000 sequencer, Molecular Dynamics).

### 3. Cell Culture and Isolation of Poly A⁺ RNA

In addition to commercially available poly A⁺ RNAs, RNA from Ebstein Barr virus infected lymphoblast cell lines of the patients who were homozygous, heterozygous, or non-carriers of AST1 were cultured for expression studies. Lymphoblasts were cultured in RPMI 1640 medium (Gibco BRL) supplemented with 1 mM sodium pyruvate (Gibco BRL), 10% FCS (Biological Industries), and 1% Penicillin/Streptomycin (GibcoBRL). Poly A⁺ RNA was isolated by Dynabeads mRNA DIRECT Kit (Dynal) according to the manufacturer's instructions.

### 4. Northern Blot Hybridization

The AAA1 specific probe (mixture of the variants I, III, IV, VII, and X) was generated by RT-PCR using human lung, testis, kidney and fetal liver poly A⁺ RNAs as templates. The probe was radiolabelled with α[³²P]-dCTP using RediPrime Kit (Amersham Biosciences) according to the manufacturer's instructions. A Human Multiple Tissue 12-lane Northern blot, Fetal Multiple Tissue Northern blot, and Human Multiple Tissue Expression Array 2 (BD Clontech) were prehybridized in ExpressHyb solution (BD Clontech) for 1 h at 68 °C followed by hybridization with the specific probe for 2-5 h at 68 °C. Herring sperm DNA (100 µg/ml) was used as the blocking reagent. Filters were washed with 2 x SSC and 0.05 % SDS at room temperature and exposed to X-ray film at -20°C for 1 - 5 days.

### 5. Genotyping

Genotyping of four SNPs that are located either in the coding region or near the exon-intron boundaries of AAA1 was done 1) using altered restriction sites (SNP_538567 or SNP_574953, Table 1B) or 2) single base pair extension (SBE) with the chemistry of Molecular Dynamics on a Megabase 1000 sequencer (Molecular Dynamics) according to the suggestion made by the manufacturer (Table 10). Allele calling was performed by using the MegaBACE SNP Profiler software (Molecular Dynamics.

All the markers were in Hardy-Weinberg equilibrium and observed Mendel errors were less than 0.1%.

### 6. In vitro translation of the AAA1 and characterization of AAA1 antibody

In order to investigate whether AAA1 is translated to any polypeptide, *in vitro* translation experiments were performed (Figure 21). Capped RNAs of AAA1 gene variants defined by SEQ ID NOS: 16 and 22 were transcribed from DNA constructs with the aid of T7 RNA polymerases (mMESSAGE mMASCHINE system, Ambion, USA). Translation was performed with rabbit reticulocyte lysate translation machinery (Riboprobe in vitro translation system, Promega) in the presence of S³⁵ -labelled methionine in the reaction mixture. The Xenopus elongation factor α (pTRI-Xef) DNA template was used as a positive control for transcription and translation. In negative control, water was used instead of DNA. The translated polypeptides were detected by autoradiography after Tris-Tricine SDS-PAGE.

### B. Results

### 1. Characterization of AAA1 at RNA Level

### A. Genomic Structure of Different Splice Variants of AAA1

The human AAA1 gene spans 520 kb of the genomic contig NT_000380 (nucleotides 163615-684776) and it is divided into 18 exons (Figure 13 and Table 11). The cDNA sequences of AAA1 splice variants were assembled by using RT-PCR with primers designed for predicted exons. The genomic location of the primers is shown in the Figure 13 and the sequence in the Table 9. Using cDNAs from human lung, kidney, testis and fetal liver as templates, twelve splice variants were identified repeatedly (variants I-XII in the Figure 14). All variants share exon 6, but use alternative exons to encode the 5' and 3' ends of the transcript. In six variants out of ten, the sequence flanking the putative ATG translation initiation site (GCCATGC) contains the -3 purine, but not the +4 guanine residue, of the Kozak consensus sequence.

### B. Expression Profiling of AAA1 Using RT-PCR and Northern Blot Hybridization

Northern blot analysis of a multiple tissue expression array showed that AAA1 is expressed in several human tissues (Figure 16). Strong expression was seen in testis, brain, placenta, lung, heart, skeletal muscle, kidney, liver, fetal liver, and fetal lung. In multiple tissue northern blots, two main transcripts (2.4 and 7.5 kb in size) were detected (Figure 17). Additionally, all sample lanes showed extensive label streaming which can be an indication of several low-abundant transcripts with different sizes.

By RT-PCR exceptionally strong splicing and tissue specific differences in AAA1 expression could be found (Figure18). For example, expression of splice variants I and IV was particularly strong in the lungs consistent with the association between AAA1 and asthma.

In the *in vitro* translation experiment, the Xenopus elongation factor α (pTRI-Xef) DNA template used as a positive control resulted in the synthesis of a 50-kDa polypeptide as expected. However, neither of the two investigated constructs was translated into polypeptides strongly arguing that AAA1 functions as a non-coding RNA gene (Figure 21).

To further investigate the translation of the AAA1 gene, a polyclonal antibody against the constant region (YVRRNAGRQFSHC) of the gene product was produced in rabbits. AAA1 peptide synthesis and antibody production were purchased from Sigma-Genosys Ltd (London Road, Pampisford, Cambridge). To test the specificity of the antibodies, Glutathione S-transferase (GST) -fusion proteins for AAA1 were produced with the pGEX 4T-3 GST fusion expression vector (Amersham Biosciences) according to the manufacturer's instructions. (Figure 21). The antibody displays high affinity against the the recombinant AAA1 protein produced in bacterial lysate with no cross-reactivity between the GST construct alone. In spite of that, the antibody did not reveal any reactivity either in Western blots (spleen, skeletal muscle, uterine muscle, colon muscle, colon epithelium, kidney, testis and prostate) or in immunohistochemistry (bronchial tissue, HepG2 cell line) (data not shown).

In addition, formalin fixed, paraffin-embedded specimens of normal adult human bronchus tissue and HepG2-cells (positive for AAA1 RT-PCR) were used for immunohistochemistry. Immunohistochemical analyses were performed using rabbit AAA1 antibody and the ABC method (Vectastain Elite ABC kit, Vector Laboratories, Burlingame, California).Finally, AAA1 does not appear to have a mouse counterpart. Concluding from our data, AAA1 may be a non-coding RNA gene and is unlikely to encode a functional protein.

### C. The Predicted Structure of the AAA1 Peptides

The cDNA sequences of twelve AAA1 splice variants are shown in Fig. 20. All predicted AAA1 proteins are small peptides (size from 34 aa to 74 aa) that do not show significant identity to any known modular structures or motifs. All isoforms contain the same core sequence (AYVRRNAGRQFSHCNLHAHQFLVRRKQ) flanked by alternative amino- and carboxyterminal tails (Figure 15).

### 2. Characterization Of AAA1 as a Genetic Regulator of Asthma Related Traits

### A. Association Analysis

AST1 covered the exons 3 to 10 of AAA1 (Figure 13). For the association analysis we chose one exonic SNP (SNP 517278) and four additional SNPs near exon-intron boundaries of AAA1 (Table 12). All the polymorphisms were identified previously (Table 3). The analysis for high total serum IgE level was done in the same data set as previously (304 disease associated and 220 control chromosomes, Table 2 and 8). Best haplotype associations of AAA1 are shown in Table 12. The best observe associations reached the χ² values of 8.9-13.6 and the permutation test showed statistically biased haplotype distribution for high IgE. Based on 10,000 simulations, the best marker-wise P value ≤0.0001 was observed for SNP_517278 in exon 10. Corrected P value for testing multiple markers simultaneously was 0.0002.

### B. Variable alternative splicing for AAA1 depending on genotype

To study further whether AST1 effects on the expression of AAA1 we studied lymphoblast cell lines from the asthma patients who were homozygous, heterozygous, or non-carriers of AST1. Using RT-PCR with the primer pair SCF10 and ASKAR (Table 9) significant differences were found between patients and the differences were dependent on genotype (Figure 19). Only the non-carrier of AST1 processes normal amount of the exon 6-10b transcript, whereas the homozygote and heterozygotes show either an absent transcript or smaller splice variants. Differences in expression patterns suggest that AST1 can effect splicing of the gene and thereby, increase the risk of asthma related diseases among AST1 carriers compared to that among AST1 non-carriers.

### REFERENCES

Dantzker, D.R. et al. 1987. Standards for the diagnosis and care of patients with chronic obstructive pulmonary disease (COPD) and asthma. Am. Rev. Respir. Dis. 136: 225-243.
Becker, K.G. et al. 1998. Clustering of non-major histocompatibility complex susceptibility candidate loci in human autoimmune diseases. Proc. Natl. Acad Sci. U.S.A. 95: 9979-84.
Daniels, S.E. et al. 1996. A genome-wide search for quantitative trait loci underlying asthma. Nature 383: 247-50.
Dizier, M.H. et al. 2000. Genome screen for asthma and related phenotypes in the French EGEA study. Am. J. Respir. Crit. Care Med. 162: 1812-8.
Jacob, H.J. et al. 1992. Genetic dissection of autoimmune type I diabetes in the BB rat. Nat. Genet. 2: 56-60.
Laitinen, T., et al. 2001. A susceptibility locus for asthma-related traits on chromosome 7 revealed by genome-wide scan in a founder population. Nat. Genet. 28: 87-91.
Leaves, N.I. et al. 2002. A detailed genetic map of the chromosome 7 bronchial hyper-responsiveness locus. Eur. J. Hum. Genet. 10:177-82.
Malerba, G. et al. 2000. Linkage studies to asthma and atopy phenotypes on chromosomes 7, 12, and 19 in the Italian population. Am. J. Hum. Genet. 67: 330.
Mathias, R.A. et al. 2001. Genome-wide linkage analyses of total serum IgE using variance components analysis in asthmatic families. Genet. Epidemiol. 20: 340-55.
Ober, C. et al. 2000. A second-generation genomewide screen for asthma-susceptibility alleles in a founder population. Am. J. Hum. Genet. 67: 1154-62.
Polvi A et al. 2002. Physical map of an asthma susceptibility locus in 7p15-p14 and an association study of TCRG. Eur. J. Hum. Genet. 10, 658-665 (2002).
Remmers, E.F. et al. 1996. A genome scan localizes five non-MHC loci controlling collagen-induced arthritis in rats. Nat. Genet. 14: 82-5.
Satsangi, J. et al. 1996. Two stage genome-wide search in inflammatory bowel disease provides evidence for susceptibility loci on chromosomes 3, 7 and 12. Nat. Genet. 14: 199-202.
Sawcer, S., et al. 1996. A genome screen in multiple sclerosis reveals susceptibility loci on chromosome 6p21 and 17q22. Nat. Genet. 13: 464-8.
Sevon, P. et al. 2001a. TreeDT: Gene mapping by tree disequilibrium test. In KDD-2001. Proceedings of the Seventh ACM SIGKDD International Conference on Knowledge Discovery and Data Mining, pp 365-370. Editors: Provost, F. and Srikant, R. Publisher ACM press, San Francisco, CA USA (www.acm.org/sigkdd/kdd2001)
Sevon, P. et al. 2001b. TreeDT: Gene mapping by tree disequilibrium test (extended version). In the publication series Report C-2001, Nr 32:6-7 published by Department of Computer Science, University of Helsinki
Toivonen, H.T. et al. 2000. Data mining applied to linkage disequilibrium mapping. Am. J. Hum. Genet. 67: 133-45.
Wjst, M., et al. 1999. A genome-wide search for linkage to asthma. German Asthma Genetics Group. Genomics 58: 1-8.
Xu, J. et al. 2001. Genomewide Screen and Identification of Gene-Gene Interactions for Asthma-Susceptibility Loci in Three U.S. Populations: Collaborative Study on the Genetics of Asthma. Am. J. Hum. Genet. 68: 1437-1446.
Xu J et al (2000). Major genes regulating total serum immunoglobulin E levels in families with asthma. Am J Hum Genet. 67:1163-73.
Yokouchi, Y. et al. 2000. Significant evidence for linkage of mite-sensitive childhood asthma to chromosome Sq31-q33 near the interleukin 12 B locus by a genome-wide search in Japanese families. Genomics 66: 152-60.
Daly, M.J. et al. 2001.High-resolution haplotype structure in the human genome. Nat Genet. 29: 229-32.
Dantzker, D.R. et al. 1987. Standards for the diagnosis and care of patients with chronic obstructive pulmonary disease (COPD) and asthma. Arn. Rev. Respir. Dis. 136: 225-243.
Johnson, E.N. et al. 2002. Heterotrimeric G protein signaling: role in asthma and allergic inflammation. J Allergy Clin Immunol. 109: 592-602.
Kauppi, P. et al. 1998. Verification of self-reported asthma and allergy in subjects and in their family members volunteering for gene mapping studies Resp. Med. 92: 1281-1288.
Laitinen, T. et al. 1997. Genetic control of serum IgE levels and asthma: Linkage and linkage disequilibrium studies in an isolated population. Hum. Molec. Genetics 6: 2069-2076.
Laitinen, T. et al. 2001. A susceptibility locus for asthma-related traits on chromosome 7 revealed by genome-wide scan in a founder population. Nat. Genet. 28: 87-91.
Michel U 2002. Non-coding ribonucleic acids--a class of their own? Int. Rev. Cytol. 218: 143-219.
Numata et al. 2003. Identification of Putative Noncoding RNAs Among the RIKEN Mouse Full-Length cDNA Collection. Genome Res. 13: 1301-06.
Palczewski, K. et al. 2000. Crystal structure of rhodopsin: A G protein-coupled receptor. Science 289: 739-45.
Park, Y. et al. 2002. Identification of G protein-coupled receptors for Drosophila PRXamide peptides, CCAP, corazonin, and AKH supports a theory of ligand-receptor coevolution. PNAS 88: 11423-28.
Rana, B.K. et al. 2001. Genetic variations and polymorphisms of G protein-coupled receptors: functional and therapeutic implications. Annu. Rev. Pharmacol. Toxicol. 41: 593-624.

The above examples are provided to illustrate the invention, but not to limit its scope; other variants of the invention will be readily apparent to those of ordinary skill in the and are encompassed by the claims of the invention.

**Table 1. Primers that were used in SNP genotyping with SBE method (A); primers, restriction enzymes, and the length of the digested PCR product of corresponding alleles used in SNP genotyping with altered digestion (B); primer pairs and the length of the corresponding PCR product used in genotyping of the small insertions and deletions (C).**

**Table 1A.**

| **SNP ID** | **Nucleotide NT_000380.3** | **Type¹** | **Fw primer** | **Rev primer** | **PE PRIMER** |
|---|---|---|---|---|---|
| dbSNP: 323926 | 506401 | C/T | CCCCCTGCTCTCTCTCTCTT | GGGATTTATCCATTTCCTCCA | ATGCCAGCATCATACTTCCTG |
| dbSNP: 323906 | 509240 | C/G | GATCTTCCATTTCACCAAGCA | GATGGATATTTAGCTCCCCCTTA | CACACTAGCAAAGTCAGGCAAT |
| dbSNP: 323917 | 515224 | C/G | TGAGTTGATGAATCTGCTGGA | GGCAATGCCAATTAAGAGGA | CACGAGCCCTGGATTACCTAC² |
| dbSNP: 143266 | 515632 | A/G | CAATGGATCACTGCAGCCTA | TTCCCACCCATTAGTTTTTCC | AGAACAAGCTATGCCAGAGACTG² |
| dbSNP: 324377 | 529556 | A/C | AACACAGCCCTCCAGACACT | ATACCTGGCCAGCGGTTTA | TGGAAAAATTTGAACATTATTGGG |
| dbSNP: 324373 | 531632 | A/G | CTATTGGGCCCTGGAGACTT | ATGCAGCTTGCATTGTTCAC | GGTCTCTGGGACCCCTTC² |
| dbSNP: 182718 | 543580 | C/T | AGGGATCATGTCTCCAGCAC | TTAAATGCCCACTAGCACCA | GAGCAGAGATCAGAAACAGGCT² |
| dbSNP: 324384 | 555608 | A/G | TGTGTCTCCTTCTTTGTGTTCATA | TGAAAGCAGGGCTACCATTT | GCAGGAACAGAAAACGAAATACC² |
| dbSNP: 324396 | 563704 | C/T | CACAGGGAGGAAAGGAACAG | GATGGGATTGAGGAGCTTGA | ACAGTGGATGGTTTCTTGGC |
| dbSNP: 324981 | 591694 | A/T | GGCCATCTGATAAAGCAGGA | TGATGCATAGGAATGCAAGG | AGTCTCCAGTGAATCGCCAA2 |
| dbSNP: 325456 | 617392 | C/T | GATGAAAAGGGAACCATTCCT | TGCTTTTCCTGTGTCCATTG | ATGCCCTAATTCTAAAACCAATGA |
| dbSNP: 62911 | 638799 | C/T | TGACATGTTCCCTGCATTTG | AGAGGGCTTTGTGCTCTCTG | CACCCTCCCTGGTGACCT² |

| | | | | | |
|---|---|---|---|---|---|
| ¹ Polymorphism detected in assays employing primers specified in this table ² extension primers (Pe) designed in reverse orientation | | | | | |

| **Table 1B.** Primer, restriction enzymes, and sizes of the corresponding alleles after digestion used In SNP genotyping of AST1. | | | | | | | | |
|---|---|---|---|---|---|---|---|---|
| | | | | | | | | |
| | | | | | | | | |

| **Marker** | **Forward primer** | **Reverse primer** | **Enzyme** | **Product size*** | **Allele 1** | **Product size*** | **Allele 2** | **Note** |
|---|---|---|---|---|---|---|---|---|
| SNP_490331 | gcctgccctagagacatcag | tggctgccttcttcatctttg | HpyCH4V | 180+110 | c | 180+90+20 | a | special combinations of fragments when heterozygous for the SNPs 490331 or 490390 |
| SNP_490390 | gcctgccctagagacatcag | tggctgccttcttcatctttg | HpyCH4V | 180+110 | a | 180+80+30 | g | |
| SNP_490820 | ccaattctcatccaaaaggc | | Dralll | 330 | t | 250+80 | g | plasmid tail with the Dralil restriction site |
| SNP_513659 | | tggatttgcatttcgtctaatg | Mnll | 220 | g | 170+50 | c | plasmid tail |
| SNP_514743 | ggaaactggaagtcaacacca | tctccctcagaaaagccaga | Sspl | 420 | g | 210+210 | a | |
| SNP_516094 | cctgcaataaacattctcataattaaa | ggtgggattacaggcatgag | Faul | 400 | t | 210+190 | c | |
| SNP_516174 | gaagtggacttactgcatcaaaga | ggggtttcaccgtgttagc | Sfcl | 400 | t | 200+200 | g | |
| SNP_516267 | gctctagaacccgctagcat | gcctcagcctctggagtagc | BsrBl | 380 | a | 180+200 | g | |
| SNP_520598 | gcaaaagttgagggggattc | accatgcctcgccatagtaa | Nlalll | 300 | c | 130+170 | t | |
| SNP_521640 | aggaacccttcccagcag | cccaaagccctgagaaaaat | Blpl | 360 | a | 180+180 | g | |
| SNP_522363 | tgcaccagctttttgaggta | tgtaacatcccccagggact | EcoNl | 380 | g | 220+160 | a | |
| SNP_526991 | ctttagtgggaaggctgtgg | aagccactaatatttgggatgg | Bsawl | 400 | c | 230+170 | a | |
| SNP_528709 | tgctctttttgacagccaatc | ggctgctttgatctgaggac | Ncol | 360 | t | 180+180 | g | |
| SNP_529142 | ccataagcctcgcctatttt | gtgggcactgaggcatagat | Taql | 410 | a | 240+170 | g | |
| SNP_529820 | caaatcctcgttctcctgct | tacccagaggctgagaaagg | BsaAl | 400 | t | 220+180 | c | |
| SNP_530177 | cctttctcagcctctgggta | gtgagctgagttcgcgctat | Styl | 380 | t | 230+150 | c | |
| SNP_538567 | ctcagaacactgctgccaga | gctctgggatgcgttaactt | EcoNl | 380 | g | 220+160 | a | |
| SNP_541906 | ttgatctggccaaatggaac | ctaaacaaaaaggggctgcaa | Aflll | 350 | c | 180+170 | a | |
| SNP_546333 | gcatgtgaatacagcacttgg | ctcccgagttcaagcaattc | Haelll | 290+90 | a | 190+100+90 | g | |
| SNP_563585 | ccctacaagttaccctgcaca | cagaggtttggatgggattg | Hindlll | 375 | c | 190+185 | t | |
| SNP_563930 | tcaagctcctcaatcccatc | gtggaggaagtgccttttca | Stul | 330+40 | g | 150+180+40 | a | |
| SNP_564782 | aaaggcagccagtgtctgtt | ggcagagagtcccacagtga | Mspl | 400 | a | 170+230 | g | |
| SNP_574953 | tctcagcctgctacccctta | aagcacaagcatcaggttca | Ahdl | 365 | a | 180+185 | g | |
| SNP_585883 | tcaacagaggcatgattgga | gcagatggaggtagagctgttt | Sfcl | 370 | g | 180+190 | c | |
| SNP_640764 | | tagcacaatgcctgccctat | Sapl | 340 | t | 290+50 | c | plasmid tail |
| SNP_647327 | aggcaagtcacctgctcttc | gctgcagaaagaaccaaagg | BstEll | 455 | c | 210+245 | t | |
| SNP_662764 | tgtctacctgttggcctgtg | gtcttgtgcatctcccaggt | Mboll | 455 | a | 240+215 | g | |
| SNP_662803 | catggagaaggaaggtcagg | ctagcactggcactgcccta | Ddel | 270 | c | 220+50 | t | |
| SNP_663133 | atgactgcatgcactgctta | tctgcaaaccgaggctatct | SfaNl | 400 | t | 140+260 | c | |
| | | | | | | | | |

**Table 2. HPM permutation test done using the following parameters: maximum pattern length 40 markers, one gap allowed for missing data and possible errors, and chi-square threshold for association 13.0. The observed scores (=number of qualified haplotype patterns spanning across the marker) , and marker wise P values based on 10 000 permutations are shown. The critical region (=AST1) is highlighted.**

| **Marker** | **HPM Score** | **Marker wise P value** |
|---|---|---|
| NM4 | 0 | 1 |
| NM5 | 0 | 1 |
| D7S683 | 0 | 1 |
| D7S656_2 | 0 | 1 |
| NM2 | 0 | 1 |
| MIT_MH26 | 0 | 1 |
| NM8 | 0 | 1 |
| NM9 | 0 | 1 |
| NM11 | 0 | 1 |
| G42099 | 0 | 1, |
| SNP_490331 | 0 | 1 |
| SNP490391 | 0 | 1 |
| SNP_490820 | 0 | 1 |
| SNP_506401 | 0 | 1 |
| SNP_509240 | 0 | 1 |
| NM50 | 0 | 1 |
| SNP_513659 | 0 | 1 |
| SNP_514743 | 0 | 1 |
| SNP_515224 | 0 | 1 |
| SNP_515632 | 0 | 1 |
| SNP 516094 | 0 | 1 |
| SNP_516174 | 0 | 1 |
| SNP_516267 | 0 | 1 |
| NM49_516568 | 0 | 1 |
| NM51_517022 | 2 | 0.0139 |
| G42102_518794 | 7 | 0.0105 |
| NM53_519564 | 12 | 0.0091 |
| SNP_520598 | 15 | 0.0083 |
| SNP_521640 | 20 | 0.0079 |
| SNP_522363 | 23 | 0.0075 |
| SNP_526991 | 23 | 0.0076 |
| SNP_528709 | 30 | 0.007 |
| SNP_529142 | 33 | 0.0066 |
| SNP_529556 | 40 | 0.0062 |
| SNP_529820 | 33 | 0.0061 |
| SNP_530177 | 34 | 0.0059 |
| SNP_531632 | 34 | 0.0054 |
| D7S497_538277 | 40 | 0.0052 |
| SNP_538567 | 38 | 0.0052 |
| SNP_541906 | 36 | 0.0054 |
| SNP_543580 | 35 | 0.0054 |
| SNP_546333 | 35 | 0.0054 |
| SNP_555608 | 35 | 0.0051 |
| NM45_560804 | 35 | 0.0049 |
| SNP_563585 | 35 | 0.0049 |
| SNP_563704 | 28 | 0.0046 |
| SNP_563930 | 23 | 0.005 |
| SNP_564782 | 16 | 0.0063 |
| SNP_574953 | 10 | 0.0071 |
| SNP_585883 | 4 | 0.0093 |
| SNP_591694 | 3 | 0.0098 |
| SNP_617392 | 3 | 0.0085 |
| SNP_638799 | 0 | 1 |
| SNP_640764 | 0 | 1 |
| G42097 | 0 | 1 |
| SNP_647327 | 0 | 1 |
| SNP_662764 | 0 | 1 |
| SNP_662803 | 0 | 1 |
| SNP 663133 | 0 | 1 |
| NM13 | 0 | 1 |
| NM46 | 0 | 1 |
| -D7S484 | 0 | 1 |

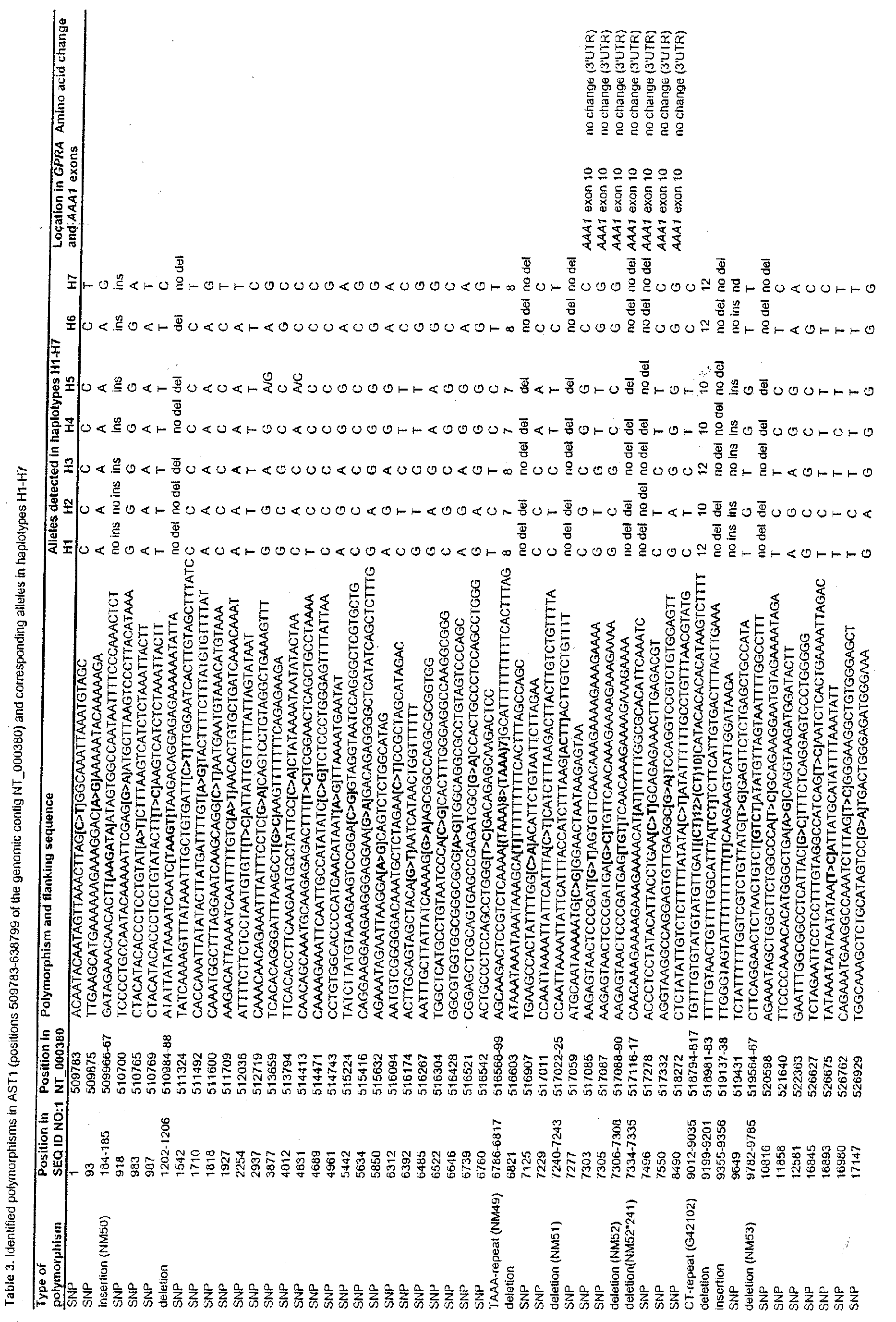

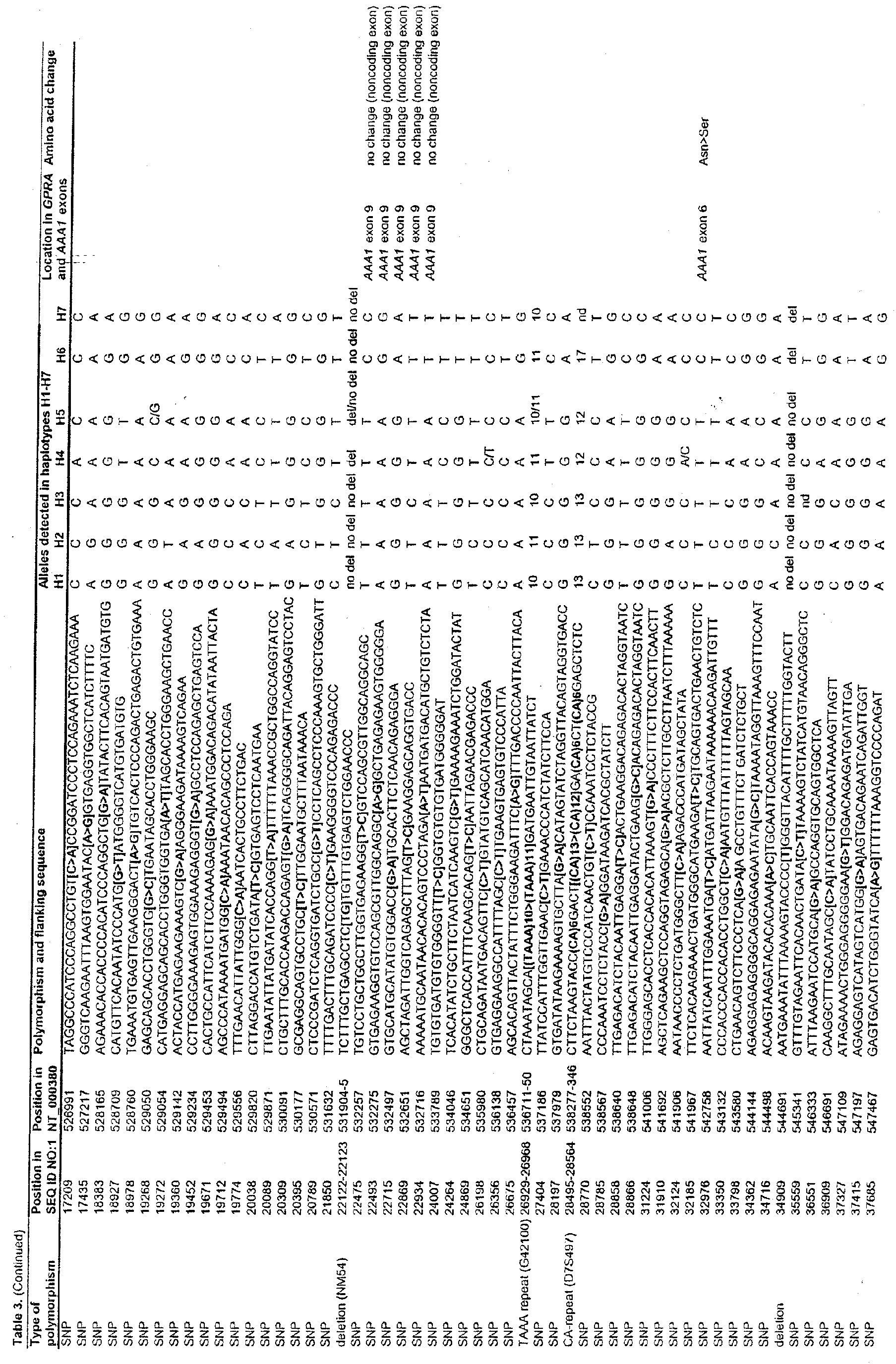

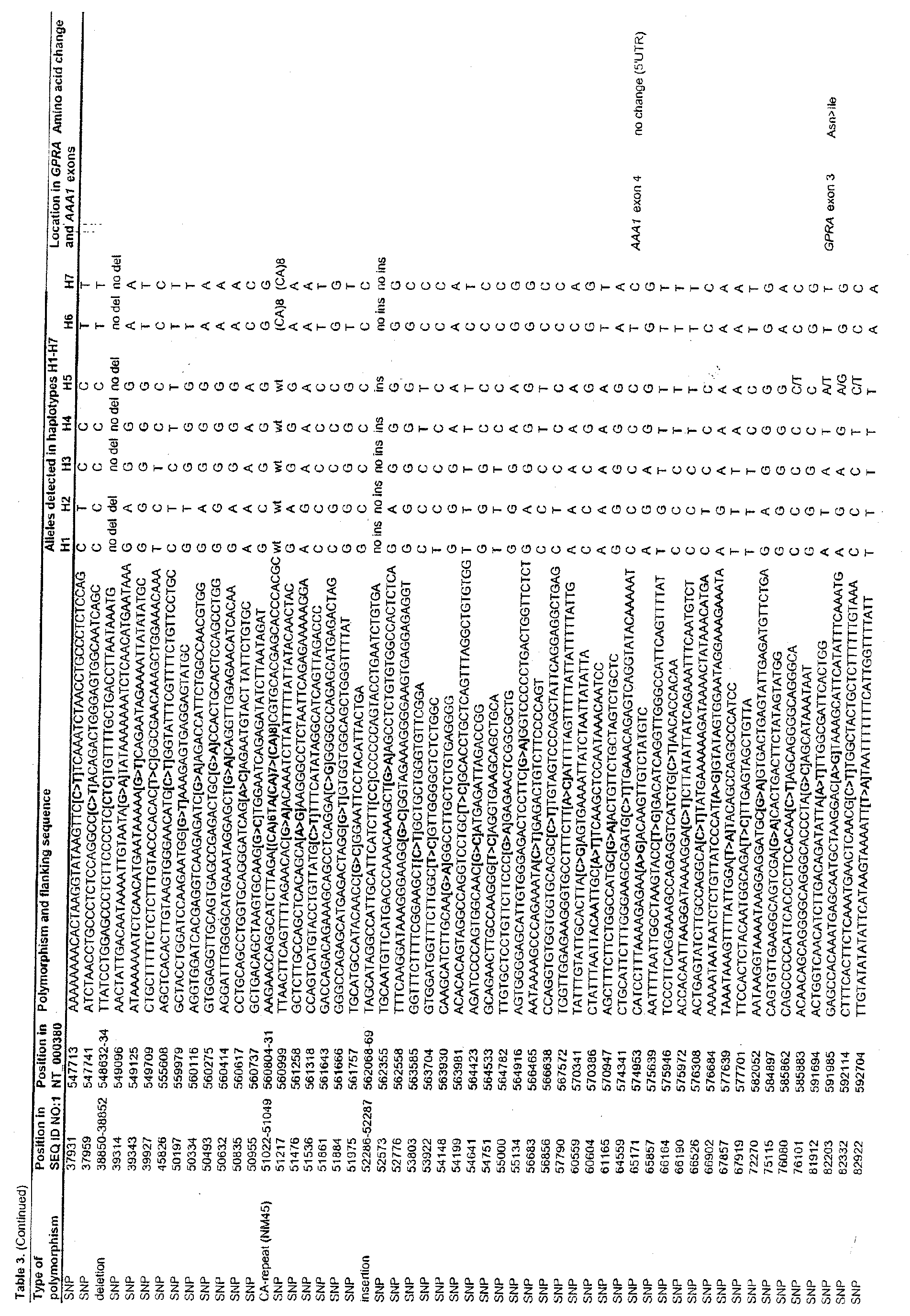

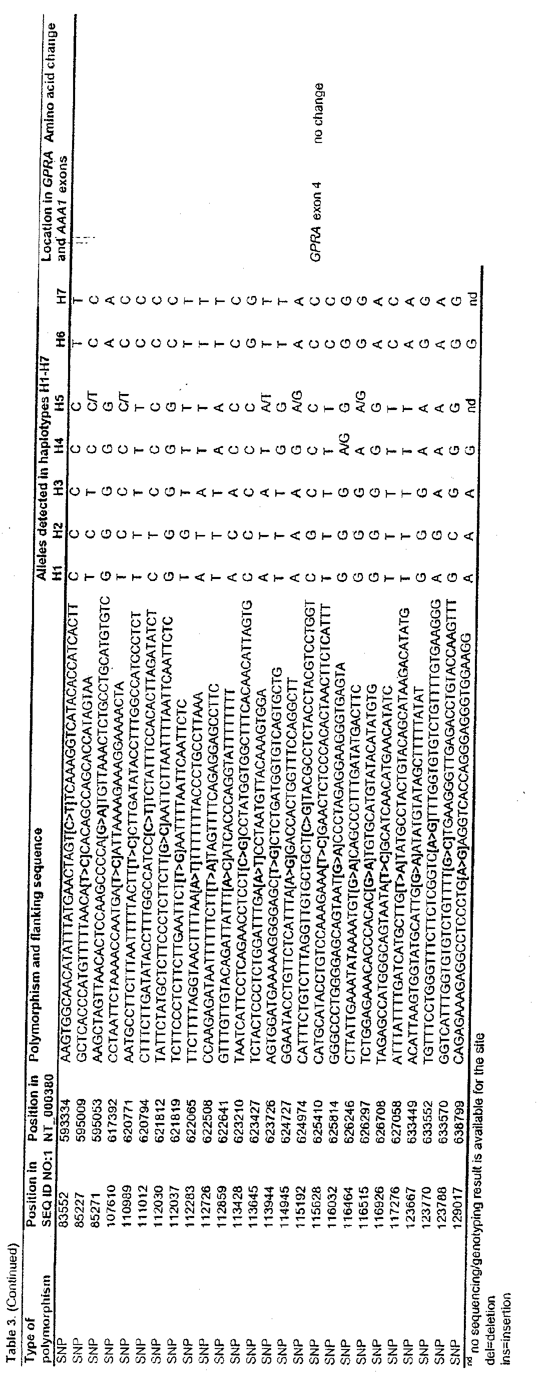

**Table 4. Primers used in the cloning of GPRA.**

| **Primer** | **Sequence** | **Exon** | **Splice variant** |
|---|---|---|---|
| JEGE1F1 | tctgtgcctccgttcagcag | 5' UTR | - |
| JEGE1F2 | aagctggactccctcactcagc | 5' UTR | - |
| JEGE1F3 | agcaaggacagtgaggctcaacc | 5' UTR | - |
| JEGE9aR1 | ctggcatgaataactggggagttc | 3' UTR | A |
| JEGE9aR2 | tttgtcttgtgcatctcccaggta | 3' UTR | A |
| JEGE9bR1 | tatagccctccctggtgaatctga | 3' UTR | B |
| JEGE9bR2 | gtgccctggtaagcagtgagaagt | 3' UTR | B |
| JEGE5F1 | tgagcaattgataactctgtgggtcctc | E2a | - |
| JEGExR2 | AAATAAGCTGTGGCATCCTCATCCAG | 3' UTR | C |
| JEGExR1 | TGGCATCCTCATCCAGGGATATTTGC | 3' UTR | C |
| VAUE3R1 | GGAAGGCCACGATGGTCATGTATGG | E5 | - |
| Vau8R1 | ATGACGAGGGTGGGGTGAAGTTGG | 3' UTR | A |
| Vau8R2 | GAATGGTGGGGAAGGAAGGCGTTT | 3' UTR | A |
| Vau1000R1 | ggccatcctgctgtgacccatttt | 3' UTR | B |
| AS8FX1 | ATGAGATGCAGATTCTGTCCAAG | E9a | A |
| AS8FX2 | TGCACAAGACAAATGTTCTAATGA | 3'UTR | A |
| AS8F5.1 | CAGCTATAACCGAGGACTCATCTC | E7 | - |
| AS8F7.1 | TGTTGGAGTCCATACTTCCTGTT | E8 | - |
| VAUE3R2 | AAAAGACAGGCTCCAGGCGATCACA | E5 | - |
| A2E2F | gattcttccccagtggcttgcactgaa | E1 | - |
| A3E3R | TGATGGCCAGCTGAGTCACAAAGAAGG | E2a | - |

**Table5. Primer pairs used in re-sequecing of the exons and exon/intron bondaries of GPRA**

| **Primer** | **Forward primer** | **Reverse primer** |
|---|---|---|
| *GPRA* ex1 | actcagctgcaggagcaag | tgacactcttaagttccagcagtc |
| *GPRA* ex2a | aggaggaagaaatccagcct | tgaccgatgcgttacatttt |
| *GPRA* ex2b.1 | gccatacattgttagtaacctgaaa | cctcatccagggatatttgc |
| *GPRA* ex2b.2 | ttcctaccaacaagaactccaa | cgatgatgaattagaacatacaacttt |
| *GPRA* ex3 | taagtcaaagaactcctaccttgc | agcaaaggaaatacattaaaoatcaaa |
| *GPRA* ex4 | ctgccctctttcacccagta | agccacccaccttccttagt |
| *GPRA* ex5 | gcttctgttcaagcttcccttt | gtgtggtcctgtcctgacg |
| *GPRA* ex6 | tggatcctcatggtcactttc | tctctgctggcatagcttga |
| *GPRA* ex7 | ttgagagagtctgagcattcca | ccaaattattcaacccatagcc |
| *GPRA* ex8 | tgacatcaatgctccaaacaa | tgtcatgattaaggcggtttc |
| *GPRA* ex9a | ttaacatgtctacttgccttttca | tctgcaaaccgaggctatct |
| *GPRA* ex9b | gcagagctgtcacccaaaat | agcctgggcaacaagagtaa |

**Table 6A. Primers used in GPRA SNP genotyping.**

| **SNP ID** | **Nucleotide NT 000380.3** | **Type** | **Fw primer** | **Rev primer** | **Pe primer** |
|---|---|---|---|---|---|
| dbSNP: 324981 | 591694 | A/T | GGCCATCTGATAAAGCAGGA | TGATGCATAGGAATGCAAGG | AGTCTCCAGTGAATCGCCAA |

| **Table 6B**. Primer pairs, rectrction enzymes, and corresbonding allele sizes used in genotyping of GPRA exonic SNPs. | | | | | | | |
|---|---|---|---|---|---|---|---|
| Six digit number in the name of the SNP shows its position in the genomic contig NT_000380. | | | | | | | |
| | | | | | | | |

| Marker | Forward primer | Reverse primer | Enzyme | Product size* | Allele 1 | Product size* | Allele 2 |
|---|---|---|---|---|---|---|---|
| SNP_640764 | aactgactaaactaggtgccacgtcgtccatacatgaccatcgtgctctt | tagcacaatgcctgccctat | Sapl | 340 | t | 290+50 | c |
| SNP_662764 | tgtctacctgttggcctgtg | gtcttgtgcatctcccaggt | Mboll | 455 | a | 240+215 | g |
| SNP_662803 | catggagaaggaaggtcagg | ctagcactggcactgcccta | Ddel | 270 | c | 220+50 | t |
| SNP_663133 | atgactgcatgcactgctta | tctgcaaaccgaggctatct | SfaNI | 400 | t | 140+260 | c |
| | | | | | | | |
| * product sizes are approximitations | | | | | | | |

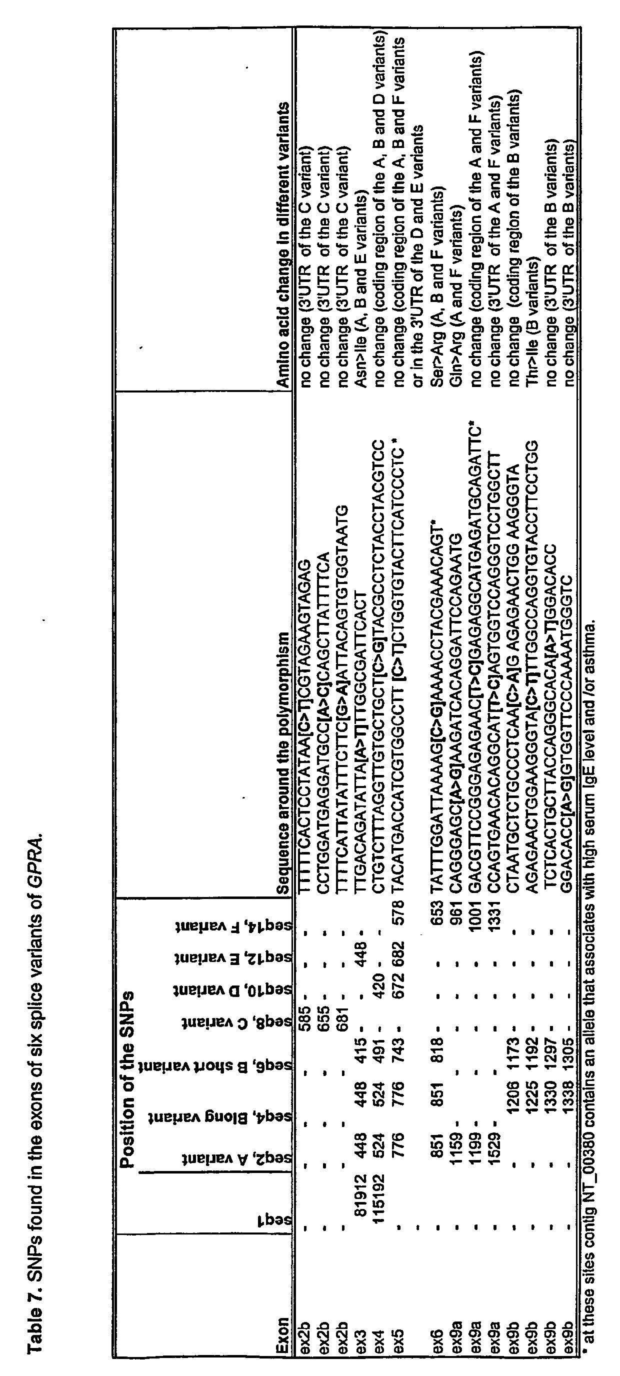

**Table 8. Best haplotype associations of GPRA for high serum IgE level in the data set of 304 disease associated and 220 control chromosomes when one gap (marked with an asterisks) was allowed in the haplotype patterns.**

| High IgE | Control | Conf | χ² | ***G P R A markers*** | | | | |
|---|---|---|---|---|---|---|---|---|
| | | | | SNP591694 Exon 3 | SNP640763 Exon 5 | SNP662763 Exon 9A | SNP662803 Exon 9A | SNP663133 Exon 9A |
| 51 | 16 | 0.761 | 10.3 | - | C | - | - | - |
| 44 | 14 | 0.759 | 8.5 | T | C | - | - | - |
| 40 | 12 | 0.769 | 8.5 | - | C | * | T | - |
| 39 | 12 | 0.765 | 7.9 | - | C | * | T | C |
| 39 | 12 | 0.765 | 7.9 | - | C | G | - | - |
| 39 | 12 | 0.765 | 7.9 | - | C | G | T | - |
| 38 | 12 | 0.76 | 7.3 | - | C | G | * | C |
| 38 | 12 | 0.76 | 7.3 | - | C | G | T | C |
| 35 | 11 | 0.761 | 6.8 | T | C | * | T | - |
| 34 | 11 | 0.756 | 6.2 | T | C | * | T | C |
| 34 | 11 | 0.756 | 6.2 | T | C | G | - | - |
| 34 | 11 | 0.756 | 6.2 | T | C | G | T | - |
| 33 | 11 | 0.75 | 5.7 | T | C | G | * | C |
| 33 | 11 | 0.75 | 5.7 | T | C | G | T | C |

| | | | | | | | | |
|---|---|---|---|---|---|---|---|---|
| High IgE, number of disease-associated haplotypes with a specific pattern; Control, number of control haplotypes with a specific pattern; Confidence, percentage of haplotypes with the specific pattern that is associate with disease, chi-square value for disease association of the specific haplotype pattern. | | | | | | | | |

**Table 9. Primers used for cloning of full length cDNAs for AAA1.**

| **Primer** | **Exon** | **Sequence** |
|---|---|---|
| **IF** | 1 | 5' AGAATGAGTCTCTGATGACTTT 3' |
| **IIF** | 2 | 5' ACTTGCTGTTCATAGAATTGCAA 3' |
| **SF9** | 4 | 5' TGACTTCTCCCCAGATTTTTGTAT 3' |
| **exIF** | 5 | 5' ACACATACAAAGTGCCTACCACAT 3' |
| **SR13** | 9 | 5' TTGAAACTGTATTTCCCATATTGC 3' |
| **ASKAF/R** | 10b | 5'AAATGCAATAAAAATGCGGAACTA 3' |
| **XR** | 11 | 5' GAGTCATTAGTCCAGAGAACAT 3' |
| **XIR** | 12 | 5' CTGCTTGGAACAGTGTATATC 3' |
| **XIIIR** | 14 | 5' TGGTCTACGTAGAATTCAGAGTA 3' |
| **XVR** | 16 | 5' CATGTGTTAATTGTGTCTTCACT 3' |
| **SR4** | 19 | 5' GGGTGTCATTTACACGAACAATAA 3' |
| **SF10** | 6 | 5' CAGTTCAGTCACTGCAATCTTCAT 3' |

**Table10. Primers used in SNP genotyping with SBE method.**

| SNP | forward | reverse | extension |
|---|---|---|---|
| 517278 | TTATGAGCTAAAGTGCCAATTAAA | AGGAAGGTGGCAGTGAACTC | CCCTCCTATACATTACCTGAA |
| 549709 | TCCCCGTCTCCTCTAGTCTTC | TGAGATGCTGTCTCTAAAATAAATAGA | TTCTCTCTTTGTACCCACAC |
| 570341 | TGTTGGTTCGATGAGAGCAT | AAGTGGGGAACAAACACTGG | GTACCTATATTTGTATTGCACTTA |

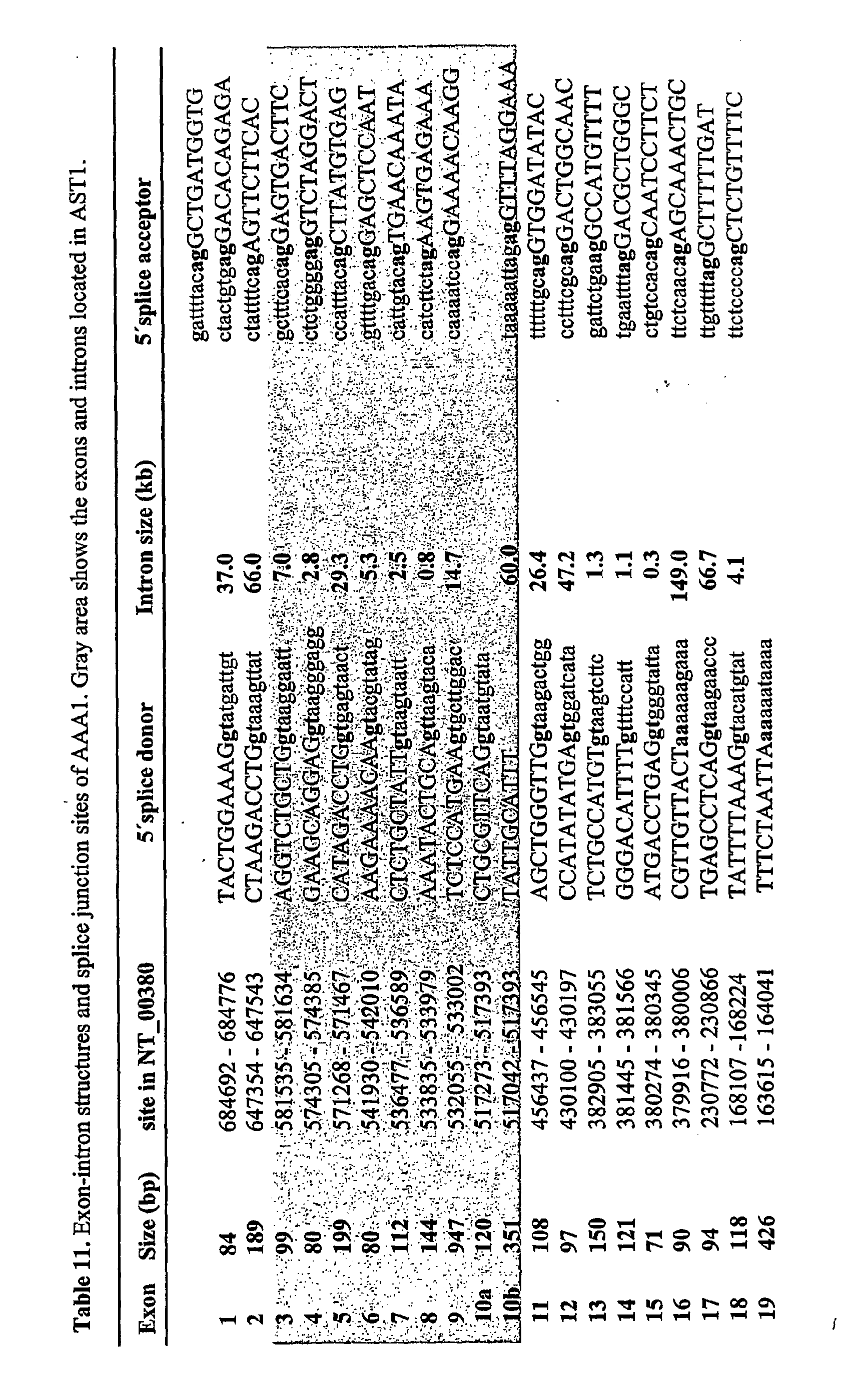

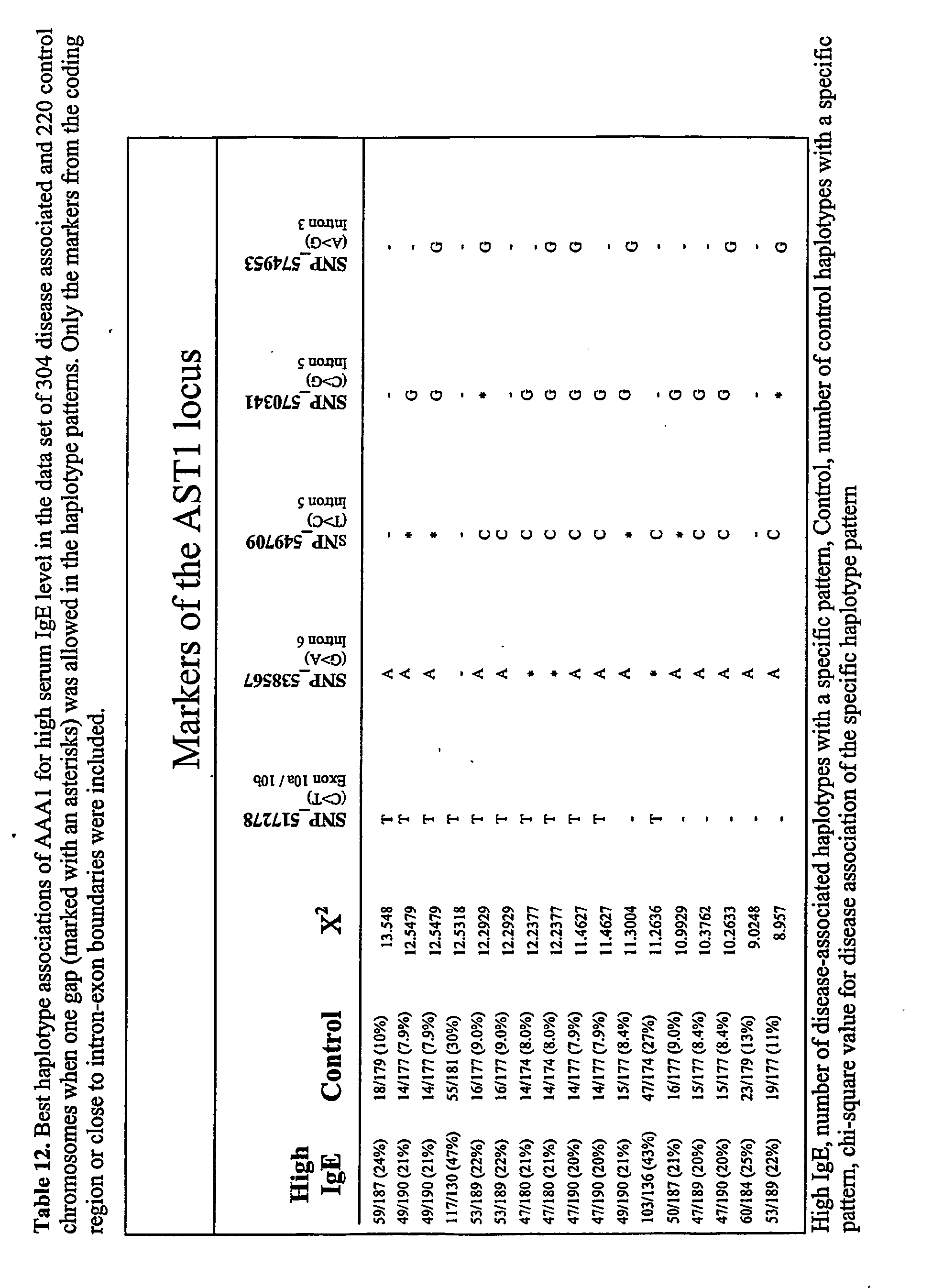

**Table 13. Haplotype specific AST1 polymorphisms for diagnostic testing. These markers can be used for the haplotype identification of an individual without phase information.**

| **Haplotype** | **Type of polymorphism** | **Position in SEQ ID NO:1** | **Position in NT_000380** | **Allele present in the spesific haplotype** | **Allele present in other haplotypes** |
|---|---|---|---|---|---|
| H1 | SNP | 4631 | 514413 | T | C |
| H1 | SNP | 51975 | 561757 | G | C |
| H1 | SNP | 53922 | 563704 | T | C |
| H2 | SNP | 5634 | 515416 | A | G |
| H2 | SNP | 6739 | 516521 | A | G |
| H2 | SNP | 7550 | 517332 | A | G |
| H2 | deletion | 9199-9201 | 518981-83 | deletion of TCT | no deletion |
| H2 | SNP | 16980 | 526762 | C | T |
| H2 | SNP | 17147 | 526929 | A | G |
| H2 | SNP | 17435 | 527217 | G | A |
| H2 | SNP | 19272 | 529054 | T | A |
| H2 | SNP | 19452 | 529234 | A | G |
| H2 | SNP | 20309 | 530091 | A | G |
| H2 | SNP | 20789 | 530571 | T | G |
| H2 | SNP | 24869 | 534651 | C | T |
| H2 | SNP | 32976 | 542758 | C | T |
| H2 | SNP | 34716 6 | 544498 | C | A |
| H2 | deletion | 38850-52 | 548632-34 | deletion of CTC | no deletion |
| H2 | SNP | 50955 | 560737 | C | G |
| H2 | SNP | 51476 | 561258 | G | A |
| H2 | SNP | 52573 | 562355 | A | G |
| H2 | SNP | 55134 | 564916 | A | G |
| H2 | SNP | 56856 | 566638 | T | C |
| H2 | SNP | 65857 | 575639 | G | T |
| H2 | SNP | 66526 | 576308 | T | C |
| H2 | SNP | 66902 | 576684 | G | A |
| H2 | SNP | 72270 | 582052 | A | G |
| H2 | SNP | 111012 | 620794 | T | C |
| H2 | SNP | 112037 | 621819 | G | T |
| H2 | SNP | 115192 | 624974 | G | C |
| H2 | SNP | 123770 | 633552 | G | A |
| H2 | SNP | 123788 | 633570 | C | G |
| H3 | SNP | 22869 | 532651 | C | T |
| H4 | deletion | 7334-35 | 517116-17 | deletion of AT | no deletion |
| H4 | SNP | 16893 | 526675 | C | T |
| H4 | SNP | 17209 | 526991 | A | C |
| H4 | SNP | 26198 | 535980 | C/T* | C |
| H4 | SNP | 32124 | 541906 | A/C* | C |
| H4 | SNP | 36551 | 546333 | A | G |
| H4 | SNP | 116032 | 625814 | A/G* | G |
| H6 | SNP | 54199 | 563981 | C | T |
| H6 | SNP | 57790 | 567572 | C | A |
| H6 | SNP | 64559 | 574341 | T | C |
| H7 | SNP | 1 | 509783 | T | C |
| H7 | SNP | 93 | 509875 | G | A |
| H7 | SNP | 918 | 510700 | A | G |
| H7 | SNP | 983 | 510765 | T | A |
| H7 | SNP | 987 | 510769 | C | T |
| H7 | SNP | 1542 | 511324 | T | C |
| H7 | SNP | 1710 | 511492 | G | A |
| H7 | SNP | 1818 | 511600 | T | C |
| H7 | SNP | 1927 | 511709 | T | A |
| H7 | SNP | 2254 | 512036 | C | T |
| H7 | SNP | 4689 | 514471 | G | C |
| H7 | SNP | 5442 | 515224 | G | C |
| H7 | SNP | 18383 | 528165 | A | G |
| H7 | SNP | 18978 | 528760 | G | A |
| H7 | SNP | 19671 | 529453 | A | G |
| H7 | SNP | 28866 | 538648 | C | G |
| H7 | SNP | 52776 | 562558 | C | G |

| | | | | | |
|---|---|---|---|---|---|
| * A rare polymorphism found only in H4 haplotype | | | | | |

**Table 14. AST1 polymorphisms specific for the different haplotype combinations for diagnostic testing. These markers can be used for the identification of certain haplotype combinations of an individual without phase information.**

| Haplotype combination | Type of polymorphism | Position in sea ID NO:1 | Position in NT_000380 | Allele present in the spesific haplotype combination | Allele present in other haplotypes |
|---|---|---|---|---|---|
| H4+H5 | SNP | 7125 | 516907 | A | C |
| H4+H5 | SNP | 18927 | 528709 | T | G |
| H4+H5 | SNP | 19268 | 529050 | C | G |
| H4+H5 | SNP | 19712 | 529494 | A | C |
| H4+H5 | deletion | 22122-23 | 531904-5 | deletion of TG | no deletion |
| H4+H5 | SNP | 24007 | 533789 | C | T |
| H4+H5 | SNP | 27404 | 537186 | T | C |
| H4+H5 | SNP | 28785 | 538567 | A | G |
| H4+H5 | SNP | 33350 | 543132 | A | C |
| H4+H5 | SNP | 33798 | 543580 | A | G |
| H4+H5 | SNP | 34362 | 544144 | C | G |
| H4+H5 | insertion | 52286-87 | 562068-69 | insertion of CC | no insertion |
| H4+H5 | SNP | 53803 | 563585 | T | C |
| H4+H5 | SNP | 55000 | 564782 | A | G |
| H4+H5 | SNP | 56683 | 566465 | T | C |
| H4+H5 | SNP | 67919 | 577701 | C | T |
| H4+H5 | SNP | 76101 | 585883 | C | G |
| H4+H5 | SNP | 82332 | 592114 | T | C |
| H4+H5 | SNP | 112726 | 622508 | A | T |
| H4+H5 | SNP | 113944 | 623726 | G | T |
| H4+H5 | SNP | 114945 | 624727 | G | A |
| H4+H5 | SNP | 116464 | 626246 | A | G |
| H4+H5 | SNP | 123667 | 633449 | A | G |
| H2+H4+H5 | SNP | 4012 | 513794 | A | C |
| H2+H4+H5 | SNP | 4961 | 514743 | G | A |
| H2+H4+H5 | SNP | 5850 | 515632 | G | A |
| H2+H4+H5 | SNP | 6312 | 516094 | T | C |
| H2+H4+H5 | SNP | 6392 | 516174 | T | G |
| H2+H4+H5 | SNP | 6485 | 516267 | A | G |
| H2+H4+H5 | SNP | 6522 | 516304 | G | C |
| H2+H4+H5 | SNP | 6646 | 516428 | G | A |
| H2+H4+H5 | SNP | 6760 | 516542 | C | T |
| H2+H4+H5 | deletion | 6821 | 516603 | deletion of T | no deletion |
| H2+H4+H5 | deletion | 7240-43 | 517022-25 | deletion of ACTT | no deletion |
| H2+H4+H5 | SNP | 7277 | 517059 | G | C |
| H2+H4+H5 | SNP | 7303 | 517085 | T | G |
| H2+H4+H5 | SNP | 7305 | 517087 | C | G |
| H2+H4+H5 | deletion | 7306-8 | 517088-90 | deletion of TGT | no deletion |
| H2+H4+H5 | SNP | 7496 | 517278 | T | C |
| H2+H4+H5 | SNP | 8490 | 518272 | T | C |
| H2+H4+H5 | SNP | 9649 | 519431 | G | T |
| H2+H4+H5 | deletion | 9782-9785 | 519564-67 | deletion of GTCT | no deletion |
| H2+H4+H5 | SNP | 11858 | 521640 | G | A |
| H2+H4+H5+H7 | SNP | 3877 | 513659 | C | G |
| H2+H4+H5+H7 | SNP | 7229 | 517011 | T | C |
| H2+H4+H5+H7 | SNP | 10816 | 520598 | C | T |
| H2+H4+H5+H7 | SNP | 12581 | 522363 | C | G |
| H2+H4+H5+H7 | SNP | 19360 | 529142 | A | G |
| H2+H4+H5+H7 | SNP | 19774 | 529556 | A | C |
| H2+H4+H5+H7 | SNP | 20038 | 529820 | C | T |
| H2+H4+H5+H7 | SNP | 20395 | 530177 | C | T |
| H6+H7 | SNP | 22475 | 532257 | C | T |
| H6+H7 | SNP | 22493 | 532275 | G | A |
| H6+H7 | SNP | 22715 | 532497 | A | G |
| H6+H7 | SNP | 22934 | 532716 | T | A |
| H6+H7 | SNP | 24264 | 534046 | T | G |
| H6+H7 | SNP | 26356 | 536138 | T | C |
| H6+H7 | SNP | 26675 | 536457 | G | A |
| H6+H7 | SNP | 28197 | 537979 | A | G |
| H6+H7 | SNP | 28858 | 538640 | C | T |
| H6+H7 | SNP | 31224 | 541006 | A | G |
| H6+H7 | SNP | 32185 | 541967 | C | T |
| H6+H7 | deletion | 34909 | 544691 | deletion of T | no deletion |
| H6+H7 | SNP | 37327 | 547109 | T | G |
| H6+H7 | SNP | 37415 | 547197 | A | G |
| H6+H7 | SNP | 37685 | 547467 | G | A |
| H6+H7 | SNP | 37959 | 547741 | T | C |
| H6+H7 | SNP | 39343 | 549125 | T | G |
| H6+H7 | SNP | 50493 | 560275 | A | G |
| H6+H7 | SNP | 50835 | 560617 | C | A |
| H6+H7 | CA-repeat | 51022-49 | 560804-31 | (CA)8 | (CA)6TA(CA)7 |
| H6+H7 | SNP | 51536 | 561318 | T | C |
| H6+H7 | SNP | 51884 | 561666 | T | G |
| H6+H7 | SNP | 60604 | 570386 | T | A |
| H6+H7 | SNP | 61165 | 570947 | A | G |
| H6+H7 | SNP | 75115 | 584897 | A | G |
| H6+H7 | SNP | 82922 | 592704 | A | T |
| H6+H7 | SNP | 83552 | 593334 | T | C |
| H6+H7 | SNP | 85271 | 595053 | A | G |
| H6+H7 | SNP | 110989 | 620771 | C | T |
| H6+H7 | SNP | 112030 | 621812 | C | G |
| H6+H7 | SNP | 113428 | 623210 | G | C |
| H6+H7 | SNP | 115628 | 625410 | C | T |
| H6+H7 | SNP | 116515 | 626297 | A | G |
| H6+H7 | SNP | 116926 | 626708 | C | T |
| H6+H7 | SNP | 117276 | 627058 | A | T |
| H2+H6+H7 | SNP | 28770 | 538552 | T | C |
| H2+H6+H7 | SNP | 31910 | 541692 | A | G |
| H2+H6+H7 | SNP | 37931 | 547713 | T | C |
| H2+H6+H7 | SNP | 39314 | 549096 | A | G |
| H2+H6+H7 | SNP | 50197 | 559979 | T | G |
| H2+H6+H7 | SNP | 50334 | 560116 | A | G |
| H2+H6+H7 | SNP | 50632 | 560414 | A | G |
| H2+H6+H7 | SNP | 51217 | 560999 | A | G |
| H2+H6+H7 | SNP | 51861 | 561643 | G | C |
| H4+H5+H6+H7 | SNP | 54148 | 563930 | A | G |
| H4+H5+H6+H7 | SNP | 54641 | 564423 | C | G |
| H4+H5+H6+H7 | SNP | 54751 | 564533 | C | T |
| H4+H5+H6+H7 | SNP | 60559 | 570341 | G | C |
| H4+H5+H6+H7 | SNP | 66164 | 575946 | T | C |
| H4+H5+H6+H7 | SNP | 67857 | 577639 | A | T |
| H2+H7 | SNP | 16845 | 526627 | C | T |
| H2+H7 | SNP | 20089 | 529871 | A | T |
| H2+H4+H5+H6+H7 | SNP | 21850 | 531632 | T | C |
| H2+H4+H5+H6+H7 | SNP | 36909 | 546691 | A | C |
| H2+H4+H5+H6+H7 | SNP | 39927 | 549709 | C | T |
| H2+H4+H5+H6+H7 | SNP | 45826 | 555608 | T | C |
| H2+H4+H5+H6+H7 | SNP | 65171 | 574953 | G | A |
| H2+H4+H5+H6+H7 | SNP | 112283 | 622065 | T | A |
| H2+H4+H5+H6+H7 | SNP | 112859 | 622641 | C | A |

**Table 15. The cellular location of transiently expressed GPRA A, B, B-short, C, D, E, and F variants. Results are means at least from two independent experiments.**

| | GPRA A | GPRA B | GPRA B-short | GPRA C | GPRA D | GPRA E | GPRA F |
|---|---|---|---|---|---|---|---|
| cytoplasm | 29% | 48% | 100% | 100% | 100% | 100% | 100% |
| Plasma membrane | 71% | 52% | 0% | 0% | 0% | 0% | 0% |

## Claims

1. A method for determining risk of a pulmonary disease associated with lower airways obstruction or an IgE mediated disease in an individual, comprising;
determining whether a biological sample from an individual contains haplotypes H2, H4, H5, and H7 in the AST-1 locus as defined by SEQ ID NO:1, the presence of at least one of said haplotypes in the sample indicating risk of a pulmonary disease associated with lower airways obstruction or an IgE mediated disease, wherein
i) haplotype H2 is defined by the following polymorphisms:
- nucleotide C located in position 515224 in contig NT_000380 (i.e. position 5442 in SEQ ID NO:1);
- nucleotide C located in position 522363 in contig NT_000380 (i.e. position 12581 in SEQ ID NO:1);
- nucleotide A located in position 529556 in contig NT_000380 (i.e. position 19774 in SEQ ID NO:1);
- nucleotide G located in position 546333 in contig NT_000380 (i.e. position 36551 in SEQ ID NO:1);
- nucleotide T located in position 555608 in contig NT_000380 (i.e. position 45826 in SEQ ID NO:1);
- nucleotide C located in position 563704 in contig NT_000380 (i.e. position 53922 in SEQ ID NO:1); and
- nucleotide G located in position 585883 in contig NT_000380 (i.e. position 70101 in SEQ ID NO:1);
ii) haplotype H4 is defined by the following polymorphisms:
- nucleotide C located in position 515224 in contig NT_000380 (i.e. position 5442 in SEQ ID NO:1);
- nucleotide C located in position 522363 in contig NT_000380 (i.e. position 12581 in SEQ ID NO:1);
- nucleotide A located in position 529556 in contig NT_000380 (i.e. position 19774 in SEQ ID NO:1);
- nucleotide A located in position 546333 in contig NT_000380 (i.e. position 36551 in SEQ ID NO:1);
- nucleotide T located in position 555608 in contig NT_000380 (i.e. position 45826 in SEQ ID NO:1);
- nucleotide C located in position 563704 in contig NT_000380 (i.e. position 53922 in SEQ ID NO:1); and
- nucleotide C located in position 585883 in contig NT_000380 (i.e. position 76101 in SEQ ID NO:1);
iii) haplotype H5 is defined by the following polymorphisms:
- nucleotide C located in position 515224 in contig NT_000380 (i.e. position 5442 in SEQ ID NO:1);
- nucleotide C located in position 522363 in contig NT_000380 (i.e. position 12581 in SEQ ID NO:1);
- nucleotide A located in position 529556 in contig NT_000380 (i.e. position 19774 in SEQ ID NO:1);
- nucleotide G located in position 546333 in contig NT_000380 (i.e. position 36551 in SEQ ID NO:1);
- nucleotide T located in position 555608 in contig NT_000380 (i.e. position 45826 in SEQ ID NO:1);
- nucleotide C located in position 563704 in contig NT_000380 (i.e. position 53922 in SEQ ID NO:1); and
- nucleotide C located in position 585883 in contig NT_000380 (i.e. position 76101 in SEQ ID NO:1);
iv) haplotype H7 is defined by the following polymorphisms:
- nucleotide G located in position 515224 in contig NT_000380 (i.e. position 5442 in SEQ ID NO:1);
- nucleotide C located in position 522363 in contig NT_000380 (i.e. position 12581 in SEQ ID NO:1);
- nucleotide A located in position 529556 in contig NT_000380 (i.e. position 19774 in SEQ ID NO:1);
- nucleotide G located in position 546333 in contig NT_000380 (i.e. position 36551 in SEQ ID NO:1);
- nucleotide T located in position 555608 in contig NT_000380 (i.e. position 45826 in SEQ ID NO:1);
- nucleotide C located in position 563704 in contig NT_000380 (i.e. position 53922 in SEQ ID NO:1); and
- nucleotide G located in position 585883 in contig NT_000380 (i.e. position 76101 in SEQ ID NO:1).

2. The method of claim 1, wherein the presence of at least one of said haplotypes is determined by identifying a polymorphic site occurring in a position listed in Table 14.

3. The method of claim 1, wherein the determining is performed by allele specific amplification, allele specific hybridization, single strand conformation polymorphism (SSCP), oligonucleotide ligation assay, single-base extension assay, or restriction fragment length polymorphism (RFLP).

4. The method of claim 1, wherein said disease is chronic obstructive pulmonary disease (COPD) or asthma.

5. A kit for use in the diagnostics of predisposition to a pulmonary disease associated with lower airways obstruction or an IgE mediated disease, said kit comprising;
a container; and in the container:
compounds, preferably labeled, capable of detecting AST-1 haplotypes H2, H4, H5 and H7 as defined in claim 1.

6. The kit of claim 5, wherein said compounds are capable of detecting polymorphic sites occurring in positions listed in Table 14.

7. The kit of claim 5, wherein the compounds are primers or probes.

8. The kit of claim 5, wherein said disease is chronic obstructive pulmonary disease (COPD) or asthma.

## Patentansprüche

1. Verfahren zur Bestimmung des Risikos einer Lungenerkrankung, die mit einer Verlegung der unteren Atemwege verbunden ist, oder einer IgE-vermittelten Erkrankung in einem Individuum, wobei das Verfahren umfasst:
Bestimmen, ob eine biologische Probe von einem Individuum Haplotypen H2, H4, H5 und H7 im durch SEQ ID NO: 1 definierten AST-1-Locus enthält, wobei das Vorliegen von mindestens einem dieser Haplotypen in der Probe ein Risiko einer Lungenerkrankung, die mit einer Verlegung der unteren Atemwege verbunden ist, oder einer IgE-vermittelten Erkrankung anzeigt, und wobei
i) Haplotyp H2 durch die folgenden Polymorphismen definiert ist:
- Nucleotid C an Position 515224 im contig NT_000380 (d.h. Position 5442 in SEQ ID NO:1);
- Nucleotid C an Position 522363 im contig NT_000380 (d.h. Position 12581 in SEQ ID NO:1);
- Nucleotid A an Position 529556 im contig NT_000380 (d.h. Position 19774 in SEQ ID NO:1);
- Nucleotid G an Position 546333 im contig NT_000380 (d.h. Position 36551 in SEQ ID NO:1);
- Nucleotid T an Position 555608 im contig NT_000380 (d.h. Position 45826 in SEQ ID NO:1);
- Nucleotid C an Position 563704 im contig NT_000380 (d.h. Position 53922 in SEQ ID NO:1); und
- Nucleotid G an Position 585883 im contig NT_000380 (d.h. Position 76101 in SEQ ID NO:1);
ii) Haplotyp H4 durch die folgenden Polymorphismen definiert ist:
- Nucleotid C an Position 515224 im contig NT_000380 (d.h. Position 5442 in SEQ ID NO:1);
- Nucleotid C an Position 522363 im contig NT_000380 (d.h. Position 12581 in SEQ ID NO:1);
- Nucleotid A an Position 529556 im contig NT_000380 (d.h. Position 19774 in SEQ ID NO:1);
- Nucleotid A an Position 546333 im contig NT_000380 (d.h. Position 36551 in SEQ ID NO:1);
- Nucleotid T an Position 555608 im contig NT_000380 (d.h. Position 45826 in SEQ ID NO:1);
- Nucleotid C an Position 563704 im contig NT_000380 (d.h. Position 53922 in SEQ ID NO:1); und
- Nucleotid C an Position 585883 im contig NT_000380 (d.h. Position 76101 in SEQ ID NO:1);
iii) Haplotyp H5 durch die folgenden Polymorphismen definiert ist:
- Nucleotid C an Position 515224 im contig NT_000380 (d.h. Position 5442 in SEQ ID NO:1);
- Nucleotid C an Position 522363 im contig NT_000380 (d.h. Position 12581 in SEQ ID NO:1);
- Nucleotid A an Position 529556 im contig NT_000380 (d.h. Position 19774 in SEQ ID NO:1);
- Nucleotid G an Position 546333 im contig NT_000380 (d.h. Position 36551 in SEQ ID NO:1);
- Nucleotid T an Position 555608 im contig NT_000380 (d.h. Position 45826 in SEQ ID NO:1);
- Nucleotid C an Position 563704 im contig NT_000380 (d.h. Position 53922 in SEQ ID NO:1); und
- Nucleotid C an Position 585883 im contig NT_000380 (d.h. Position 76101 in SEQ ID NO:1);
iv) Haplotyp H7 durch die folgenden Polymorphismen definiert ist:
- Nucleotid G an Position 515224 im contig NT_000380 (d.h. Position 5442 in SEQ ID NO:1);
- Nucleotid C an Position 522363 im contig NT_000380 (d.h. Position 12581 in SEQ ID NO:1);
- Nucleotid A an Position 529556 im contig NT_000380 (d.h. Position 19774 in SEQ ID NO:1);
- Nucleotid G an Position 546333 im contig NT_000380 (d.h. Position 36551 in SEQ ID NO:1);
- Nucleotid T an Position 555608 im contig NT_000380 (d.h. Position 45826 in SEQ ID NO:1);
- Nucleotid C an Position 563704 im contig NT_000380 (d.h. Position 53922 in SEQ ID NO:1); und
- Nucleotid G an Position 585883 im contig NT_000380 (d.h. Position 76101 in SEQ ID NO:1).

2. Verfahren nach Anspruch 1, wobei das Vorliegen von mindestens einem der Haplotypen durch Identifizieren einer polymorphen Stelle bestimmt wird, die an einer in Tabelle 14 aufgelisteten Position vorkommt.

3. Verfahren nach Anspruch 1, wobei das Bestimmen durch Allel-spezifische Amplifikation, Allel-spezifische Hybridisierung, Einzelstrang-Konformationspolymorphismus (SSCP), Oligonucleotidligierungs-Assay, Einzelbasenverlängerungs-Assay oder Restriktionsfragmentlängenpolymorphismus (RFLP) durchgeführt wird.

4. Verfahren nach Anspruch 1, wobei die Krankheit chronisch-obstruktive Lungenerkrankung (COPD) oder Asthma ist.

5. Kit zur Verwendung in der Diagnostik einer Veranlagung für eine Lungenerkrankung, die mit einer Verlegung der unteren Atemwege verbunden ist, oder für eine IgE-vermittelte Erkrankung, wobei der Kit umfasst:
einen Behälter; und im Behälter:
Verbindungen, vorzugsweise markiert, die in der Lage sind, AST-1-Haplotypen H2, H4, H5 und H7 wie in Anspruch 1 definiert nachzuweisen.

6. Kit nach Anspruch 5, wobei die Verbindungen in der Lage sind, polymorphe Stellen nachzuweisen, die an den in Tabelle 14 aufgelisteten Positionen vorkommen.

7. Kit nach Anspruch 5, wobei die Verbindungen Primer oder Sonden sind.

8. Kit nach Anspruch 5, wobei die Krankheit chronisch-obstruktive Lungenerkrankung (COPD) oder Asthma ist.

## Revendications

1. Procédé de détermination du risque d'une maladie pulmonaire associée à une obstruction des voies respiratoires inférieures ou d'une maladie médiée par les IgE chez un individu, comprenant :
la détermination si un échantillon biologique provenant d'un individu contient les haplotypes H2, H4, H5 et H7 dans le locus AST-1 tel que représenté par SEQ ID n° 1, la présence d'au moins l'un desdits haplotypes dans l'échantillon indiquant le risque d'une maladie pulmonaire associée à une obstruction des voies respiratoires inférieures ou d'une maladie médiée par les IgE, où
i) l'haplotype H2 est défini par les polymorphismes suivants :
- nucléotide C localisé en position 515224 dans le NT-000380 contigu (c'est-à-dire, la position 5442 dans SEQ ID n° 1) ;
- nucléotide C localisé en position 522363 dans le NT-000380 contigu (c'est-à-dire, la position 12581 dans SEQ ID n° 1) ;
- nucléotide A localisé en position 529556 dans le NT-000380 contigu (c'est-à-dire, la position 19774 dans SEQ ID n° 1) ;
- nucléotide G localisé en position 546333 dans le NT-000380 contigu (c'est-à-dire, la position 36551 dans SEQ ID n° 1) ;
- nucléotide T localisé en position 555608 dans le NT-000380 contigu (c'est-à-dire, la position 45826 dans SEQ ID n° 1) ;
- nucléotide C localisé en position 563704 dans le NT-000380 contigu (c'est-à-dire, la position 53922 dans SEQ ID n° 1) ; et
- nucléotide G localisé en position 585883 dans le NT-000380 contigu (c'est-à-dire, la position 76101 dans SEQ ID n° 1) ;
ii) l'haplotype H4 est défini par les polymorphismes suivants :
- nucléotide C localisé en position 515224 dans le NT-000380 contigu (c'est-à-dire, la position 5442 dans SEQ ID n° 1) ;
- nucléotide C localisé en position 522363 dans le NT-000380 contigu (c'est-à-dire, la position 12581 dans SEQ ID n° 1) ;
- nucléotide A localisé en position 529556 dans le NT-000380 contigu (c'est-à-dire, la position 19774 dans SEQ ID n° 1) ;
- nucléotide A localisé en position 546333 dans le NT-000380 contigu (c'est-à-dire, la position 36551 dans SEQ ID n° 1) ;
- nucléotide T localisé en position 555608 dans le NT-000380 contigu (c'est-à-dire, la position 45826 dans SEQ ID n° 1) ;
- nucléotide C localisé en position 563704 dans le NT-000380 contigu (c'est-à-dire, la position 53922 dans SEQ ID n° 1) ; et
- nucléotide C localisé en position 585883 dans le NT-000380 contigu (c'est-à-dire, la position 76101 dans SEQ ID n° 1) ;
iii) l'haplotype H5 est défini par les polymorphismes suivants :
- nucléotide C localisé en position 515224 dans le NT-000380 contigu (c'est-à-dire, la position 5442 dans SEQ ID n° 1) ;
- nucléotide C localisé en position 522363 dans le NT-000380 contigu (c'est-à-dire, la position 12581 dans SEQ ID n° 1) ;
- nucléotide A localisé en position 529556 dans le NT-000380 contigu (c'est-à-dire, la position 19774 dans SEQ ID n° 1) ;
- nucléotide G localisé en position 546333 dans le NT-000380 contigu (c'est-à-dire, la position 36551 dans SEQ ID n° 1) ;
- nucléotide T localisé en position 555608 dans le NT-000380 contigu (c'est-à-dire, la position 45826 dans SEQ ID n° 1) ;
- nucléotide C localisé en position 563704 dans le NT-000380 contigu (c'est-à-dire, la position 53922 dans SEQ ID n° 1) ; et
- nucléotide C localisé en position 585883 dans le NT-000380 contigu (c'est-à-dire, la position 76101 dans SEQ ID n° 1) ;
iv) l'haplotype H7 est défini par les polymorphismes suivants :
- nucléotide G localisé en position 515224 dans le NT-000380 contigu (c'est-à-dire, la position 5442 dans SEQ ID n° 1) ;
- nucléotide C localisé en position 522363 dans le NT-000380 contigu (c'est-à-dire, la position 12581 dans SEQ ID n° 1) ;
- nucléotide A localisé en position 529556 dans le NT-000380 contigu (c'est-à-dire, la position 19774 dans SEQ ID n° 1) ;
- nucléotide G localisé en position 546333 dans le NT-000380 contigu (c'est-à-dire, la position 36551 dans SEQ ID n° 1) ;
- nucléotide T localisé en position 555608 dans le NT-000380 contigu (c'est-à-dire, la position 45826 dans SEQ ID n° 1) ;
- nucléotide C localisé en position 563704 dans le NT-000380 contigu (c'est-à-dire, la position 53922 dans SEQ ID n° 1) ; et
- nucléotide G localisé en position 585883 dans le NT-000380 contigu (c'est-à-dire, la position 76101 dans SEQ ID n° 1).

2. Procédé selon la revendication 1, dans lequel la présence d'au moins l'un desdits haplotypes est déterminée par l'identification d'un site polymorphique apparaissant dans une position énumérée dans le tableau 14.

3. Procédé selon la revendication 1, dans lequel la détermination est réalisée par une amplification spécifique de l'allèle, une hybridation spécifique de l'allèle, un polymorphisme de conformation simple brin (SSCP), un test de ligature d'oligonucléotides, un test d'extension de base simple, ou un polymorphisme de longueur des fragments de restriction (RFLP).

4. Procédé selon la revendication 1, dans lequel ladite maladie est la broncho-pneumopathie chronique obstructive (BPCO) ou l'asthme.

5. Kit pour une utilisation dans le diagnostic d'une prédisposition à une maladie pulmonaire associée à une obstruction des voies respiratoires inférieures ou une maladie médiée par les IgE, ledit kit comprenant :
un récipient ; et dans le récipient :
des composés, de préférence marqués, capables de détecter les haplotypes H2, H4, H5 et H7 dans AST-1 tels que définis dans la revendication 1.

6. Kit selon la revendication 5, dans lequel lesdits composés sont capables de détecter des sites polymorphiques apparaissant dans les positions énumérées dans le tableau 14.

7. Kit selon la revendication 5, dans lequel les composés sont des amorces ou des sondes.

8. Kit selon la revendication 5, dans lequel ladite maladie est la broncho-pneumopathie chronique obstructive (BPCO) ou l'asthme.
